(19) Europäisches Patentamt
European Patent Office
Office européen des brevets

(11) EP 4 410 792 A1

(12) # EUROPEAN PATENT APPLICATION
published in accordance with Art. 153(4) EPC

(43) Date of publication:
**07.08.2024 Bulletin 2024/32**

(21) Application number: **22875192.1**

(22) Date of filing: **30.09.2022**

(51) International Patent Classification (IPC):
***C07D 471/04** (2006.01)* ***C07D 401/14** (2006.01)*
***A61K 31/496** (2006.01)* ***A61K 31/4375** (2006.01)*
***A61P 35/00** (2006.01)*

(52) Cooperative Patent Classification (CPC):
**A61K 31/4375; A61K 31/496; A61P 35/00; C07D 401/14; C07D 471/04**

(86) International application number:
**PCT/CN2022/123443**

(87) International publication number:
**WO 2023/051812 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2021 CN 202111160607**
**03.12.2021 CN 202111472095**
**17.01.2022 CN 202210050069**
**07.03.2022 CN 202210217852**
**29.03.2022 CN 202210322736**
**10.05.2022 CN 202210506382**
**02.06.2022 CN 202210625556**

(71) Applicant: **Xizang Haisco Pharmaceutical Co., Ltd.**
**Lhoka, Tibet 856099 (CN)**

(72) Inventors:
- **LI, Yao**
**Zedang Town Shannan, Tibet 856000 (CN)**
- **ZHANG, Haoliang**
**Zedang Town Shannan, Tibet 856000 (CN)**
- **CHEN, Lei**
**Zedang Town Shannan, Tibet 856000 (CN)**
- **FANG, Linyong**
**Zedang Town Shannan, Tibet 856000 (CN)**
- **WANG, Long**
**Zedang Town Shannan, Tibet 856000 (CN)**
- **LUO, Yufeng**
**Zedang Town Shannan, Tibet 856000 (CN)**
- **TANG, Pingming**
**Zedang Town Shannan, Tibet 856000 (CN)**
- **YU, Yan**
**Zedang Town Shannan, Tibet 856000 (CN)**
- **ZHANG, Chen**
**Zedang Town Shannan, Tibet 856000 (CN)**
- **YAN, Pangke**
**Zedang Town Shannan, Tibet 856000 (CN)**

(74) Representative: **Casalonga**
**Casalonga & Partners**
**Bayerstraße 71/73**
**80335 München (DE)**

(54) **NITROGEN-CONTAINING HETEROCYCLIC DERIVATIVE PARP INHIBITOR AND USE THEREOF**

(57) Provided are a compound represented by formula (1), a stereoisomer, pharmaceutically acceptable salt, solvate, and eutectic or deuterated compound thereof, or a pharmaceutical composition comprising same, and a use thereof as a PARP-1 inhibitor in the preparation of a medication for treating related diseases. Each group in formula (I) is as defined in the description.

EP 4 410 792 A1

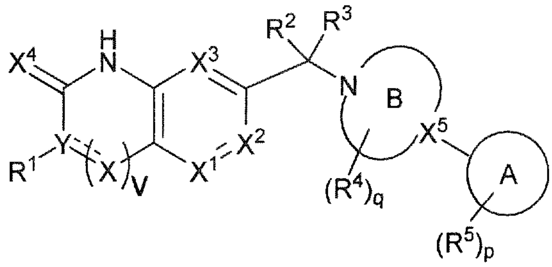

**(I)**

## Description

Technical Field

The present invention belongs to the field of drugs, and in particular relates to a small molecule compound having a PARP-1 inhibitory activity, or a stereoisomer, pharmaceutically acceptable salt, solvate and eutectic or deuterated compound thereof, and the use thereof in the preparation of a drug for treating related diseases.

Background Art

Approximately 5% of breast cancer patients are associated with germline mutations in the BRCA1/2 genes (3% in the BRCA1 gene and 2% in the BRCA2 gene). Most breast cancers caused by BRCA1 mutations are triple-negative breast cancers (70%), while BRCA2 mutations are more likely to cause oestrogen receptor-positive breast cancers (70%). The BRCA1/2 genes are tumour suppressor genes and play an important role in DNA damage repair, normal cell growth, etc. Mutations in the genes can inhibit the normal repair ability after DNA damage and cause homologous recombination deficiency (HRD), that is, loss of BRCA function or mutation or loss of function in other homologous recombination-related genes, making repair of DNA double-strand breaks impossible through homologous recombination repair (HRR), eventually leading to cancer.

Poly(ADP-ribose) polymerase (PARP) is a DNA repair enzyme that plays a key role in the DNA repair pathway. PARP is activated by DNA damage and breakage. As a molecular sensor of DNA damage, it has the function of identifying and binding to the DNA break location, thereby activating and catalysing the polyADP ribosylation of the receptor protein and participating in the DNA repair process. PARP plays a key role in the process of DNA single-strand base excision and repair. In HRD tumour cells, DNA double-strand breaks cannot be repaired, and PARP inhibitors block the single-strand repair, resulting in a "synthetic lethal" effect, leading to tumour cell death.

PARP inhibitors have a "trapping" effect on the PARP protein, causing the PARP protein that binds to damaged DNA to be trapped on the DNA, directly causing other DNA repair proteins to be unable to bind, eventually leading to cell death. At present, several PARP inhibitors have been successfully developed, such as olaparib, rucapalib and niraparib. However, adverse reactions limit their ability to be used in combination with chemotherapy drugs. This may be related to the lack of selectivity of marketed PARP inhibitors against the PARP family. These side effects include intestinal toxicity caused by tankyrase inhibition and haematological toxicity caused by PARP-2 inhibition. Therefore, it is of great clinical significance to develop highly selective PARP-1 inhibitors and reduce the toxic and side effects associated with non-selective PARP inhibitors.

Summary of the Invention

The objective of the present invention is to provide a compound that inhibits PARP-1, or a stereoisomer, solvate, or pharmaceutically acceptable salt thereof, and the medical application thereof. The compound has the advantages of good efficacy, low toxic and side effects, high safety, strong selectivity, good pharmacokinetics, high bioavailability, and no inhibition to CYP enzymes.

The present invention relates to a compound represented by formula (I), (1-1), (1-2), (II), (II-a), (11-b), (III), (III-a), (III-b), (IV), (IV-a), (IV-b), (V), (VI) or (VII), or a stereoisomer, solvate or pharmaceutically acceptable salt thereof,

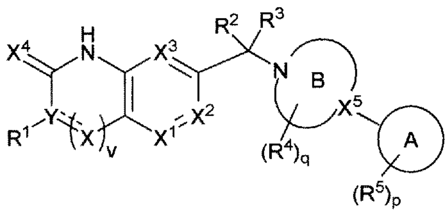
(I)

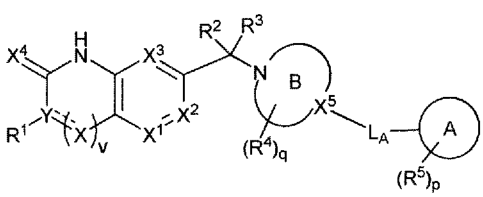
(I-1)

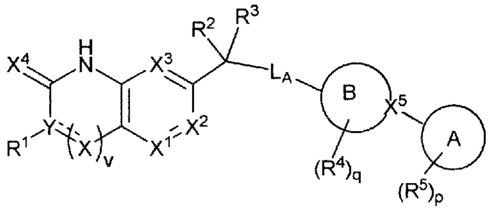 (I-2)

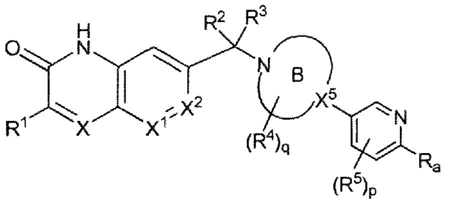 (II)

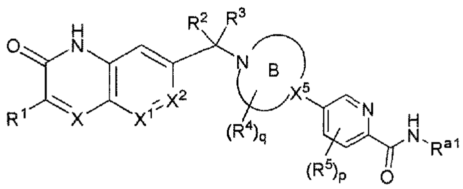 (II-a)

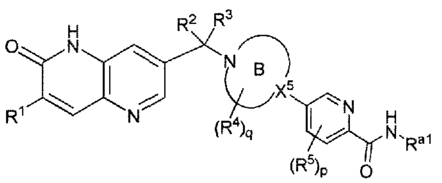 (II-b)

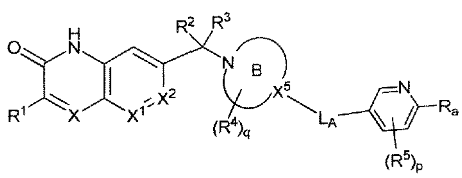 (III)

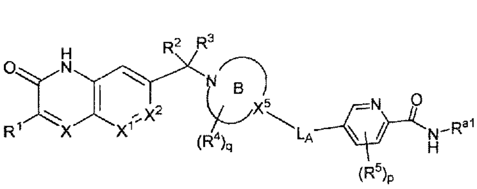 (III-a)

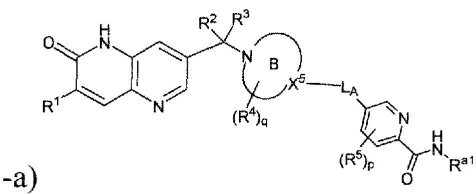 (III-b)

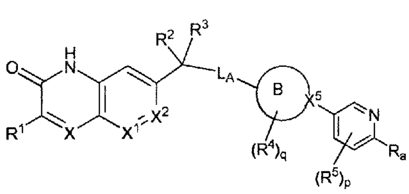 (IV)

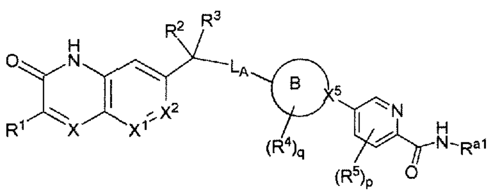 (IV-a)

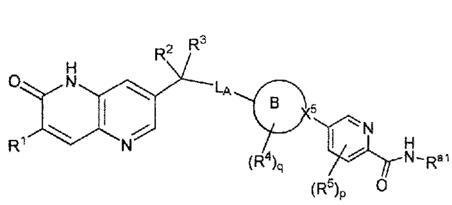 (IV-b)

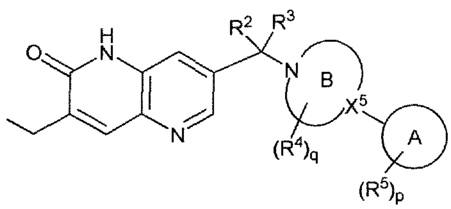 (V)

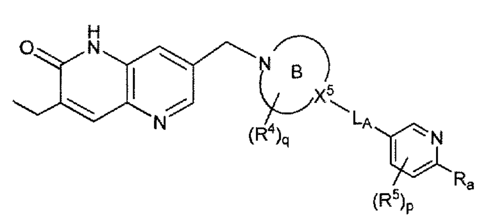 (VI)

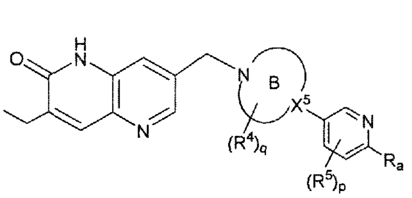 (VII)

wherein each X is independently selected from $CR^x$, $C(R^x)_2$, O, N or $NR^x$;

Y is selected from N, C or CH; in some embodiments, Y is selected from N or C; in some embodiments, Y is selected from C;

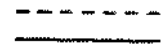

represents a single bond or a double bond, provided that when

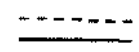

represents a single bond, X is selected from $C(R^x)_2$, O or $NR^x$;
v is selected from 1, 2 or 3; in some embodiments, v is selected from 1 or 2; in some embodiments, v is selected from 1;
$X^1$, $X^2$ and $X^3$ are each independently selected from N or $CR^x$; in some embodiments, $X^1$ is selected from N, and $X^2$ and $X^3$ are selected from $CR^x$; in some embodiments, $X^1$, $X^2$ and $X^3$ are selected from N; in some embodiments, $X^1$ is selected from N, $X^2$ is selected from N, and $X^3$ is selected from $CR^x$; in some embodiments, $X^1$ is selected from N, $X^2$ is selected from $CR^x$, and $X^3$ is selected from N; in some embodiments, $X^1$, $X^2$ and $X^3$ are selected from $CR^x$; in some embodiments, $X^1$ is selected from $CR^x$, and $X^2$ and $X^3$ are selected from N; in some embodiments, $X^1$ is selected from $CR^x$, $X^2$ is selected from N, and $X^3$ is selected from $CR^x$; in some embodiments, $X^1$ is selected from $CR^x$, $X^2$ is selected from $CR^x$, and $X^3$ is selected from N;
$X^4$ is selected from O or S; in some embodiments, $X^4$ is selected from O; in some embodiments, $X^4$ is selected from S;
$X^5$ is independently selected from N, C or $CR^x$; in some embodiments, $X^5$ is selected from N or $CR^x$; in some embodiments, $X^5$ is independently selected from N, C or CH; in some embodiments, $X^5$ is selected from N; in some embodiments, $X^5$ is selected from $CR^x$; in some embodiments, $X^5$ is selected from CH; in some embodiments, $X^5$ is selected from C;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O; in some embodiments, each $R^x$ is independently selected from H, D, halogen, cyano, amino, hydroxyl, $C_{1-4}$ alkyl, halo $C_{1-4}$ alkyl, halo $C_{1-4}$ alkoxy, deuterated $C_{1-4}$ alkyl, deuterated $C_{1-4}$ alkoxy, $C_{1-4}$ alkoxy, $C_{2-4}$ alkenyl, $C_{2-4}$ alkynyl, $C_{1-2}$ alkyl-O-$C_{1-2}$ alkyl, $-(CH_2)_r-C_{3-6}$ monocyclic cycloalkyl, $-(CH_2)_r-C_{5-7}$ bicyclic spiro cycloalkyl, $-(CH_2)_r$-(4- to 6-membered heterocycloalkyl), or $-(CH_2)_r$-(7- to 9-membered bicyclic spiro heterocycloalkyl); or two $R^x$ on the same carbon atom together form =O; in some embodiments, each $R^x$ is independently selected from H, D, F, Cl, cyano, amino, hydroxyl, $C_{1-2}$ alkyl, halo $C_{1-2}$ alkyl, deuterated $C_{1-2}$ alkyl, $C_{1-2}$ alkoxy, $-(CH_2)_r-C_{3-4}$ monocyclic cycloalkyl, - $(CH_2)_r-C_{5-6}$ bicyclic spiro cycloalkyl, $-(CH_2)_r$-(4- to 5-membered heterocycloalkyl), or $-(CH_2)_r$-(7- to 8-membered bicyclic spiro heterocycloalkyl); or two $R^x$ on the same carbon atom together form =O; in some embodiments, each $R^x$ is independently selected from H, D, F, Cl, cyano, hydroxyl, $C_{1-2}$ alkyl, halo $C_{1-2}$ alkyl, or deuterated $C_{1-2}$ alkyl; or two $R^x$ on the same carbon atom together form =O; in some embodiments, each $R^x$ is independently selected from H, D, $C_{1-2}$ alkyl, halo $C_{1-2}$ alkyl, or deuterated $C_{1-2}$ alkyl; or two $R^x$ on the same carbon atom together form =O; in some embodiments, each $R^x$ is independently selected from H or D, or two $R^x$ on the same carbon atom together form =O; in some embodiments, each $R^x$ is independently selected from H or D;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1, 2 or 3 groups selected from D, halogen, cyano, amino, hydroxyl, $C_{1-6}$ alkyl or $C_{1-6}$ alkoxy; in some embodiments, $R^1$ is selected from halogen, nitro, cyano, amino, hydroxyl, - $SF_5$, $C_{1-4}$ alkyl, $C_{1-4}$ alkoxy, $C_{2-4}$ alkenyl, $C_{2-4}$ alkynyl, $C_{1-2}$ alkyl-O-$C_{1-2}$ alkyl, - $(CH_2)_r-C_{3-6}$ monocyclic cycloalkyl, $-(CH_2)_r-C_{5-7}$ bicyclic spiro cycloalkyl, $-(CH_2)_r$-(4- to 6-membered heterocycloalkyl), or $-(CH_2)_r$-(6- to 9-membered bicyclic spiro heterocycloalkyl), wherein the alkyl, alkoxy, alkenyl, alkynyl, cycloalkyl, or heterocycloalkyl is optionally further substituted with 1, 2 or 3 groups selected from D, halogen, cyano, amino, hydroxyl, $C_{1-2}$ alkyl, or $C_{1-2}$ alkoxy; in some embodiments, $R^1$ is selected from F, Cl, cyano, amino, hydroxyl, $C_{1-2}$ alkyl, $C_{1-2}$ alkoxy, $C_{2-3}$ alkenyl, $C_{1-2}$ alkyl-O-$C_{1-2}$ alkyl, $-(CH_2)_r-C_{3-4}$ monocyclic cycloalkyl, $-(CH_2)_r-C_{5-7}$ bicyclic spiro cycloalkyl, $-(CH_2)_r$-(4- to 5-membered heterocycloalkyl), or $-(CH_2)_r$-(6- to 8-membered bicyclic spiro heterocycloalkyl), wherein the alkyl, alkoxy, alkenyl, alkynyl, cycloalkyl, or heterocycloalkyl is optionally further substituted with 1, 2 or 3 groups selected from D, halogen, cyano, amino, hydroxyl, $C_{1-2}$ alkyl, or $C_{1-2}$ alkoxy; in some embodiments, $R^1$ is selected from cyano, $C_{1-2}$ alkyl, $C_{2-3}$ alkenyl, $C_{1-2}$ alkyl-O-$C_{1-2}$ alkyl, $C_{3-4}$ monocyclic cycloalkyl, or 4- to 5-membered heterocycloalkyl, wherein the alkyl, alkenyl, cycloalkyl, or heterocycloalkyl is optionally further substituted with 1, 2 or 3 groups selected from D, F, Cl, cyano, amino, hydroxyl, $C_{1-2}$ alkyl, or $C_{1-2}$ alkoxy; in some embodiments, $R^1$ is selected from cyano, $C_{1-2}$ alkyl, $C_{2-3}$ alkenyl, $C_{1-2}$ alkyl-O-$C_{1-2}$ alkyl, or $C_{3-4}$ monocyclic cycloalkyl;
each r is independently selected from 0, 1, 2 or 3; in some embodiments, each r is independently selected from 0

or 1; in some embodiments, r is selected from 0;

$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl; in some embodiments, $R^2$ and $R^3$ are each independently selected from H, D, halogen, cyano, amino, hydroxyl, $C_{1-2}$ alkyl-O-$C_{1-2}$ alkyl, hydroxy $C_{1-4}$ alkyl, $C_{1-4}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form $C_{3-5}$ cycloalkyl or 4- to 5-membered heterocycloalkyl; in some embodiments, $R^2$ and $R^3$ are each independently selected from H, D, halogen, cyano, amino, hydroxyl, $C_{1-2}$ alkyl-O-$C_{1-2}$ alkyl, hydroxy $C_{1-2}$ alkyl, $C_{1-2}$ alkoxy, halo $C_{1-2}$ alkyl, halo $C_{1-2}$ alkoxy, deuterated $C_{1-2}$ alkyl, deuterated $C_{1-2}$ alkoxy or $C_{1-2}$ alkyl; or $R^2$ and $R^3$ together with the carbon atom to which they are attached form $C_{3-4}$ cycloalkyl or 4-membered heterocycloalkyl; in some embodiments, $R^2$ and $R^3$ are each independently selected from H, D, F, hydroxyl, halo $C_{1-2}$ alkyl, deuterated $C_{1-2}$ alkyl or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H, D, or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H, D, or $C_{1-2}$ alkyl; or $R^2$ and $R^3$ together with the carbon atom to which they are attached form $C_{3-4}$ cycloalkyl; in some embodiments, $R^2$ and $R^3$ are each independently selected from H or D;

each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O or $C_{3-5}$ cycloalkyl; in some embodiments, each $R^4$ is independently selected from D, F, Cl, cyano, amino, hydroxyl, -$SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; or two $R^4$ on the same carbon atom together with the carbon atom to which they are attached form =O or $C_{3-4}$ cycloalkyl; in some embodiments, each $R^4$ is independently selected from D, F, Cl, cyano, $C_{1-2}$ alkyl, halo $C_{1-2}$ alkyl or deuterated $C_{1-2}$ alkyl; or two $R^4$ on the same carbon atom together with the carbon atom to which they are attached form 3- to 4-membered cycloalkyl; in some embodiments, each $R^4$ is independently selected from D, halogen, cyano, amino, hydroxyl, -$SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O; in some embodiments, each $R^4$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; or two $R^4$ on the same carbon atom together with the carbon atom to which they are attached form =O; in some embodiments, each $R^4$ is independently selected from D, F, Cl, cyano, amino, hydroxyl, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; or two $R^4$ on the same carbon atom together with the carbon atom to which they are attached form =O; in some embodiments, each $R^4$ is independently selected from D, F, Cl, methyl, ethyl, methoxy, ethoxy, -$CH_2F$, -$CHF_2$, -$CF_3$, -$CH_2CH_2F$, - $CH_2CHF_2$, -$CH_2CF_3$, -$CHFCH_2F$, -$CHFCHF_2$, -$CHFCF_3$, -$CF_2CH_2F$, -$CF_2CHF_2$, - $CF_2CF_3$, -$CH_2D$, -$CHD_2$, -$CD_3$, -$CH_2CH_2D$, -$CH_2CHD_2$, -$CH_2CD_3$, -$CHDCH_2D$, - $CHDCHD_2$, -$CHDCD_3$, -$CD_2CH_2D$, -$CD_2CHD_2$, or -$CD_2CD_3$; or two $R^4$ on the same carbon atom together with the carbon atom to which they are attached form =O;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; in some embodiments, each $R^5$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; in some embodiments, each $R^5$ is independently selected from D, F, Cl, cyano, amino, hydroxyl, $C_{1-2}$ alkyl, $C_{1-2}$ alkoxy, halo $C_{1-2}$ alkyl, halo $C_{1-2}$ alkoxy, deuterated $C_{1-2}$ alkyl, or deuterated $C_{1-2}$ alkoxy; in some embodiments, each $R^5$ is independently selected from D, F, Cl, $C_{1-2}$ alkyl, halo $C_{1-2}$ alkyl, or deuterated $C_{1-2}$ alkyl; in some embodiments, each $R^5$ is independently selected from D, F, Cl, methyl, ethyl, -$CH_2F$, -$CHF_2$, -$CF_3$, -$CH_2CH_2F$, -$CH_2CHF_2$, -$CH_2CF_3$, -$CHFCH_2F$, -$CHFCHF_2$, -$CHFCF_3$, -$CF_2CH_2F$, -$CF_2CHF_2$, -$CF_2CF_3$, -$CH_2D$, -$CHD_2$, -$CD_3$, -$CH_2CH_2D$, -$CH_2CHD_2$, - $CH_2CD_3$, -$CHDCH_2D$, -$CHDCHD_2$, -$CHDCD_3$, -$CD_2CH_2D$, -$CD_2CHD_2$, or - $CD_2CD_3$;

p is selected from 0, 1, 2 or 3; in some embodiments, p is selected from 0, 1 or 2; in some embodiments, p is selected from 0 or 1; in some embodiments, p is selected from 0;

ring B is piperazinyl, and q is selected from 1 or 2; in some embodiments, ring B is piperazinyl, and q is selected from 1; or

ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5-to 10-membered saturated fused heterocycle containing 1-4 nitrogen atoms, or 5- to 11-membered saturated spiro heterocycle containing 1-4 nitrogen atoms, and q is selected from 0, 1, 2 or 3; in some embodiments, ring B is selected from piperidyl; in some embodiments, ring B is selected from 5-membered saturated monocyclic heterocycloalkane containing 1-2 nitrogen atoms, 6-membered saturated

bridged heterocycle containing 1, 2, 3 or 4 nitrogen atoms, 7-membered saturated bridged heterocycle containing 1, 2, 3 or 4 nitrogen atoms, 8-membered saturated bridged heterocycle containing 1, 2, 3 or 4 nitrogen atoms, 8-membered saturated fused heterocycle containing 1, 2, 3 or 4 nitrogen atoms, 9-membered saturated fused heterocycle containing 1, 2, 3 or 4 nitrogen atoms, 10-membered saturated fused heterocycle containing 1, 2, 3 or 4 nitrogen atoms, 7-membered saturated spiro heterocycle containing 1, 2, 3 or 4 nitrogen atoms, 8-membered saturated spiro heterocycle containing 1, 2, 3 or 4 nitrogen atoms, 9-membered saturated spiro heterocycle containing 1, 2, 3 or 4 nitrogen atoms, 10-membered saturated spiro heterocycle containing 1, 2, 3 or 4 nitrogen atoms, or 11-membered saturated spiro heterocycle containing 1, 2, 3 or 4 nitrogen atoms, and q is selected from 0, 1 or 2; in some embodiments, ring B is 6-membered saturated bridged heterocycle containing 1, 2, 3 or 4 nitrogen atoms, 7-membered saturated bridged heterocycle containing 1, 2, 3 or 4 nitrogen atoms, 8-membered saturated bridged heterocycle containing 1, 2, 3 or 4 nitrogen atoms, 7-membered saturated spiro heterocycle containing 1, 2, 3 or 4 nitrogen atoms, 9-membered saturated spiro heterocycle containing 1, 2, 3 or 4 nitrogen atoms, or 11-membered saturated spiro heterocycle containing 1, 2, 3 or 4 nitrogen atoms, and q is selected from 0, 1 or 2; in some embodiments,

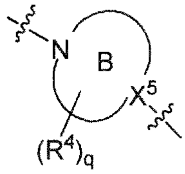

is selected from

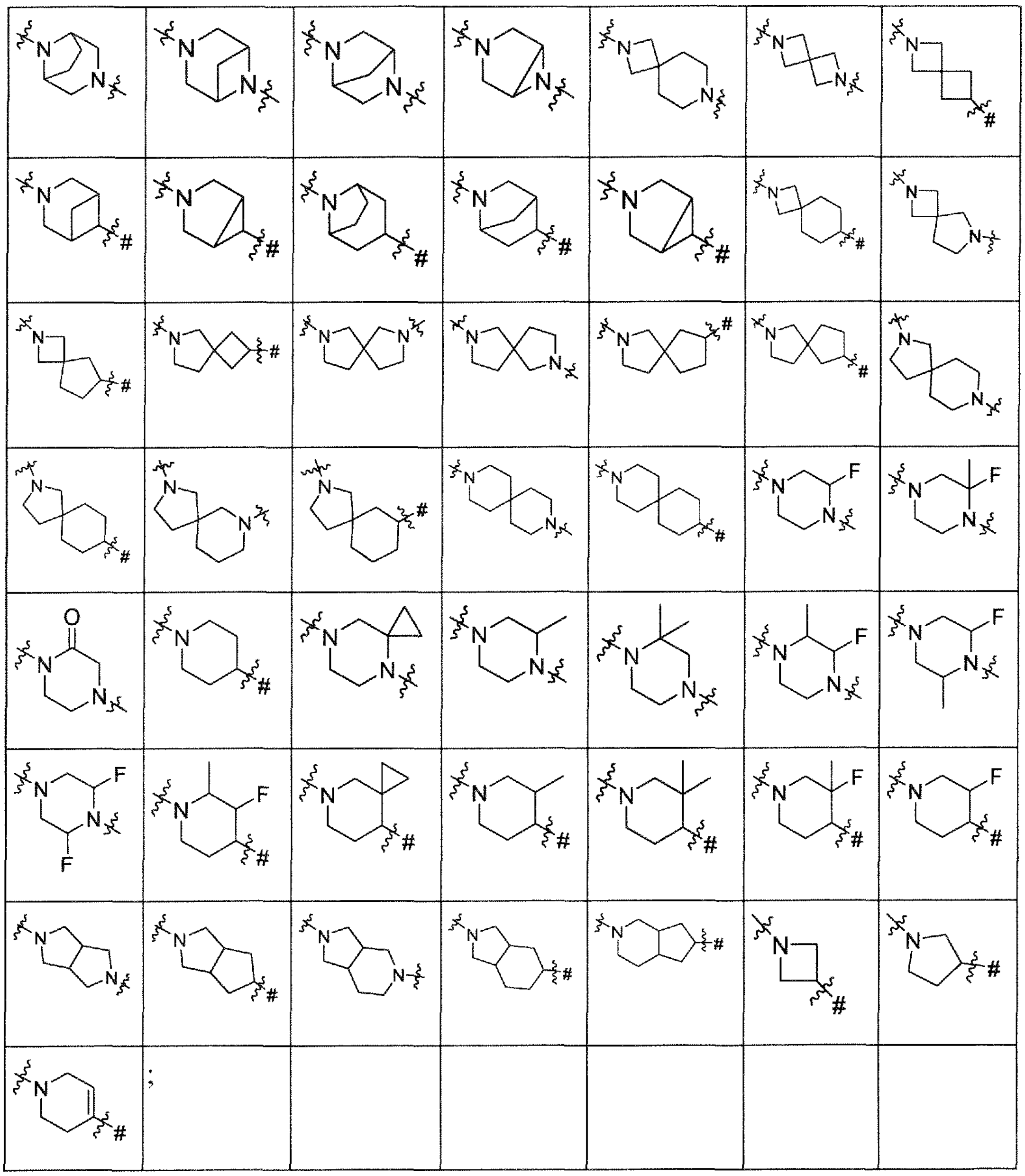

;

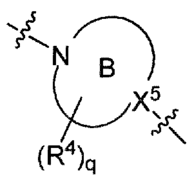

is selected from

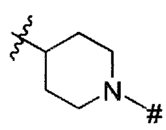 or 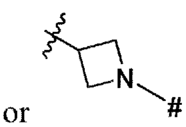;

# represents position $X^5$ in ring B, wherein groups with undefined connection positions can be connected at both ends; $L_A$ is selected from a bond, -NH-, $-NR^{a1}-$, -O-, -S-, -S(=O)-, $-S(=O)_2-$, -NH-C(=O)-, -C(=O)-NH-, $C_{1-6}$ alkyl, $C_{1-6}$

alkoxy, halo $C_{1-6}$ alkyl, halo $C_{1-6}$ alkoxy, deuterated $C_{1-6}$ alkyl or deuterated $C_{1-6}$ alkoxy; in some embodiments, $L_A$ is selected from -NH-, -$NR^{a1}$-, -O-, -S-, -S(=O)-, -$S(=O)_2$-, -NH-C(=O)-, -C(=O)-NH-, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; in some embodiments, $L_A$ is selected from -NH-, -N($C_{1-4}$ alkyl)-, -O-, -S-, -S(=O)-, -$S(=O)_2$- or $C_{1-4}$ alkyl; in some embodiments, in some embodiments, $L_A$ is selected from -NH-, -N($C_{1-2}$ alkyl)-, -O- or -S-; in some embodiments, $L_A$ is selected from -NH-, -N($CH_3$)- or -O-; in some embodiments, $L_A$ is selected from -NH- or -N($CH_3$)-; in some embodiments, $L_A$ is selected from -O-;

ring A is selected from 5- to 6-membered monocyclic heteroaromatic ring containing 1, 2, 3, 4 or 5 nitrogen, oxygen or sulphur atoms, wherein the heteroaromatic ring is further substituted with 1-3 substituents selected from $R_a$; in some embodiments, ring A is selected from 5-membered monocyclic heteroaromatic ring containing 1, 2, 3, 4 or 5 nitrogen, oxygen or sulphur atoms, or 6-membered monocyclic heteroaromatic ring containing 1, 2, 3, 4 or 5 nitrogen, oxygen or sulphur atoms, wherein the heteroaromatic ring is further substituted with 1, 2 or 3 substituents selected from $R_a$; in some embodiments, ring A is selected from 6-membered monocyclic heteroaromatic ring containing 1, 2, 3, 4 or 5 nitrogen, oxygen or sulphur atoms, wherein the heteroaromatic ring is further substituted with 1 substituent selected from $R_a$; in some embodiments, ring A is selected from 6-membered monocyclic heteroaromatic ring containing 1 or 2 nitrogen atoms, wherein the heteroaromatic ring is further substituted with 1 substituent selected from $R_a$; in some embodiments, ring A is selected from 5-membered monocyclic heteroaromatic ring containing 2, 3, 4 or 5 nitrogen, oxygen or sulphur atoms, wherein the heteroaromatic ring is further substituted with 1 substituent selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$; in some embodiments, ring A is selected from 8- to 10-membered bicyclic fused heteroaromatic ring containing 1, 2, 3, 4 or 5 nitrogen, oxygen or sulphur atoms, or 8- to 10-membered bicyclic fused aromatic ring, wherein the heteroaromatic ring or aromatic ring is optionally further substituted with 1, 2 or 3 substituents selected from $R_b$; in some embodiments, ring A is selected from 8-membered bicyclic fused heteroaromatic ring containing 1, 2, 3, 4 or 5 nitrogen, oxygen or sulphur atoms, 9-membered bicyclic fused heteroaromatic ring containing 1, 2, 3, 4 or 5 nitrogen, oxygen or sulphur atoms, 10-membered bicyclic fused heteroaromatic ring containing 1, 2, 3, 4 or 5 nitrogen, oxygen or sulphur atoms, 8-membered bicyclic fused aromatic ring, 9-membered bicyclic fused aromatic ring, or 10-membered bicyclic fused aromatic ring, wherein the heteroaromatic ring or aromatic ring is optionally further substituted with 1 substituent selected from $R_b$; in some embodiments, ring A is selected from 8-membered bicyclic fused heteroaromatic ring containing 1, 2, 3, 4 or 5 nitrogen, oxygen or sulphur atoms, 9-membered bicyclic fused heteroaromatic ring containing 1, 2, 3, 4 or 5 nitrogen, oxygen or sulphur atoms, 10-membered bicyclic fused heteroaromatic ring containing 1, 2, 3, 4 or 5 nitrogen, oxygen or sulphur atoms, 8-membered bicyclic fused aromatic ring, 9-membered bicyclic fused aromatic ring, or 10-membered bicyclic fused aromatic ring, wherein the heteroaromatic ring or aromatic ring is optionally further substituted with 1 substituent selected from $R_b$;

each $R_a$ is independently selected from -C(O)N($R^{a1}$)$_2$, -$NR^{a1}$C(O)O$R^{a1}$, - $NR^{a1}$C(O)N($R^{a1}$)$_2$, -C(=S)N($R^{a1}$)$_2$, -$S(O)_2$N($R^{a1}$)$_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, -NH$C_{1-6}$ alkyl, -N($C_{1-6}$ alkyl)$_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy; in some embodiments, each $R_a$ is independently selected from -C(O)N($R^{a1}$)$_2$, - $NR^{a1}$C(O)O$R^{a1}$, -$NR^{a1}$C(O)N($R^{a1}$)2, -C(=S)N($R^{a1}$)$_2$, -$S(O)_2$N($R^{a1}$)$_2$, 5-membered monocyclic heteroaryl containing 1, 2, 3, 4 or 5 nitrogen, oxygen or sulphur atoms, 6-membered monocyclic heteroaryl containing 1, 2, 3, 4 or 5 nitrogen, oxygen or sulphur atoms, 4- to 7-membered monocyclic heterocycloalkyl containing 1, 2, 3 or 4 nitrogen, oxygen or sulphur atoms, or 3- to 7-membered monocyclic cycloalkyl, wherein the heteroaryl, heterocycloalkyl, or cycloalkyl is optionally further substituted with 1, 2 or 3 substituents selected from D, halogen, cyano, hydroxyl, amino, -NH$C_{1-2}$ alkyl, -N($C_{1-2}$ alkyl)$_2$, $C_{1-2}$ alkyl, halo $C_{1-2}$ alkyl, $C_{1-2}$ alkoxy, halo $C_{1-2}$ alkoxy, deuterated $C_{1-2}$ alkyl or deuterated $C_{1-2}$ alkoxy; in some embodiments, each $R_a$ is independently selected from -C(O)N($R^{a1}$)$_2$, -$NR^{a1}$C(O)O$R^{a1}$, - $NR^{a1}$C(O)N($R^{a1}$)$_2$, -C(=S)N($R^{a1}$)$_2$, -$S(O)_2$N($R^{a1}$)$_2$, 5-membered monocyclic heteroaryl containing 1, 2, 3 or 4 nitrogen, oxygen or sulphur atoms, 4-membered monocyclic heterocycloalkyl containing 1 or 2 nitrogen, oxygen or sulphur atoms, 5-membered monocyclic heterocycloalkyl containing 1 or 2 nitrogen, oxygen or sulphur atoms, 6-membered monocyclic heterocycloalkyl containing 1 or 2 nitrogen, oxygen or sulphur atoms, 4-membered monocyclic cycloalkyl, 5-membered monocyclic cycloalkyl, or 6-membered monocyclic cycloalkyl, wherein the heteroaryl, heterocycloalkyl, or cycloalkyl is optionally further substituted with 1, 2 or 3 substituents selected from D, halogen, cyano, hydroxyl, amino, -$NHCH_3$, -$NHCH_2CH_3$, -N($CH_3$)$_2$, methyl, ethyl, methoxy, ethoxy, -$CH_2F$, -$CHF_2$, -CF3, - $CH_2CH_2F$, -$CH_2CHF_2$, -$CH_2CF_3$, -$CHFCH_2F$, -$CHFCHF_2$, -$CHFCF_3$, -$CF_2CH_2F$, - $CF_2CHF_2$, -$CF_2CF_3$, -$OCHF_2$, -$OCH_2F$, -$OCF_3$, -$OCH_2CH_2F$, -$OCH_2CHF_2$, - $OCH_2CF_3$, -$OCHFCH_2F$, -$OCHFCHF_2$, -$OCHFCF_3$, -$OCF_2CH_2F$, -$OCF_2CHF_2$, - $OCF_2CF_3$, -$CH_2D$,

$-CHD_2$, $-CD_3$, $-CH_2CH_2D$, $-CH_2CHD_2$, $-CH_2CD_3$, $-CHDCH_2D$, - $CHDCHD_2$, $-CHDCD_3$, $-CD_2CH_2D$, $-CD_2CHD_2$, $-CD_2CD_3$, $-OCHD_2$, $-OCH_2D$, - $OCD_3$, $-OCH_2CH_2D$, $-OCH_2CHD_2$, $-OCH_2CD_3$, $-OCHDCH_2D$, $-OCHDCHD_2$, - $OCHDCD_3$, $-OCD_2CH_2D$, $-OCD_2CHD_2$, or $-OCD_2CD_3$;

each $R_b$ is independently selected from $-C(O)N(R^{a1})_2$, $-NR^{a1}C(O)OR^{a1}$, - $NR^{a1}C(O)N(R^{a1})_2$, $-C(=S)N(R^{a1})_2$, $-S(O)_2N(R^{a1})_2$, =O, D, halogen, cyano, hydroxyl, amino, $-NHC_{1-6}$ alkyl, $-N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy; in some embodiments, each $R_b$ is independently selected from $-C(O)N(R^{a1})_2$, - $NR^{a1}C(O)OR^{a1}$, $-NR^{a1}C(O)N(R^{a1})_2$, $-C(=S)N(R^{a1})_2$, $-S(O)_2N(R^{a1})_2$, =O, D, halogen, cyano, hydroxyl, amino, $-NHC_{1-2}$ alkyl, $-N(C_{1-2}$ alkyl$)_2$, $C_{1-2}$ alkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl or deuterated $C_{1-2}$ alkoxy; in some embodiments, each $R_b$ is independently selected from $-C(O)N(R^{a1})_2$, =O, D, halogen, cyano, hydroxyl, amino, $-NHC_{1-2}$ alkyl, $-N(C_{1-2}$ alkyl$)_2$, $C_{1-2}$ alkyl, $C_{1-2}$ alkoxy, $C_{1-2}$ alkyl-O-$C_{1-2}$ alkyl, halo $C_{1-2}$ alkyl, halo $C_{1-2}$ alkoxy, deuterated $C_{1-2}$ alkyl or deuterated $C_{1-2}$ alkoxy; in some embodiments, each $R_b$ is independently selected from $-C(O)N(R^{a1})_2$, =O, D, halogen, cyano, hydroxyl, amino, $-NHC_{1-2}$ alkyl, $-N(C_{1-2}$ alkyl$)_2$, $C_{1-2}$ alkyl, or halo $C_{1-2}$ alkyl; in some embodiments, each $R_b$ is independently selected from $-C(O)N(R^{a1})_2$, =O, D, halogen, cyano, hydroxyl, amino, $-NHCH_3$, $-NHCH_2CH_3$, $-N(CH_3)_2$, methyl, ethyl, $-CH_2F$, - $CHF_2$, $-CF_3$, $-CH_2CH_2F$, $-CH_2CHF_2$, $-CH_2CF_3$, $-CHFCH_2F$, $-CHFCHF_2$, or - $CHFCF_3$;

each $R^{a1}$ is independently selected from H, D, $C_{1-6}$ alkyl, $C_{3-12}$ cycloalkyl, 3- to 12-membered heterocycloalkyl, 5- to 8-membered heteroaryl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy, wherein the cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy; in some embodiments, each $R^{a1}$ is independently selected from H, D, $C_{1-4}$ alkyl, $C_{3-6}$ monocyclic cycloalkyl, $C_{5-11}$ bicyclic spiro cycloalkyl, $C_{6-8}$ bicyclic bridged cycloalkyl, $C_{7-10}$ bicyclic fused cycloalkyl, 4- to 6-membered heterocycloalkyl, 6- to 9-membered bicyclic spiro heterocycloalkyl, $C_{6-8}$ bicyclic bridged heterocycloalkyl, $C_{7-10}$ bicyclic fused heterocycloalkyl, 5- to 6-membered heteroaryl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy, wherein the cycloalkyl, heterocycloalkyl, or heteroaryl is optionally further substituted with 1-3 substituents selected from D, halogen, $C_{1-4}$ alkyl, halo $C_{1-4}$ alkyl, $C_{1-4}$ alkoxy, halo $C_{1-4}$ alkoxy, deuterated $C_{1-4}$ alkyl or deuterated $C_{1-4}$ alkoxy; in some embodiments, each $R^{a1}$ is independently selected from $C_{1-4}$ alkyl, $C_{3-6}$ monocyclic cycloalkyl, $C_{5-7}$ bicyclic spiro cycloalkyl, 4- to 6-membered heterocycloalkyl, 5- to 6-membered heteroaryl, $C_{1-2}$ alkyl-O-$C_{1-2}$ alkyl, halo $C_{1-4}$ alkyl or deuterated $C_{1-4}$ alkyl, wherein the cycloalkyl, heterocycloalkyl, or heteroaryl is optionally further substituted with 1-3 substituents selected from D, halogen, $C_{1-4}$ alkyl, halo $C_{1-4}$ alkyl, or deuterated $C_{1-4}$ alkyl; in some embodiments, each $R^{a1}$ is independently selected from $C_{1-4}$ alkyl, $C_{3-4}$ monocyclic cycloalkyl, 5-membered heteroaryl, $C_{1-2}$ alkyl-O-$C_{1-2}$ alkyl, halo $C_{1-4}$ alkyl or deuterated $C_{1-4}$ alkyl, wherein the cycloalkyl or heteroaryl is optionally further substituted with 1-3 substituents selected from D, halogen, or $C_{1-4}$ alkyl; in some embodiments, each $R^{a1}$ is independently selected from methyl, ethyl, propyl, isopropyl, tert-butyl, cyclopropyl, cyclobutyl, 5-membered heteroaryl, methoxymethyl, ethoxymethyl, methoxyethyl, ethoxyethyl, $-CH_2F$, $-CHF_2$, $-CF_3$, - $CH_2CH_2F$, $-CH_2CHF_2$, $-CH_2CF_3$, $-CHFCH_2F$, $-CHFCHF_2$, $-CHFCF_3$, $-CF_2CH_2F$, - $CF_2CHF_2$, $-CF_2CF_3$, $-CH_2CH_2CH_2F$, $-CH_2CH_2CHF_2$, or $-CH_2CH_2CF_3$, wherein the cyclopropyl, cyclobutyl, or 5-membered heteroaryl is optionally further substituted with 1-3 substituents selected from F, Cl, methyl, ethyl, or propyl; or

two $R^{a1}$ together with the nitrogen atom form 4- to 6-membered heterocycloalkyl; in some embodiments, two $R^{a1}$ together with the nitrogen atom form 4-, 5- or 6-membered heterocycloalkyl; in some embodiments, two $R^{a1}$ together with the nitrogen atom form 5-membered heterocycloalkyl;

alternatively, $L_A$ is selected from a bond, and the carbon atom to which $R^3$ is attached and the linking site of ring B directly form a double bond;

unless otherwise specified, the above-mentioned heterocycloalkane, heterocycloalkyl, heteroaryl, or heteroaromatic ring contains 1, 2, 3, 4 or 5 heteroatoms selected from nitrogen, oxygen or sulphur; in some embodiments, the heterocycloalkane, heterocycloalkyl, heteroaryl, or heteroaromatic ring contains 1, 2, 3 or 4 heteroatoms selected from nitrogen, oxygen or sulphur; the heterocycloalkane, heterocycloalkyl, heteroaryl, or heteroaromatic ring contains 1, 2 or 3 heteroatoms selected from nitrogen, oxygen or sulphur; the heterocycloalkane, heterocycloalkyl, heteroaryl, or heteroaromatic ring contains 1 or 2 heteroatoms selected from nitrogen, oxygen or sulphur.

Specifically, the first technical solution of the present invention relates to a compound represented by formula (I), (1-1) or (1-2), or a stereoisomer, solvate, or pharmaceutically acceptable salt thereof,

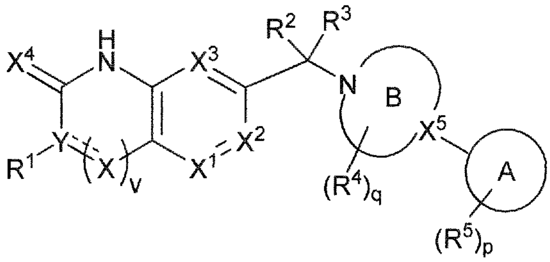

(I)

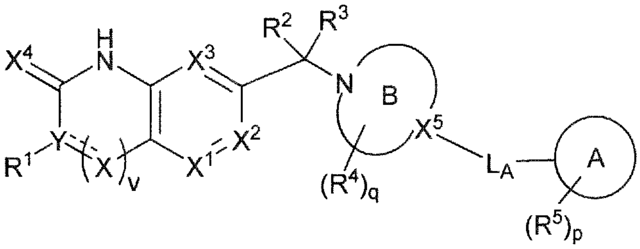

(I-1)

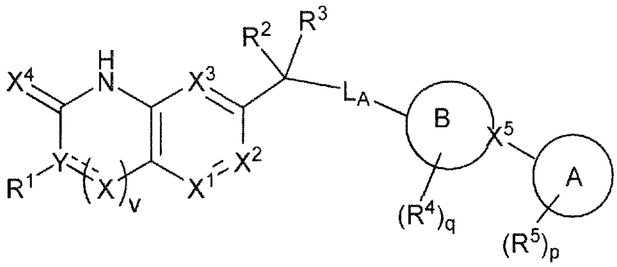

(I-2)

wherein each X is independently selected from $CR^x$, $C(R^x)_2$, O, N or $NR^x$;

Y is selected from N, C or CH;

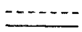

represents a single bond or a double bond, provided that when



represents a single bond, X is selected from $C(R^x)_2$, O or $NR^x$;
v is selected from 1, 2 or 3;
$X^1$, $X^2$ and $X^3$ are each independently selected from N or $CR^x$;
$X^4$ is selected from O or S;
$X^5$ is independently selected from N, C or $CR^x$; further, $X^5$ is independently selected from N or $CR^x$;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3; p is selected from 0, 1, 2 or 3;
$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;

ring B is piperazinyl, and q is selected from 1 or 2; or

ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5-to 10-membered saturated fused heterocycle containing 1-4 nitrogen atoms, or 5- to 11-membered saturated spiro heterocycle containing 1-4 nitrogen atoms, and q is selected from 0, 1, 2 or 3;

each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O or $C_{3-5}$ cycloalkyl;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

$L_A$ is selected from a bond, -NH-, -$NR^{a1}$-, -O-, -S-, -S(=O)-, -$S(=O)_2$-, -NH-C(=O)-, -C(=O)-NH-, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

each $R_a$ is independently selected from -$C(O)N(R^{a1})_2$, -$NR^{a1}C(O)OR^{a1}$, - $NR^{a1}C(O)R^{a1}$, -$NR^{a1}C(O)N(R^{a1})_2$, -$C(=S)N(R^{a1})_2$, -$S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, -$NHC_{1-6}$ alkyl, -$N(C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;

each $R_b$ is independently selected from -$C(O)N(R^{a1})_2$, -$NR^{a1}C(O)OR^{a1}$, - $NR^{a1}C(O)N(R^{a1})_2$, -$C(=S)N(R^{a1})_2$, -$S(O)_2N(R^{a1})_2$, =O, D, halogen, cyano, hydroxyl, amino, -$NHC_{1-6}$ alkyl, -$N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

each $R^{a1}$ is independently selected from H, D, $C_{1-6}$ alkyl, $C_{3-12}$ cycloalkyl, 3- to 12-membered heterocycloalkyl, 5- to 8-membered heteroaryl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy, wherein the cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy; or two $R^{a1}$ together with the nitrogen atom form 4- to 6-membered heterocycloalkyl;

alternatively, $L_A$ is selected from a bond, and the carbon atom to which $R^3$ is attached and the linking site of ring B directly form a double bond;

unless otherwise specified, the above-mentioned heterocycloalkane, heterocycloalkyl, heteroaryl, or heteroaromatic ring contains 1-5 heteroatoms selected from nitrogen, oxygen or sulphur.

Specifically, the second technical solution of the present invention relates to a compound represented by formula (I), (1-1) or (1-2), or a stereoisomer, solvate, or pharmaceutically acceptable salt thereof,

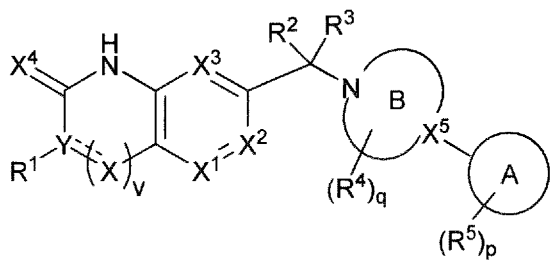

(I)

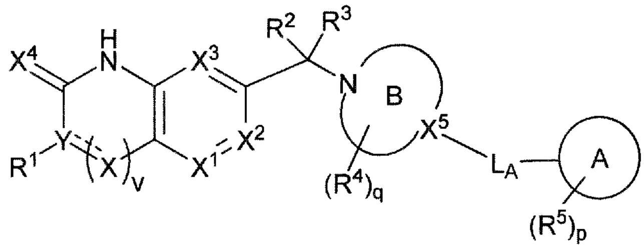

(I-1)

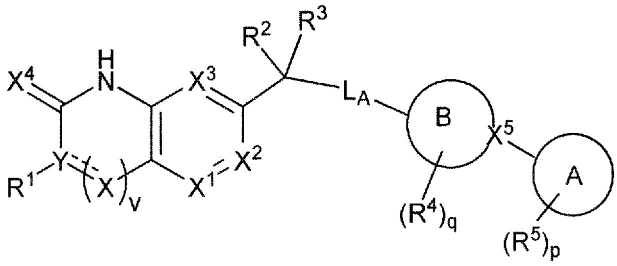

(I-2)

wherein each X is independently selected from $CR^x$, $C(R^x)_2$, O, N or $NR^x$;

Y is selected from N, C or CH;

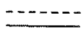

represents a single bond or a double bond, provided that when



represents a single bond, X is selected from $C(R^x)_2$, O or $NR^x$;
v is selected from 1, 2 or 3;
$X^1$, $X^2$ and $X^3$ are each independently selected from N or $CR^x$;
$X^4$ is selected from O or S;
$X^5$ is independently selected from N, C or $CR^x$; further, $X^3$ is independently selected from N or $CR^x$;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3; p is selected from 0, 1, 2 or 3;
$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;
ring B is piperazinyl, and q is selected from 1 or 2; or
ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5-to 10-membered saturated fused heterocycle containing 1-4 nitrogen atoms, or 5- to 11-membered saturated spiro heterocycle containing 1-4 nitrogen atoms, and q is selected from 0, 1, 2 or 3;
each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together

with the carbon atom to which they are attached form =O; or two $R^4$ on the same carbon atom together with the carbon atom to which they are attached form $C_{3-5}$ cycloalkyl;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

$L_A$ is selected from -NH-, -$NR^{a1}$-, -O-, -S-, -S(=O)-, -$S(=O)_2$-, -NH-C(=O)-, - C(=O)-NH-, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or

ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

each $R_a$ is independently selected from -$C(O)N(R^{a1})_2$, -$NR^{a1}C(O)OR^{a1}$, - $NR^{a1}C(O)R^{a1}$, -$NR^{a1}C(O)N(R^{a1})_2$, -$C(=S)N(R^{a1})_2$, -$S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, -$NHC_{1-6}$ alkyl, -$N(C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;

each $R_b$ is independently selected from -$C(O)N(R^{a1})_2$, -$NR^{a1}C(O)OR^{a1}$, - $NR^{a1}C(O)N(R^{a1})_2$, -$C(=S)N(R^{a1})_2$, -$S(O)_2N(R^{a1})_2$, =O, D, halogen, cyano, hydroxyl, amino, -$NHC_{1-6}$ alkyl, -$N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

each $R^{a1}$ is independently selected from H, D, $C_{1-6}$ alkyl, $C_{3-12}$ cycloalkyl, 3- to 12-membered heterocycloalkyl, 5- to 8-membered heteroaryl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy, wherein the cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;

unless otherwise specified, the above-mentioned heterocycloalkane, heterocycloalkyl, heteroaryl, or heteroaromatic ring contains 1-5 heteroatoms selected from nitrogen, oxygen or sulphur.

Specifically, the third technical solution of the present invention relates to a compound represented by formula (1) or (1-1), or a stereoisomer, solvate, or pharmaceutically acceptable salt thereof,

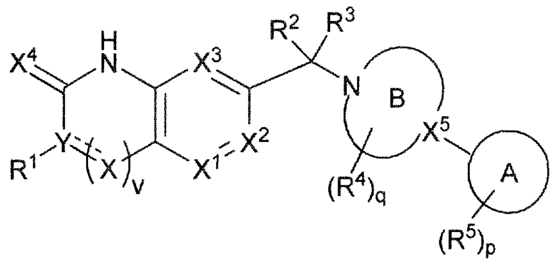

(I)

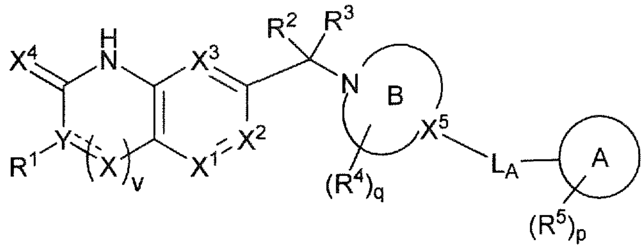

(I-1)

wherein each X is independently selected from $CR^x$, $C(R^x)_2$, O, N or $NR^x$;

Y is selected from N, C or CH;

represents a single bond or a double bond, provided that when



represents a single bond, X is selected from $C(R^x)_2$, O or $NR^x$;
v is selected from 1, 2 or 3;
$X^1$, $X^2$ and $X^3$ are each independently selected from N or $CR^x$;
$X^4$ is selected from O or S;
$X^5$ is independently selected from N or $CR^x$;
each $R^x$ is independently selected from H, D, halogen, cyano, amino, hydroxyl, -SFs, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r-$(3- to 12-membered heterocycloalkyl); or two $R^x$ on the same carbon atom together form =O;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r-$(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3; p is selected from 0, 1, 2 or 3;
$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;
each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O;
each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;
ring B is piperazinyl, and q is selected from 1 or 2; or
ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5-to 10-membered saturated fused heterocycle containing 1-4 nitrogen atoms, or 5- to 11-membered saturated spiro heterocycle containing 1-4 nitrogen atoms, and q is selected from 0, 1, 2 or 3;
$L_A$ is selected from -NH-, -O-, -S-, -S(=O)-, $-S(=O)_2-$, -NH-C(=O)-, -C(=O)-NH-, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;
ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or
ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;
$R_a$ is selected from $-C(O)N(R^{a1})_2$, $-NR^{a1}C(O)OR^{a1}$, $-NR^{a1}C(O)N(R^{a1})_2$, - $C(=S)N(R^{a1})_2$, $-S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-6}$ alkyl, $-N(C_{1-6}\ alkyl)_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;
$R_b$ is selected from $-C(O)N(R^{a1})_2$, $-NR^{a1}C(O)OR^{a1}$, $-NR^{a1}C(O)N(R^{a1})_2$, - $C(=S)N(R^{al})_2$, $-S(O)_2N(R^{a1})_2$, =O, D, halogen, cyano, hydroxyl, amino, $-NHC_{1-6}$ alkyl, $-N(C_{1-6}\ alkyl)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;
each $R^{a1}$ is independently selected from H, D, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;
unless otherwise specified, the above-mentioned heterocycloalkane, heterocycloalkyl, heteroaryl, or heteroaromatic ring contains 1-5 heteroatoms selected from nitrogen, oxygen or sulphur.

Specifically, the fourth technical solution of the present invention relates to a compound represented by formula

(1), or a stereoisomer, solvate, or pharmaceutically acceptable salt thereof,

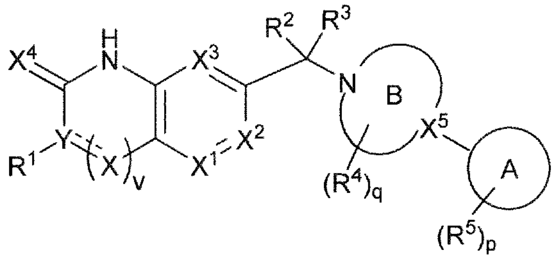

(I)

wherein each X is independently selected from $CR^x$, $C(R^x)_2$, O, N or $NR^x$;

Y is selected from N, C or CH;

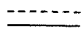

represents a single bond or a double bond, provided that when



represents a single bond, X is selected from $C(R^x)_2$, O or $NR^x$;
v is selected from 1, 2 or 3;
$X^1$, $X^2$ and $X^3$ are each independently selected from N or $CR^x$;
$X^4$ is selected from O or S;
$X^5$ is independently selected from N or $CR^x$;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O;
$R^1$ is selected from halogen, nitro, cyano, amino, hydroxyl, -SFs, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3; p is selected from 0, 1, 2 or 3;
$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;
each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O;
each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;
ring B is piperazinyl, and q is selected from 1 or 2; or
ring B is selected from piperidyl, 5-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5- to 10-membered saturated fused heterocycle containing 1-4 nitrogen atoms, or 5- to 11-membered saturated spiro heterocycle containing 1-4 nitrogen atoms, and q is selected from 0, 1, 2 or 3;
ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or
ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;
each $R_a$ is independently selected from $-C(O)N(R^{a1})_2$, $-NR^{a1}C(O)OR^{a1}$, - $NR^{a1}C(O)N(R^{a1})_2$, $-C(=S)N(R^{a1})_2$,

$-S(O)_2N(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-6}$ alkyl, $-N(C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;

each $R_b$ is independently selected from $-C(O)N(R^{a1})_2$, $-NR^{a1}C(O)OR^{a1}$, - $NR^{a1}C(O)N(R^{a1})_2$, $-C(=S)N(R^{a1})_2$, $-S(O)_2N(R^{a1})_2$, =O, D, halogen, cyano, hydroxyl, amino, $-NHC_{1-6}$ alkyl, $-N(C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

each $R^{a1}$ is independently selected from H, D, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

unless otherwise specified, the above-mentioned heterocycloalkane, heterocycloalkyl, heteroaryl, or heteroaromatic ring contains 1-5 heteroatoms selected from nitrogen, oxygen or sulphur.

The fifth technical solution of the present invention relates to the compound represented by formula (I), (1-1) or (1-2), or the stereoisomer, solvate, or pharmaceutically acceptable salt thereof according to the present invention, having a structure of formula (II), (11-a), (11-b), (III), (III-a), (III-b), (IV), (IV-a), (IV-b) or (V):

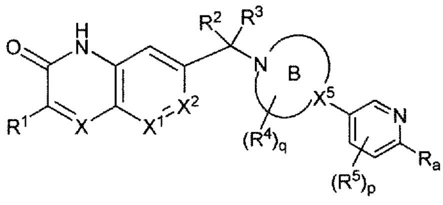
(II)

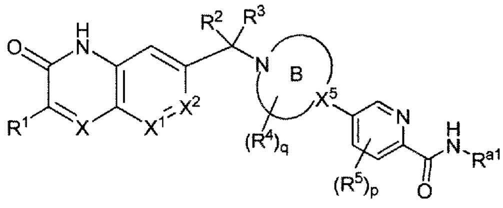
(II-a)

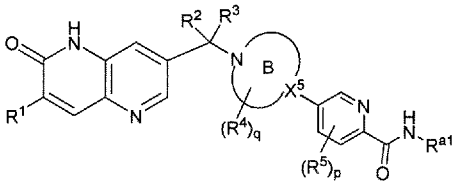
(II-b)

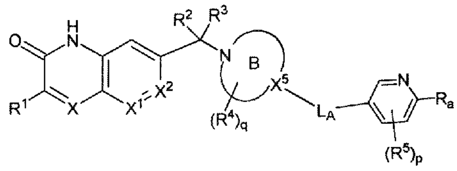
(III)

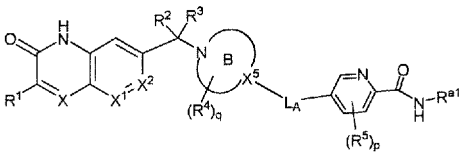
(III-a)

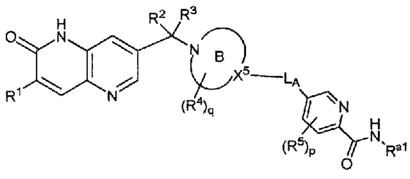
(III-b)

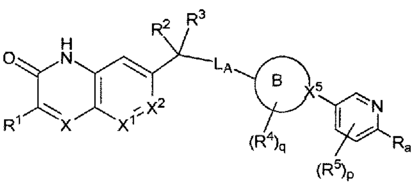
(IV)

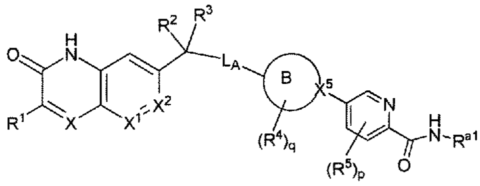
(IV-a)

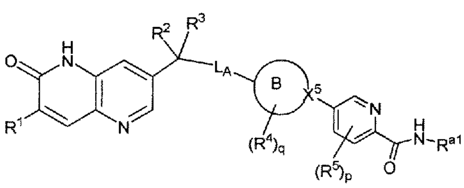
(IV-b)

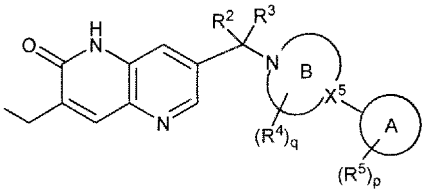
(V)

wherein X is selected from $CR^x$ or N;

$R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl or $-(CH_2)_r-$(4- to 6-membered monocyclic heterocycloalkyl);

$X^5$ is independently selected from N, C or CH; further, $X^5$ is independently selected from N or CH;

$L_A$ is selected from a bond, -NH-, -N($C_{1-4}$ alkyl)-, -O-, -S-, -S(=O)-, $-S(=O)_2-$ or $C_{1-4}$ alkyl;

alternatively, $L_A$ is selected from a bond, and the carbon atom to which $R^3$ is attached and the linking site of ring B directly form a double bond;

other groups are consistent with those mentioned above.

The sixth technical solution of the present invention relates to the compound represented by formula (I), (1-1) or (1-2), or the stereoisomer, solvate, or pharmaceutically acceptable salt thereof according to the present invention, having a structure of formula (II), (II-a), (11-b), (III), (III-a), (III-b), (IV), (IV-a) or (IV-b):

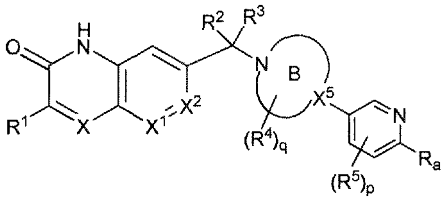
(II)

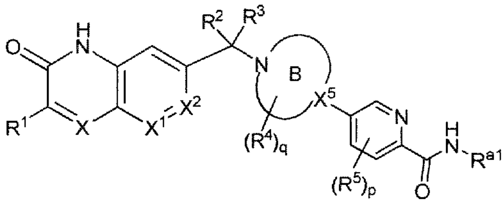
(II-a)

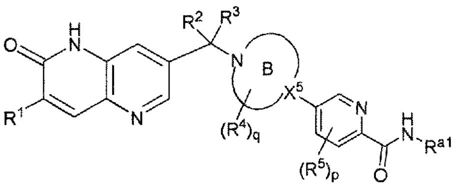
(II-b)

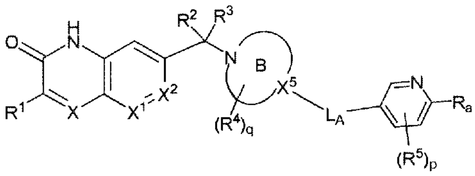
(III)

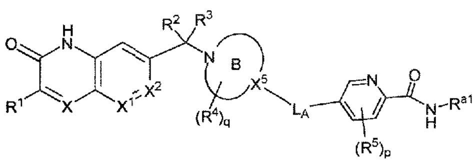
(III-a)

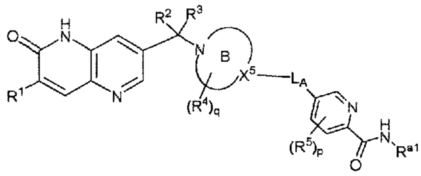
(III-b)

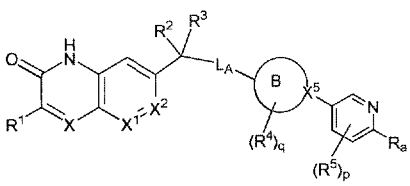
(IV)

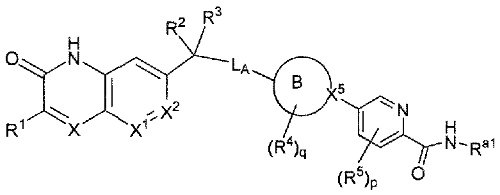
(IV-a)

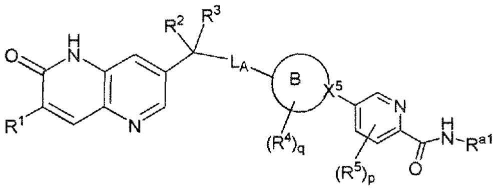
(IV-b)

wherein X is selected from $CR^x$ or N;

$R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl or $-(CH_2)_r-$(4- to 6-membered monocyclic heterocycloalkyl);

$X^5$ is independently selected from N or CH;

$L_A$ is selected from -NH-, -N($C_{1-4}$ alkyl)-, -O-, -S-, -S(=O)-, -S(=O)$_2$- or $C_{1-4}$ alkyl;

other groups are consistent with those mentioned above.

The seventh technical solution of the present invention relates to the compound represented by formula (I), (1-1) or (I-2), or the stereoisomer, solvate, or pharmaceutically acceptable salt thereof according to the present invention, having a structure of formula (II), (11-a), (II-b), (III), (III-a) or (III-b):

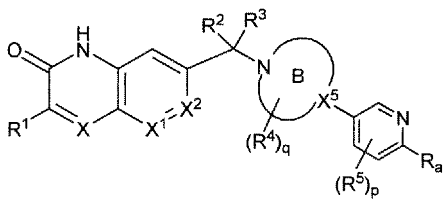
(II)

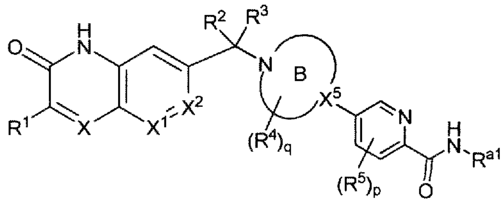
(II-a)

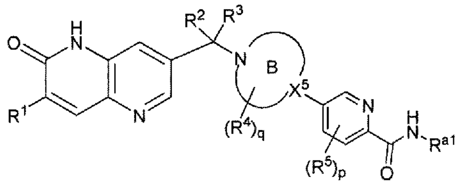
(II-b)

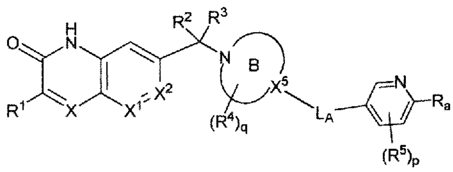
(III)

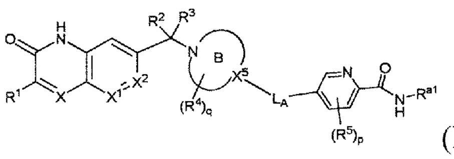
(III-a)

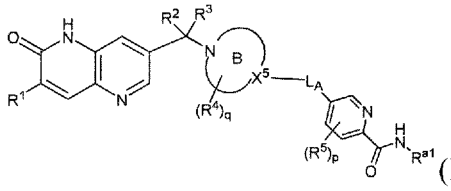
(III-b)

wherein X is selected from $CR^x$ or N;

$R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, -$(CH_2)_r$-$C_{3-6}$ monocyclic cycloalkyl or -$(CH_2)_r$-(4- to 6-membered monocyclic heterocycloalkyl);

$X^5$ is independently selected from N or CH;

$L_A$ is selected from -NH-, -O-, -S-, -S(=O)-, -S(=O)$_2$- or $C_{1-4}$ alkyl;

other groups are consistent with those mentioned above.

The eighth technical solution of the present invention relates to the compound represented by formula (I), or the stereoisomer, solvate, or pharmaceutically acceptable salt thereof according to the present invention, having a structure of formula (II), (II-a) or (II-b):

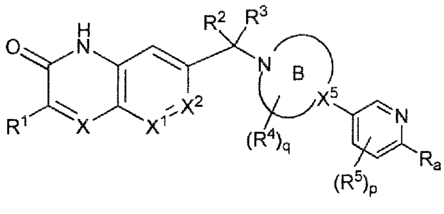
(II)

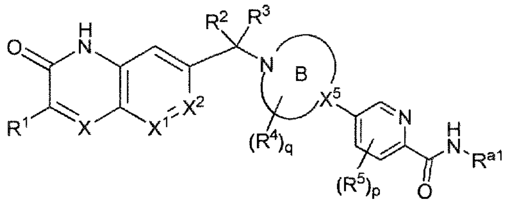
(II-a)

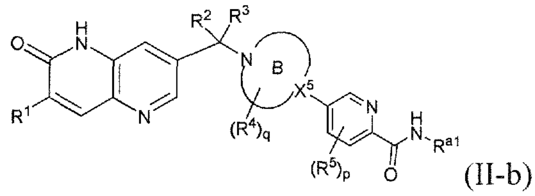

(II-b)

wherein X is selected from $CR^x$ or N;

$R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl or $-(CH_2)_r-$(4- to 6-membered monocyclic heterocycloalkyl);
$X^5$ is independently selected from N or CH;
other groups are consistent with those mentioned above.

The ninth technical solution of the present invention relates to the compound represented by formula (1), (1-1), (1-2), (II), (II-a), (II-b), (III), (III-a), (III-b), (IV), (IV-a), (IV-b), (V), (VI) or (VII), or the stereoisomer, solvate, or pharmaceutically acceptable salt thereof according to the present invention, wherein

X and $X_2$ are selected from C;
$X_1$ is selected from N;
$R^1$ is selected from ethyl;
$L_A$ is selected from a bond, -NH- or $-N(C_{1-2}$ alkyl)-;
alternatively, $L_A$ is selected from a bond, and the carbon atom to which $R^3$ is attached and the linking site of ring B directly form a double bond;
$R^2$ and $R^3$ are each independently selected from H, D, F, Cl, deuterated $C_{1-2}$ alkyl or $C_{1-2}$ alkyl; or $R^2$ and $R^3$ together with the carbon atom to which they are attached form $C_{3-4}$ cycloalkyl;
each $R^4$ is independently selected from D, F, Cl, cyano, amino, hydroxyl, $-SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; or two $R^4$ on the same carbon atom together with the carbon atom to which they are attached form =O or $C_{3-4}$ cycloalkyl;
each $R^5$ is independently selected from D, F, Cl, cyano, amino, hydroxyl, $-SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy;
ring A is selected from 7- to 10-membered bicyclic heteroaromatic ring containing 1-5 nitrogen, oxygen or sulphur atoms, wherein the heteroaromatic ring is optionally further substituted with 1-3 substituents selected from D, F, Cl, cyano, hydroxyl, amino, $C_{1-2}$ alkyl, $C_{3-4}$ cycloalkyl, 4- to 5-membered heterocycloalkyl, halo $C_{1-2}$ alkyl, halo $C_{1-2}$ alkoxy, deuterated $C_{1-2}$ alkyl or deuterated $C_{1-2}$ alkoxy;
$R_a$ is selected from $-C(O)N(R^{a1})_2$, $-NR^{a1}C(O)R^{a1}$, or 5- to 6-membered monocyclic heteroaryl containing 1-5 nitrogen, oxygen or sulphur atoms, wherein the heteroaryl is optionally further substituted with 1-3 substituents selected from D, F, Cl, cyano, hydroxyl, amino, $C_{1-2}$ alkyl, halo $C_{1-2}$ alkyl, $C_{1-2}$ alkoxy, halo $C_{1-2}$ alkoxy, deuterated $C_{1-2}$ alkyl or deuterated $C_{1-2}$ alkoxy;
each $R^{a1}$ is independently selected from H, D, $C_{1-2}$ alkyl, $C_{3-5}$ cycloalkyl, 4- to 5-membered heterocycloalkyl, 5- to 6-membered heteroaryl, halo $C_{1-2}$ alkyl, or deuterated $C_{1-2}$ alkyl, wherein the cycloalkyl, heterocycloalkyl, or heteroaryl is optionally further substituted with 1-3 substituents selected from D, F, Cl, cyano, hydroxyl, amino, $C_{1-2}$ alkyl, deuterated $C_{1-2}$ alkyl or deuterated $C_{1-2}$ alkoxy;
other groups are consistent with those mentioned above.

The tenth technical solution of the present invention relates to the compound represented by formula (1), (1-1), (1-2), (II), (II-a), (II-b), (III), (III-a), (III-b), (IV), (IV-a), (IV-b), (V), (VI) or (VII), or the stereoisomer, solvate, or pharmaceutically acceptable salt thereof according to the present invention, wherein

ring B is piperazinyl, and q is selected from 1 or 2; or
ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6-membered saturated bridged heterocycle containing 1-2 nitrogen atoms, 7-membered saturated bridged heterocycle containing 1-2 nitrogen atoms, 8-membered saturated bridged heterocycle containing 1-2 nitrogen atoms, 7-membered saturated fused heterocycle containing 1-2 nitrogen atoms, 8-membered saturated fused heterocycle containing 1-2 nitrogen atoms,

9-membered saturated fused heterocycle containing 1-2 nitrogen atoms, 10-membered saturated fused heterocycle containing 1-2 nitrogen atoms, 7-membered saturated spiro heterocycle containing 1-2 nitrogen atoms, 8-membered saturated spiro heterocycle containing 1-2 nitrogen atoms, 9-membered saturated spiro heterocycle containing 1-2 nitrogen atoms, 10-membered saturated spiro heterocycle containing 1-2 nitrogen atoms or 11-membered saturated spiro heterocycle containing 1-2 nitrogen atoms, and q is selected from 0, 1, 2 or 3;
other groups are consistent with those mentioned above.

The eleventh technical solution of the present invention relates to the compound represented by formula (I), (1-1), (II), (II-a), (II-b), (III), (III-a), (III-b), (V), (VI) or (VII), or the stereoisomer, solvate, or pharmaceutically acceptable salt thereof according to the present invention, wherein

ring B is piperazinyl, and q is selected from 1 or 2; or
ring B is selected from piperidyl, 5-membered saturated monocyclic heterocycloalkane containing 1-2 nitrogen atoms, 5- to 6-membered partially unsaturated monocyclic heterocycloalkane containing 1-2 nitrogen atoms, 6-membered saturated bridged heterocycle containing 1-2 nitrogen atoms, 7-membered saturated bridged heterocycle containing 1-2 nitrogen atoms, 8-membered saturated bridged heterocycle containing 1-2 nitrogen atoms, 7-membered saturated fused heterocycle containing 1-2 nitrogen atoms, 8-membered saturated fused heterocycle containing 1-2 nitrogen atoms, 9-membered saturated fused heterocycle containing 1-2 nitrogen atoms, 10-membered saturated fused heterocycle containing 1-2 nitrogen atoms, 7-membered saturated spiro heterocycle containing 1-2 nitrogen atoms, 8-membered saturated spiro heterocycle containing 1-2 nitrogen atoms, 9-membered saturated spiro heterocycle containing 1-2 nitrogen atoms, 10-membered saturated spiro heterocycle containing 1-2 nitrogen atoms or 11-membered saturated spiro heterocycle containing 1-2 nitrogen atoms, and q is selected from 0, 1, 2 or 3;
other groups are consistent with those mentioned above.

The twelfth technical solution of the present invention relates to the compound represented by formula (I), (1-1), (1-2), (II), (II-a), (11-b), (III), (III-a), (III-b), (IV), (IV-a), (IV-b), (V), (VI) or (VII), or the stereoisomer, solvate, or pharmaceutically acceptable salt thereof according to the present invention, wherein

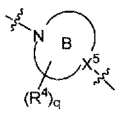

is selected from

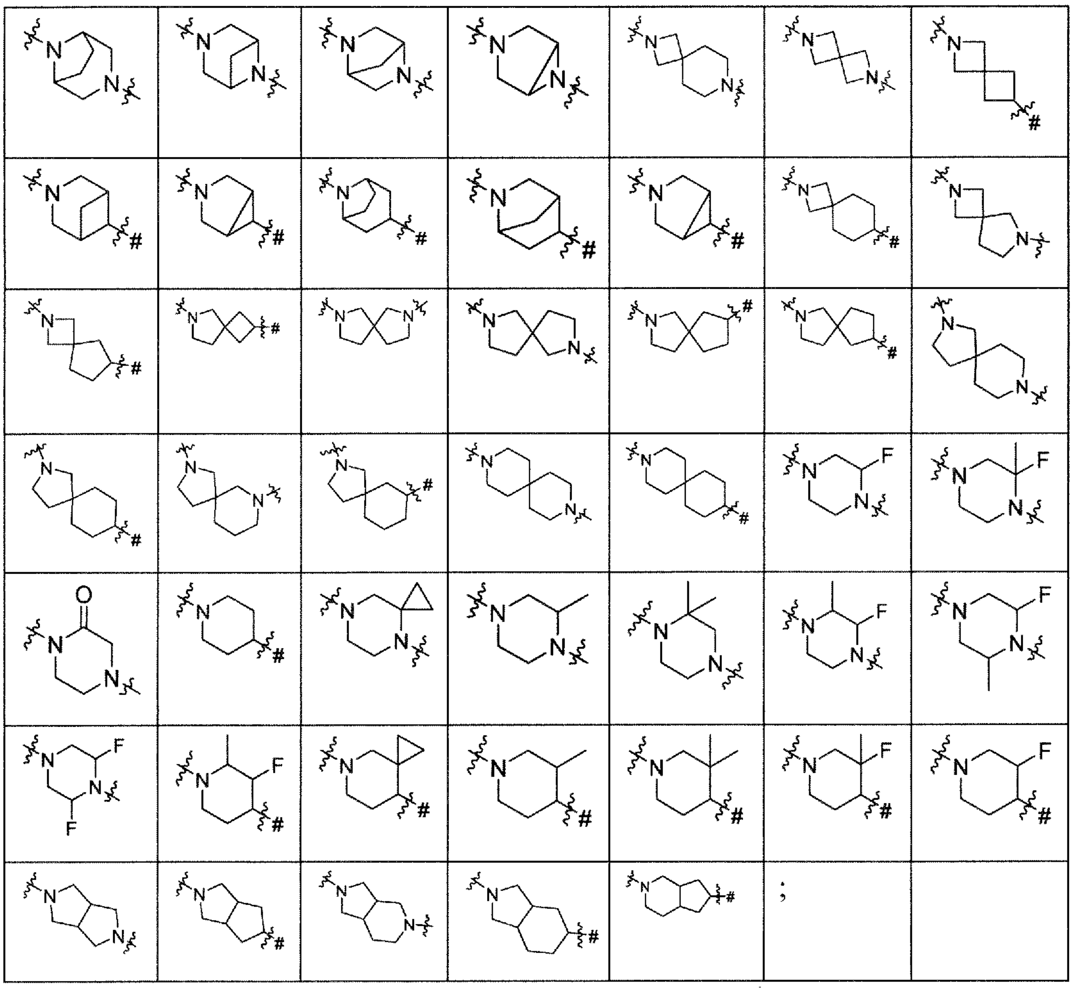

;

or

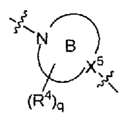

is selected from

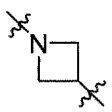, 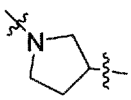 or 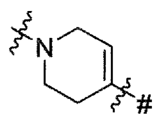;

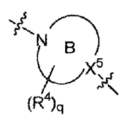

is selected from

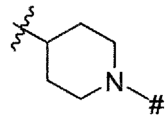 or 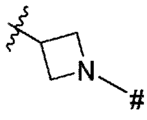;

# represents position $X^5$ in ring B, wherein groups with undefined connection positions can be connected at both ends; other groups are consistent with those mentioned above.

The thirteenth technical solution of the present invention relates to the compound represented by formula (I), (I-1), (I-2), (II), (II-a), (11-b), (III), (III-a), (III-b), (IV), (IV-a), (IV-b), (V), (VI) or (VII), or the stereoisomer, solvate, or pharmaceutically acceptable salt thereof according to the present invention, wherein

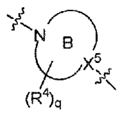

is selected from

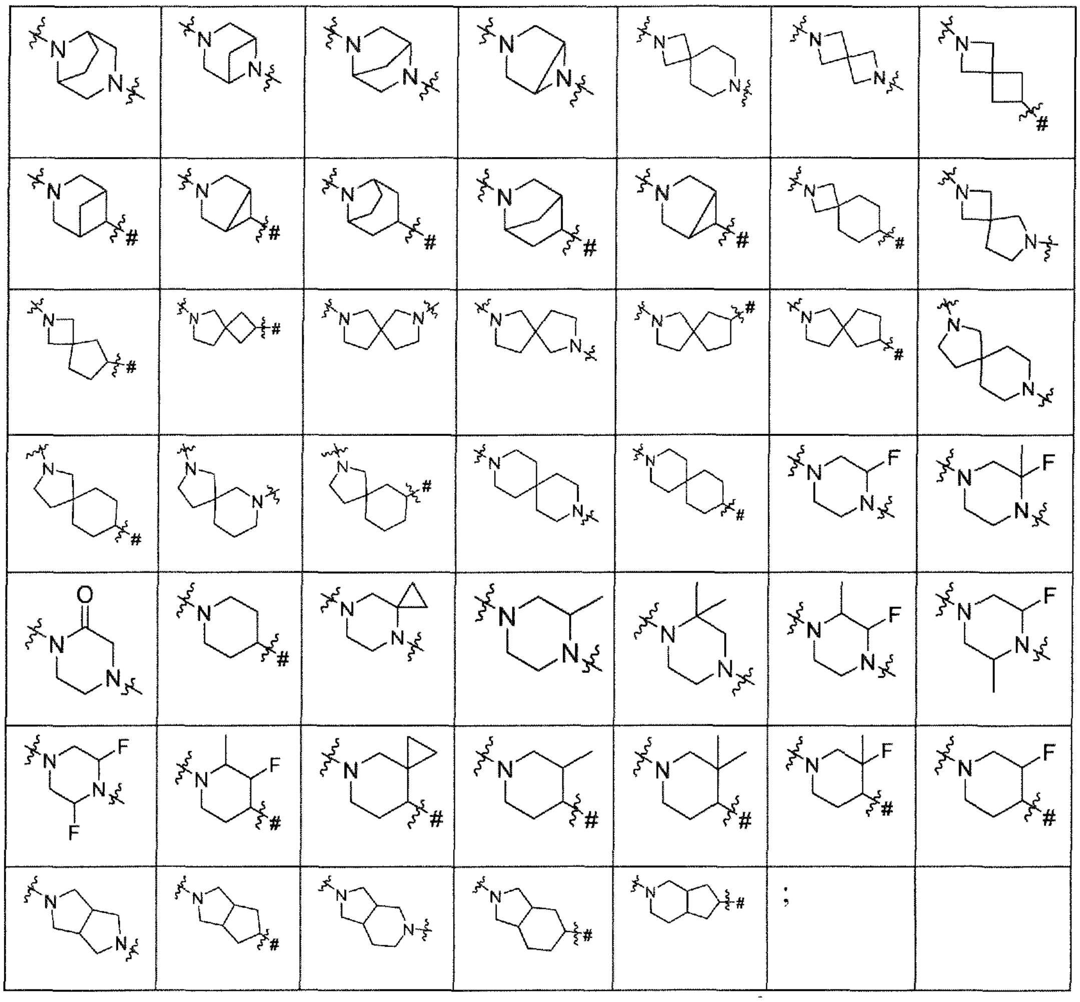

;

or

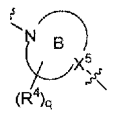

is selected from

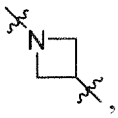, 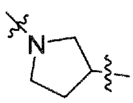 or 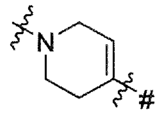;

# represents position $X^5$ in ring B, wherein groups with undefined connection positions can be connected at both ends; other groups are consistent with those mentioned above.

In the compound represented by formula (II-2), (IV), (IV-a) or (IV-b), or the stereoisomer, solvate, or pharmaceutically acceptable salt thereof according to the present invention, in some embodiments,

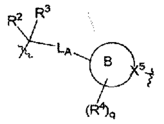

is selected from

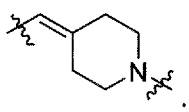.

The fourteenth technical solution of the present invention relates to the compound represented by formula (I), (1-1), (1-2), (II), (II-a), (I1-b), (III), (III-a), (III-b), (IV), (IV-a), (IV-b), (V), (VI) or (VII), or the stereoisomer, solvate, or pharmaceutically acceptable salt thereof according to the present invention, wherein

$R^1$ is selected from halogen, nitro, cyano, amino, hydroxyl, $-SF_5$, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl, $-(CH_2)_r-C_{5-9}$ spiro cycloalkyl, $-(CH_2)_r$-(4- to 6-membered monocyclic heterocycloalkyl) or $-(CH_2)_r$-(5- to 9-membered spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino, hydroxyl, $C_{1-3}$ alkyl or $C_{1-3}$ alkoxy;

each $R_a$ is independently selected from $-C(O)N(R^{a1})_2$, $-NR^{a1}C(O)OR^{a1}$, - $NR^{a1}C(O)R^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-4}$ alkyl, halo $C_{1-4}$ alkyl, $C_{1-4}$ alkoxy, halo $C_{1-4}$ alkoxy, deuterated $C_{1-4}$ alkyl, or deuterated $C_{1-4}$ alkoxy;

each r is independently selected from 0, 1 or 2;

each $R^{a1}$ is independently selected from $C_{1-4}$ alkyl, $C_{3-5}$ cycloalkyl, $C_{5-9}$ spiro cycloalkyl, 4- to 6-membered heterocycloalkyl, 5- to 9-membered spiro heterocycloalkyl, 5- to 6-membered heteroaryl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy, wherein the cycloalkyl, heterocycloalkyl, or heteroaryl is optionally further substituted with 1-3 substituents selected from D, halogen, $C_{1-4}$ alkyl, halo $C_{1-4}$ alkyl, $C_{1-4}$ alkoxy, halo $C_{1-4}$ alkoxy, deuterated $C_{1-4}$ alkyl or deuterated $C_{1-4}$ alkoxy;

$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl;

each $R^4$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; or two $R^4$ on the same carbon atom together with the carbon atom to which they are attached form =O;

each $R^5$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;
p is selected from 0, 1 or 2;
other groups are consistent with those mentioned above.

The fifteenth technical solution of the present invention relates to the compound represented by formula (1), (1-1), (I-2), (II), (II-a), (II-b), (III), (III-a), (III-b), (IV), (IV-a), (IV-b), (V), (VI) or (VII), or the stereoisomer, solvate, or pharmaceutically acceptable salt thereof according to the present invention, wherein

$R^1$ is selected from halogen, nitro, cyano, amino, hydroxyl, $-SF_5$, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r$-$C_{3-6}$ monocyclic cycloalkyl, $-(CH_2)_r$-$C_{5-9}$ spiro cycloalkyl, $-(CH_2)_r$-(4- to 6-membered monocyclic heterocycloalkyl) or $-(CH_2)_r$-(5- to 9-membered spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino, hydroxyl, $C_{1-3}$ alkyl or $C_{1-3}$ alkoxy;
each $R_a$ is independently selected from $-C(O)N(R^{a1})_2$, $-NR^{a1}C(O)OR^{a1}$, - $NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-4}$ alkyl, halo $C_{1-4}$ alkyl, $C_{1-4}$ alkoxy, halo $C_{1-4}$ alkoxy, deuterated $C_{1-4}$ alkyl or deuterated $C_{1-4}$ alkoxy;
each r is independently selected from 0, 1 or 2;
each $R^{a1}$ is independently selected from $C_{1-4}$ alkyl, $C_{3-5}$ cycloalkyl, $C_{5-9}$ spiro cycloalkyl, 4- to 6-membered heterocycloalkyl, 5- to 9-membered spiro heterocycloalkyl, $C_{1-4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy;
$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl;
each $R^4$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; or two $R^4$ on the same carbon atom together with the carbon atom to which they are attached form =O;
each $R^5$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;
p is selected from 0, 1 or 2;
other groups are consistent with those mentioned above.

The sixteenth technical solution of the present invention relates to the compound represented by formula (1), (1-1), (1-2), (II), (II-a), (11-b), (III), (III-a), (III-b), (IV), (IV-a), (IV-b), (V), (VI) or (VII), or the stereoisomer, solvate, or pharmaceutically acceptable salt thereof according to the present invention, wherein

$R^1$ is selected from cyano, $C_{1-2}$ alkyl, $C_{1-2}$ alkoxy, $C_{2-3}$ alkenyl, $C_{2-3}$ alkynyl, $C_{1-2}$ alkyl-O-$C_{1-2}$ alkyl, $-(CH_2)_r$-$C_{3-4}$ monocyclic cycloalkyl, $-(CH_2)_r$-$C_{5-7}$ spiro cycloalkyl, $-(CH_2)_r$-(4-membered monocyclic heterocycloalkyl), or $-(CH_2)_r$-(5- to 7-membered spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino or hydroxyl;
each $R_a$ is independently selected from $-C(O)N(R^{a1})_2$, $-NR^{a1}C(O)OR^{a1}$, - $NR^{a1}C(O)R^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-2}$ alkyl, halo $C_{1-2}$ alkyl, $C_{1-2}$ alkoxy, halo $C_{1-2}$ alkoxy, deuterated $C_{1-2}$ alkyl, or deuterated $C_{1-2}$ alkoxy;
each r is independently selected from 0 or 1;
p is selected from 0 or 1;
other groups are consistent with those mentioned above.

The seventeenth technical solution of the present invention relates to the compound represented by formula (I), (1-1), (1-2), (II), (II-a), (11-b), (III), (III-a), (III-b), (IV), (IV-a), (IV-b), (V), (VI) or (VII), or the stereoisomer, solvate, or pharmaceutically acceptable salt thereof according to the present invention, wherein

$R^1$ is selected from cyano, $C_{1-2}$ alkyl, $C_{1-2}$ alkoxy, $C_{2-3}$ alkenyl, $C_{2-3}$ alkynyl, $C_{1-2}$ alkyl-O-$C_{1-2}$ alkyl, $-(CH_2)_r-C_{3-4}$ monocyclic cycloalkyl, $-(CH_2)_r-C_{5-7}$ spiro cycloalkyl, $-(CH_2)_r$-(4-membered monocyclic heterocycloalkyl), or $-(CH_2)_r$-(5- to 7-membered spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino or hydroxyl;
each $R_a$ is independently selected from $-C(O)N(R^{a1})_2$, $-NR^{a1}C(O)OR^{a1}$, - $NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}\ alkyl)_2$, $C_{1-2}$ alkyl, halo $C_{1-2}$ alkyl, $C_{1-2}$ alkoxy, halo $C_{1-2}$ alkoxy, deuterated $C_{1-2}$ alkyl or deuterated $C_{1-2}$ alkoxy;
each r is independently selected from 0 or 1;
p is selected from 0 or 1;
other groups are consistent with those mentioned above.

The eighteenth technical solution of the present invention relates to the compound, or the stereoisomer, solvate, or pharmaceutically acceptable salt thereof according to the present invention, wherein

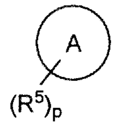

is selected from

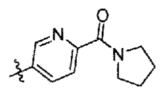 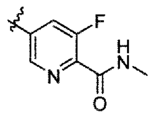 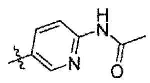 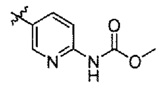 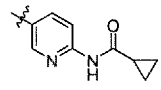 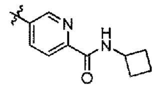

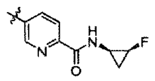 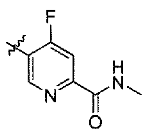 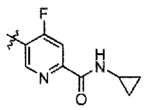 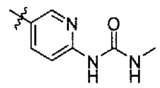 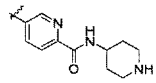 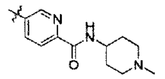

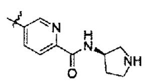 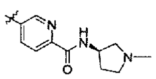 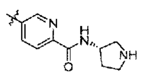 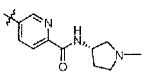 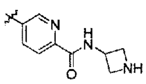 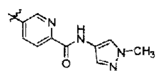

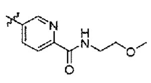 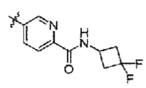 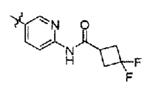 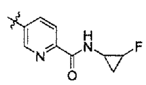

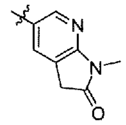 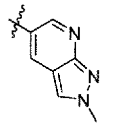 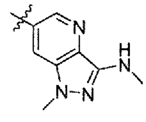 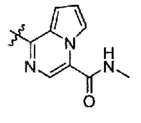 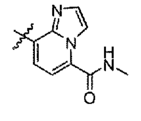 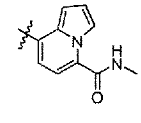

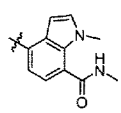 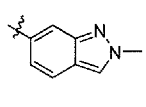 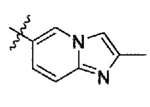 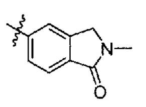 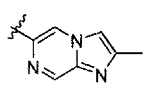 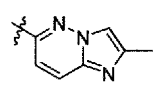

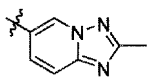 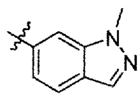 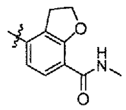 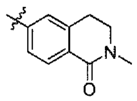 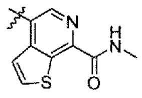 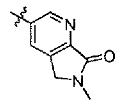

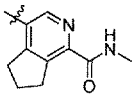 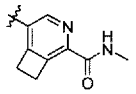 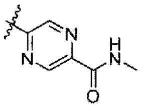 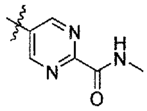 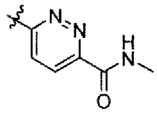 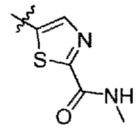

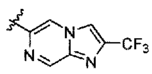 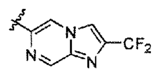 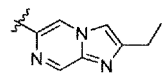

or

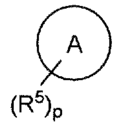

is selected from

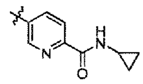;

other groups are consistent with those mentioned above.

The nineteenth technical solution of the present invention relates to the compound, or the stereoisomer, solvate, or pharmaceutically acceptable salt thereof according to the present invention, having a structure of formula (VI) or (VII):

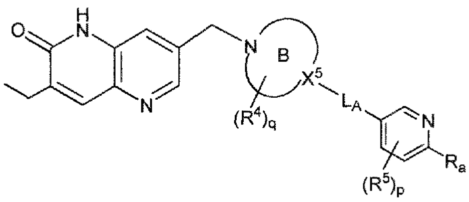 (VI) 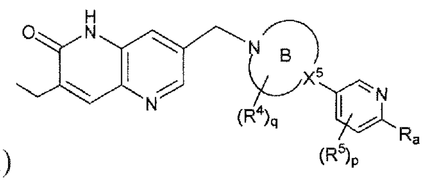 (VII)

$R_a$ is selected from -C(O)NH$R^{a1}$ or -NHC(O)$R^{a1}$;

$L_A$ is selected from a bond, NH or -N($CH_3$)-;

$X^5$ is independently selected from N, C or CH;

each $R^{a1}$ is independently selected from $C_{1-2}$ alkyl, $C_{3-5}$ cycloalkyl, 5- to 6-membered heteroaryl, halo $C_{1-2}$ alkyl or deuterated $C_{1-2}$ alkyl, wherein the cycloalkyl or heteroaryl is optionally further substituted with 1-3 substituents selected from D, F, Cl, cyano, hydroxyl, amino, $C_{1-2}$ alkyl, deuterated $C_{1-2}$ alkyl or deuterated $C_{1-2}$ alkoxy;

each $R^4$ is independently selected from D, F, Cl, cyano, $C_{1-2}$ alkyl, halo $C_{1-2}$ alkyl or deuterated $C_{1-2}$ alkyl; or two $R^4$ on the same carbon atom together with the carbon atom to which they are attached form 3- to 4-membered cycloalkyl;

each $R^5$ is independently selected from D, F, Cl, cyano, $C_{1-2}$ alkyl, halo $C_{1-2}$ alkyl or deuterated $C_{1-2}$ alkyl;

q is selected from 0, 1 or 2; p is selected from 0 or 1;

ring B is selected from piperidyl, 6-membered partially unsaturated monocyclic heterocycloalkane containing 1-2 nitrogen atoms, 6-membered saturated bridged heterocycle containing 1-2 nitrogen atoms, 7-membered saturated bridged heterocycle containing 1-2 nitrogen atoms, 8-membered saturated bridged heterocycle containing 1-2 nitrogen atoms, 8-membered saturated fused heterocycle containing 1-2 nitrogen atoms, 9-membered saturated fused heterocycle containing 1-2 nitrogen atoms, 10-membered saturated fused heterocycle containing 1-2 nitrogen atoms, 7-membered saturated spiro heterocycle containing 1-2 nitrogen atoms, 8-membered saturated spiro heterocycle

containing 1-2 nitrogen atoms, 9-membered saturated spiro heterocycle containing 1-2 nitrogen atoms, 10-membered saturated spiro heterocycle containing 1-2 nitrogen atoms or 11-membered saturated spiro heterocycle containing 1-2 nitrogen atoms;

other groups are consistent with those mentioned above.

The twentieth technical solution of the present invention relates to the compound, or the stereoisomer, solvate, or pharmaceutically acceptable salt thereof according to the present invention, wherein

each $R^{a1}$ is independently selected from methyl, ethyl, cyclopropyl, cyclobutyl, pyrazolyl, imidazolyl, thiazolyl, $CH_2F$, $CHF_2$, $CF_3$, $CH_2D$, $CHD_2$ or $CD_3$, wherein the cyclopropyl, cyclobutyl, pyrazolyl, imidazolyl, or thiazolyl is optionally further substituted with 1-3 substituents selected from D, F, Cl, cyano, hydroxyl, amino, methyl, $CH_2F$, $CHF_2$, $CF_3$, $-OCH_2D$, $-OCHD_2$, or $-OCD_3$;

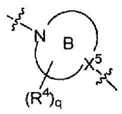

is selected from

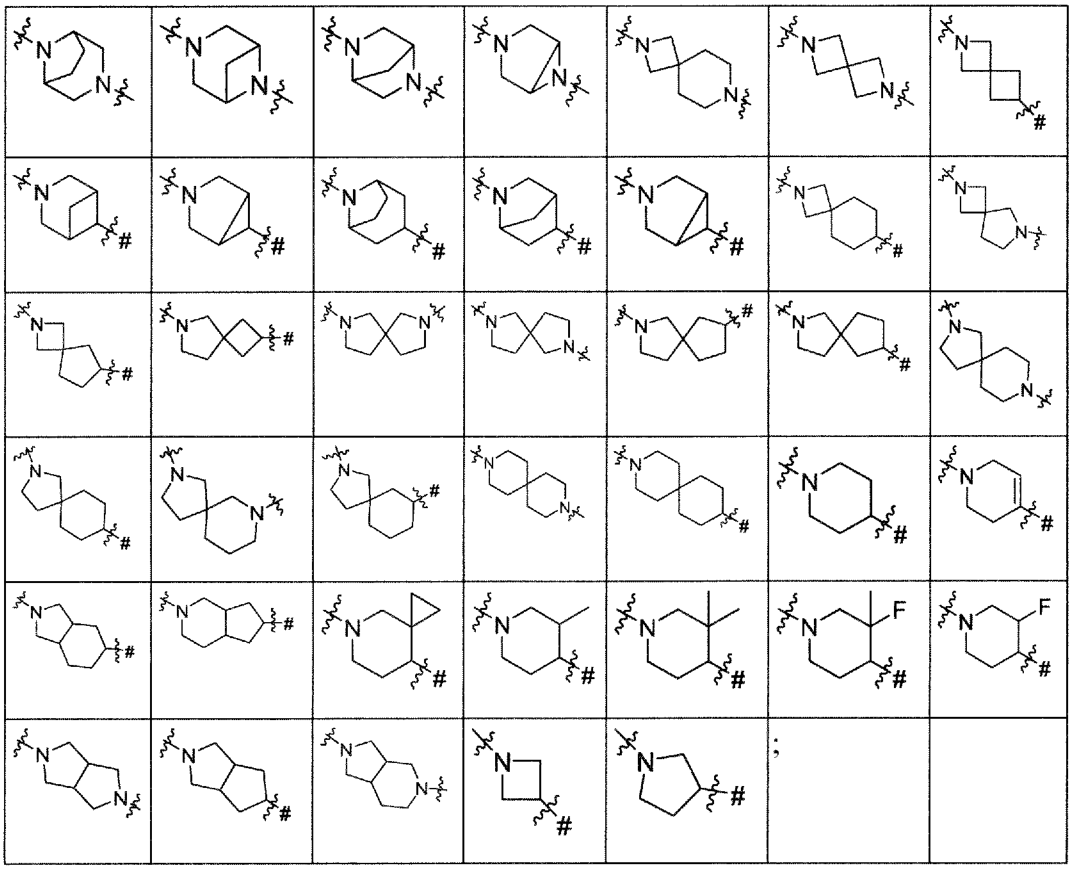

;

p is selected from 0;

other groups are consistent with those mentioned above.

The twenty-first technical solution of the present invention relates to the compound, or the stereoisomer, solvate, or pharmaceutically acceptable salt thereof according to the present invention, wherein the compound is selected from one of the following structures:

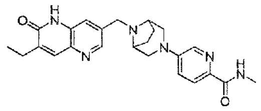 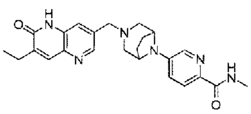 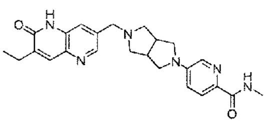 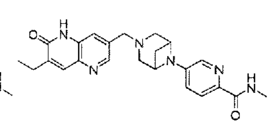

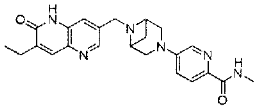 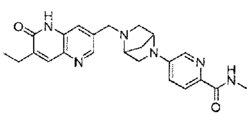 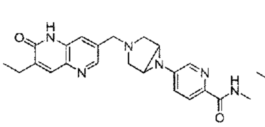 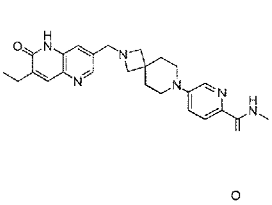

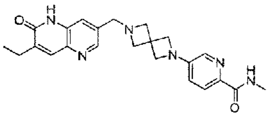 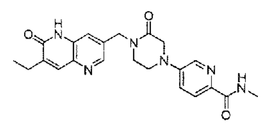 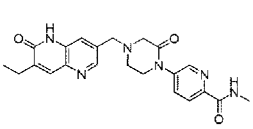 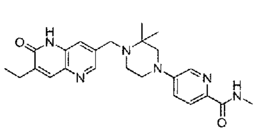

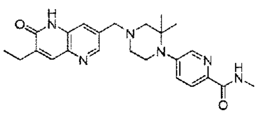 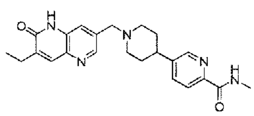 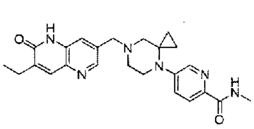 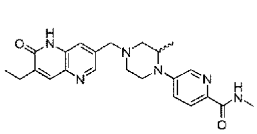

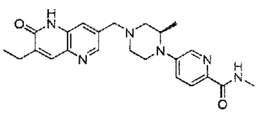 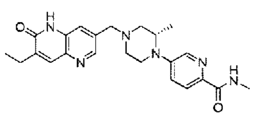 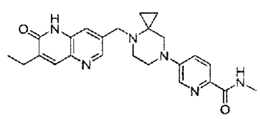 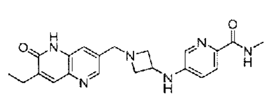

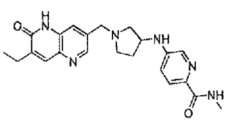 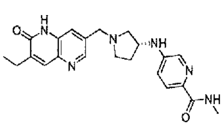 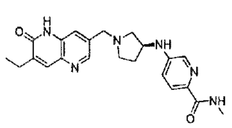 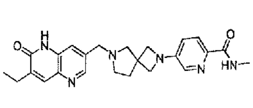

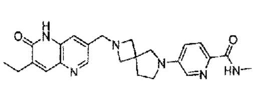 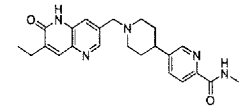 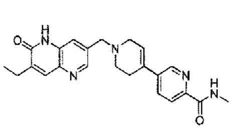 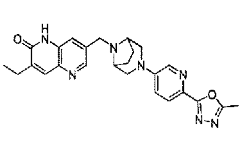

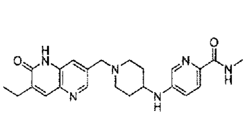 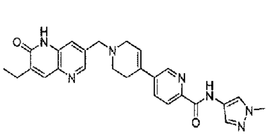 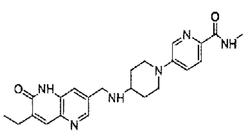 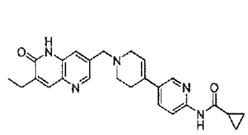

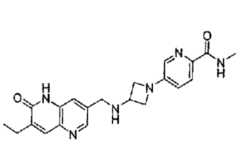 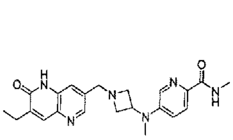 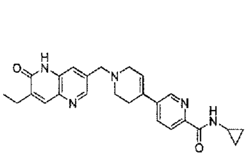 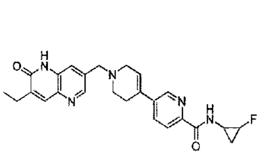

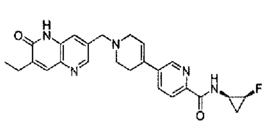 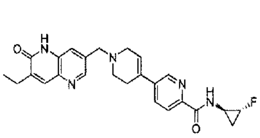 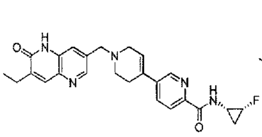 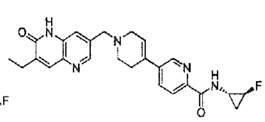

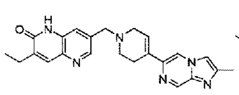
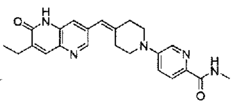
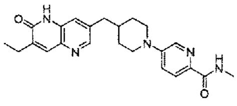
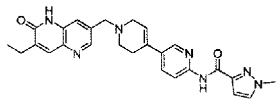

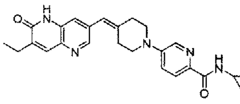
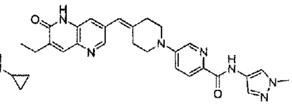
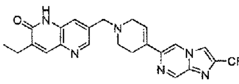
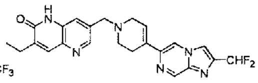

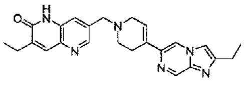
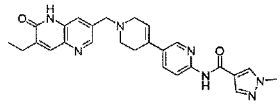

Secondly, the present invention also provides a pharmaceutical composition or pharmaceutical preparation, comprising the compound, or the stereoisomer, solvate, or pharmaceutically acceptable salt thereof according to any one of the preceding technical solutions, and a pharmaceutically acceptable excipient and/or carrier. The pharmaceutical composition can be in a unit preparation form (the unit preparation is also referred to as the "preparation strength").

Further, the composition or pharmaceutical preparation of the present invention comprises 1-1500 mg of the compound, or the stereoisomer, solvate, or pharmaceutically acceptable salt thereof according to any one of the preceding solutions, and a pharmaceutically acceptable carrier and/or excipient.

Further, the present invention also provides the use of the compound, or the stereoisomer, solvate, or pharmaceutically acceptable salt thereof, or the pharmaceutical composition according to any one of the preceding embodiments in the preparation of a drug for treating/preventing a PARP-1-mediated disease. Further, the PARP-1-mediated disease includes, but is not limited to cancer.

The present invention also provides a method for treating a disease in a mammal, comprising administering to a subject a therapeutically effective amount of the compound, or the stereoisomer, solvate, or pharmaceutically acceptable salt thereof according to any one of the preceding solutions, wherein the disease is preferably cancer, and the therapeutically effective amount is preferably 1-1500 mg. In some embodiments, the mammal according to the present invention comprises humans.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

In some embodiments, the pharmaceutical composition or preparation of the present invention contains the above-mentioned therapeutically effective amount of the compound, or the stereoisomer, solvate, or pharmaceutically acceptable salt thereof according to the present invention.

The present invention relates to a pharmaceutical composition or pharmaceutical preparation, comprising a therapeutically effective amount of the compound, or the stereoisomer, solvate, or pharmaceutically acceptable salt thereof according to the present invention, and a carrier and/or excipient. The pharmaceutical composition can be in a unit preparation form (the amount of the drug substance in the unit preparation is also referred to as the "preparation strength"). In some embodiments, the pharmaceutical composition comprises the compound, or the stereoisomer, solvate, or pharmaceutically acceptable salt thereof according to the present invention in an amount including but not limited to 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1500 mg.

The present invention also provides a method for treating a disease in a mammal, comprising administering to a subject a therapeutically effective amount of the compound, or the stereoisomer, solvate, or pharmaceutically acceptable salt thereof according to the present invention, and a pharmaceutically acceptable carrier and/or excipient, wherein the therapeutically effective amount is preferably 1-1500 mg, and the disease is preferably cancer.

The present invention also provides a method for treating a disease in a mammal, comprising administering to a subject a drug, i.e., the compound, or the stereoisomer, solvate, or pharmaceutically acceptable salt thereof according to the present invention, and a pharmaceutically acceptable carrier and/or excipient in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, which may include a composition in single-dose or multiple-dose form, wherein the kit comprises the compound, or the stereoisomer, solvate, or pharmaceutically acceptable salt thereof according to the present invention, and the amount of the compound, or the stereoisomer, solvate, or pharmaceutically acceptable salt thereof according to the present invention is the same as that in the above-mentioned pharmaceutical composition.

In the present invention, the amount of the compound, or the stereoisomer, solvate, or pharmaceutically acceptable salt thereof according to the present invention is calculated in the form of a free base in each case.

The term "preparation strength" refers to the weight of the drug substance contained in each vial, tablet or other unit preparation.

Synthetic route

Patent document WO 2021013735 A1 introduces a method for preparing a PARP-1 inhibitor, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modem Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopaedia" (1999) John Wiley & Sons, ISBN: 3-527-29645-X, in 8 volumes; "Organic Reactions" (1942-2000) John Wiley & Sons, in over 55 volumes; and "Chemistry of Functional Groups", John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalogue are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

Term

Unless otherwise specified, the terms of the present invention have the following meanings.
The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulphur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.
The term "halogen" herein refers to F, Cl, Br, I, or isotopes thereof.
The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.
The term "deuterium" refers to the isotope deuterium of hydrogen (H).
The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.
Group "$C_{x-y}$" refers to a group comprising x to y carbon atoms, for example, "$C_{1-6}$ alkyl" refers to alkyl comprising 1-6 carbon atoms.
The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms, such as "$C_{1-6}$ alkyl", "$C_{1-5}$ alkyl", "$C_{1-4}$ alkyl", "$C_{1-3}$ alkyl", "$C_{1-2}$ alkyl", "$C_{2-6}$ alkyl", "$C_{2-5}$ alkyl", "$C_{2-4}$ alkyl", "$C_{2-3}$ alkyl", "$C_{3-6}$ alkyl", "$C_{3-5}$ alkyl", "$C_{3-4}$ alkyl", "$C_{4-6}$ alkyl", "$C_{4-5}$ alkyl", and "$C_{5-6}$ alkyl". Non-limiting examples of alkyl include methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, etc. The alkyl can be further substituted with any substituent.
The term "alkylene" refers to a bivalent straight or branched saturated alkyl. Examples of alkylene include, but are not limited to, methylene, ethylidene, *etc.* The alkylene can be optionally further substituted with a substituent.
The term "haloalkyl" refers to an alkyl group in which one or more hydrogens are substituted with one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, or 1-3 halogen, or 1-2 halogen, or 1 halogen; and when the number of halogen substituents is greater than 1, the group to be substituted can be substituted with the same or different halogen. Specific examples include, but are not limited to, $-CF_3$, $-CH_2Cl$, $-CH_2CF_3$, $-CCl_2$, $CF_3$, etc.
The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.
The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents

is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and more further such as 2 to 4 carbon atoms. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a straight or branched divalent unsaturated hydrocarbon group containing at least one carbon-carbon double bond (C=C). Unless otherwise specified, the alkynylene contains 2-6 carbon atoms, preferably 2-4 carbon atoms. Non-limiting examples of alkynylene include ethynylene, and the alkenylene may be optionally substituted with a substituent.

The term "alkynyl" refers to a straight or branched hydrocarbon group containing at least one carbon-carbon triple bond (C=C) and generally comprises 2 to 18 carbon atoms; further, alkynyl comprises 2 to 8 carbon atoms; further, alkynyl comprises 2 to 6 carbon atoms, and more further, alkynyl comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a straight or branched, divalent unsaturated hydrocarbon group containing a carbon-carbon triple bond (C=C) and generally comprises 2-6 carbon atoms, and further comprises 2-4 carbon atoms. Non-limiting examples of alkynylene include ethynylene, propynylene, and butynylene, and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic; the bicyclic or polycyclic cycloalkyl may be in the form of a fused ring, a spiro ring, a bridged ring or a combination thereof, and may comprise one or more aromatic rings, but the ring system is non-aromatic as a whole; and the connection site is on a non-aromatic ring. Generally, the cycloalkyl contains 3 to 20 carbon atoms; further, the cycloalkyl contains 3-8 carbon atoms; and still further, the cycloalkyl contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

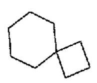, 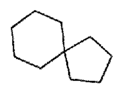, 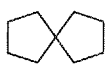, 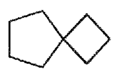,

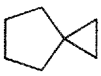, 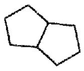, 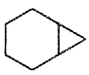, 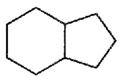, 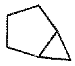, 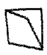, 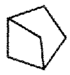, 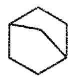, 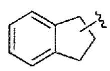,

etc., and the cycloalkyl may be optionally substituted with a substituent.

The term "cycloalkylene" refers to a divalent group of cycloalkyl.

The term "aryl" or "aromatic ring" refers to a substituted or unsubstituted aromatic 5- to 15-membered carbocycle, and includes monocyclic aryl and fused aryl. Aryl is preferably a 5- to 10-membered aromatic ring, further preferably a 5- to 9-membered aromatic ring, and further preferably a 5- to 8-membered aromatic ring. Aryl ring can be fused to an aryl ring and a non-aryl ring (such as a heteroaryl, heterocycloalkyl, or cycloalkyl ring), wherein the aryl ring is the connection site. Non-limiting examples of aryl include phenyl, naphthyl, anthryl, phenanthryl and

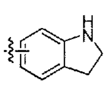, 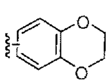, 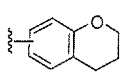, 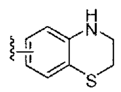, 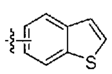, 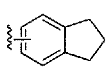, 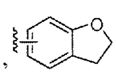, 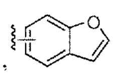,

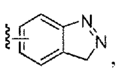, 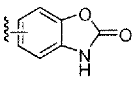, 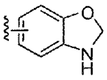, 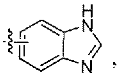, 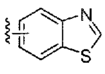, 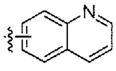 and 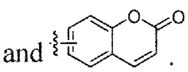.

and the aryl may be optionally further substituted with any substituent.

The term "heterocycloalkyl" or "heterocycloalkane" refers to a saturated or partially unsaturated, non-aromatic carbocycle comprising 1, 2, 3, 4 or 5 heteroatoms selected from N, S, O, P or Si. The heterocycloalkyl may be monocyclic, bicyclic or polycyclic; the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole; and the connecting site is on a non-aromatic ring. Usually, the heterocycloalkyl is a 3-20-membered ring. When the heterocycloalkyl is monocyclic heterocycloalkyl, the heterocycloalkyl is usually a 3- to 15-membered ring, or a 3-to 10-membered ring, or a 3-8-membered ring, or a 3-6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N, S and P include their oxidation states C=O, NO, S=O, $S(=O)_2$, P=O, and $P(=O)_2$. When heterocycloalkyl is a bicyclic or polycyclic ring, at least one ring contains at least one heteroatom, and the heterocycloalkyl can be a bicyclic or polycyclic ring formed by a ring containing the heteroatom(s) and a ring containing no heteroatom, or a bicyclic or polycyclic ring formed by a ring containing the heteroatom(s) and a ring containing the heteroatom(s). When heterocycloalkyl is connected to other groups, the connection site can be at a heteroatom or a carbon atom. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

The term "heteroaromatic ring" or "heteroaryl", unless otherwise specified, refers to an aromatic ring containing 1 to 4 heteroatoms selected from N, O, S, P, Si and their oxidation states, which can be monocyclic, bicyclic, or polycyclic, wherein the bicyclic or polycyclic heteroaromatic ring or heteroaryl can be bridged rings, fused rings, spiro rings and combinations thereof. Bicyclic or polycyclic heteroaromatic ring or heteroaryl can be formed by fusion of heteroaryl to aryl, of heteroaryl to heteroaryl, or of heteroaryl to cycloalkyl or heterocycloalkyl, wherein the heteroaryl is the connection site. Non-limiting examples of heteroaromatic ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl,

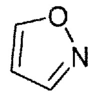,

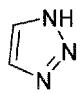, 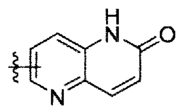, 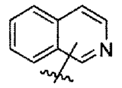, 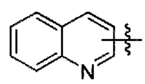,

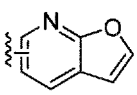, 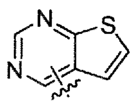, 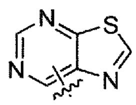, 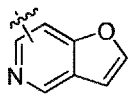,

etc. The heteroaryl may be optionally substituted with a substituent.

The term "aromatic ring" refers to an aromatic ring system containing or containing no N, S, O, P, Si and other heteroatoms, and its definition includes aryl and heteroaryl. The aromatic ring may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. Heterocycles include monocyclic heterocycles, bicyclic bridged heterocycles, bicyclic fused heterocycles and bicyclic spiro heterocycles or combinations thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl,

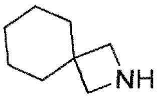, 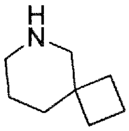, 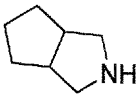, 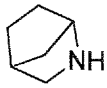, 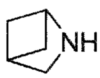,

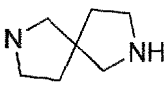, 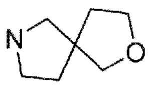, 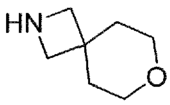, 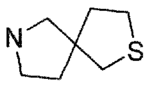,

etc., and the heterocycle may be optionally substituted with a substituent.

The term "spiro ring" refers to a polycyclic group sharing one atom (referred to as a spiro atom) between rings, which may contain 0 or at least 1 double or triple bond, and may contain 0 to 5 heteroatoms selected from N, O, S, P, Si and their oxidation states. Generally, a spiro ring is a 5- to 14-membered ring, or a 5- to 12-membered ring, or a 5- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. The spiro ring may be spirocyclic, non-limiting examples of which include

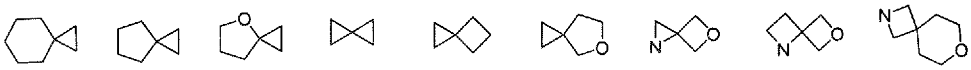

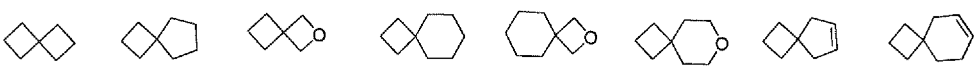

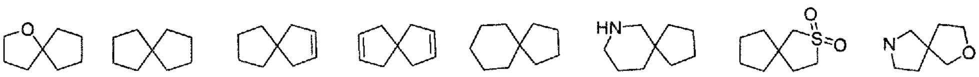,

and the spiro ring may be optionally substituted with a substituent.

The term "bicyclic spiro cycloalkyl" means that both rings forming the spiro ring are cycloalkyl.

The term "bicyclic spiro heterocycloalkyl" means that at least one of the two rings forming the spiro ring is heterocycloalkyl.

The term "fused ring" refers to a polycyclic group in which the rings share two adjacent ring atoms and one chemical bond, which may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of fused ring include purine, quinoline, isoquinoline, benzopyran, benzofuran, benzothiophene, and

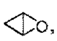, 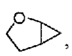, 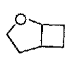, 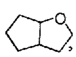, 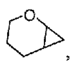, 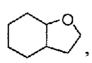, 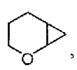, 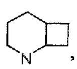, 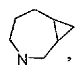, 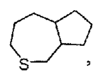,

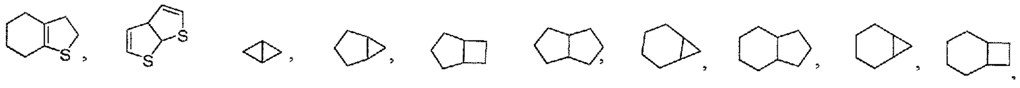

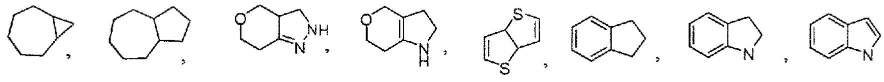
;

and the fused ring may be optionally substituted with a substituent.

The term "bridged ring" refers to a ring system in which two non-adjacent ring atoms are shared between two rings and a bridged ring may contain 1 or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of bridged ring include adamantane,

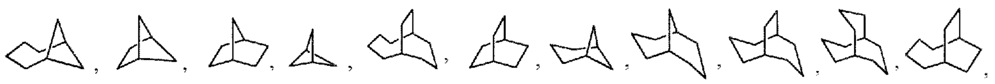

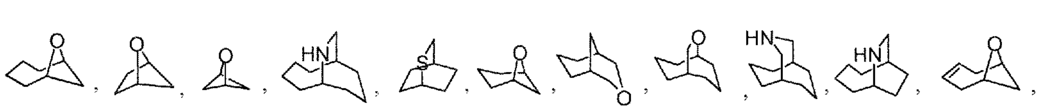

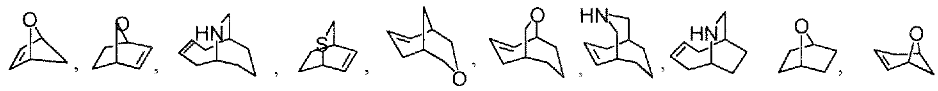

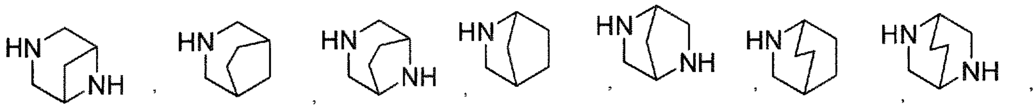

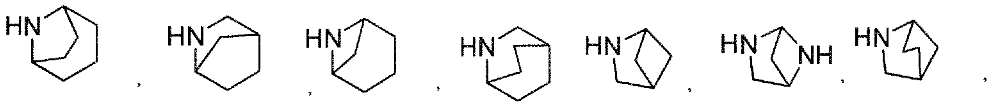

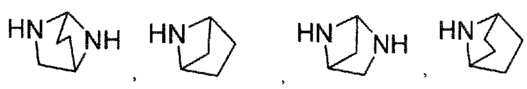

The term "bridged heterocycle" means that at least one ring in the bridged ring is heterocyclic.

The term "fused heterocycle" means that at least one ring in the fused ring is heterocyclic.

The term "spiro heterocycle" means that at least one ring in the spiro ring is heterocyclic.

Unless otherwise specified, the term "substitute" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulfonyl, sulfoxide, halo $C_{1-6}$ alkyl, halo $C_{1-6}$ alkoxy, amino, phosphonic acid, $-CO_2(C_{1-6}$ alkyl), $-OC(=O)(C_{1-6}$ alkyl), $-OCO_2(C_{1-6}$ alkyl), - $C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl)$C(=O)(C_{1-6}$ alkyl), $-NHCO_2(C_{1-6}$ alkyl), $-NHC(=O)N(C_{1-6}$ alkyl$)_2$, - $HC(=O)NH(C_{1-6}$ alkyl), $-NHC(=O)NH_2$, $-NHSO_2(C_{1-6}$ alkyl), $-SO_2N(C_{1-6}$ alkyl$)_2$, - $SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which

salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, pharmaceutically acceptable salts or eutectics thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, conn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or poly anhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

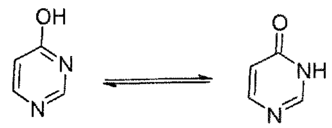

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "eutectic" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio among various components. The eutectic is a multi-component crystal, which includes both a binary eutectic formed between two neutral solids and a multi-element eutectic formed between a neutral solid and a salt or solvate.

Brief Description of the Drawings

Figure 1 shows the tumour growth curve of the mouse MDA-MB-436 subcutaneous in vivo transplanted tumour model.
Figure 2 shows the body weight change curve of the mouse MDA-MB-436 subcutaneous in vivo transplanted tumour model.

Detailed Description of Embodiments

The content of the present invention is described in detail with the following examples. If a specific condition

is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

Detection method

The structure of the compound is determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvents for determination are deuterated dimethyl sulfoxide (DMSO-d6), deuterated chloroform (CDCl3) and deuterated methanol (CD3OD); and the internal standard is tetramethylsilane (TMS);

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

Example 1

5-(5-((7-ethyl-6-oxo-5,6-dihydro-1,5-naphthyridin-3-yl)methyl)hexahydropyrrolo [3,4-c]pyrrol-2(1H)-yl)-N-methylpicolinamide (Compound 1)

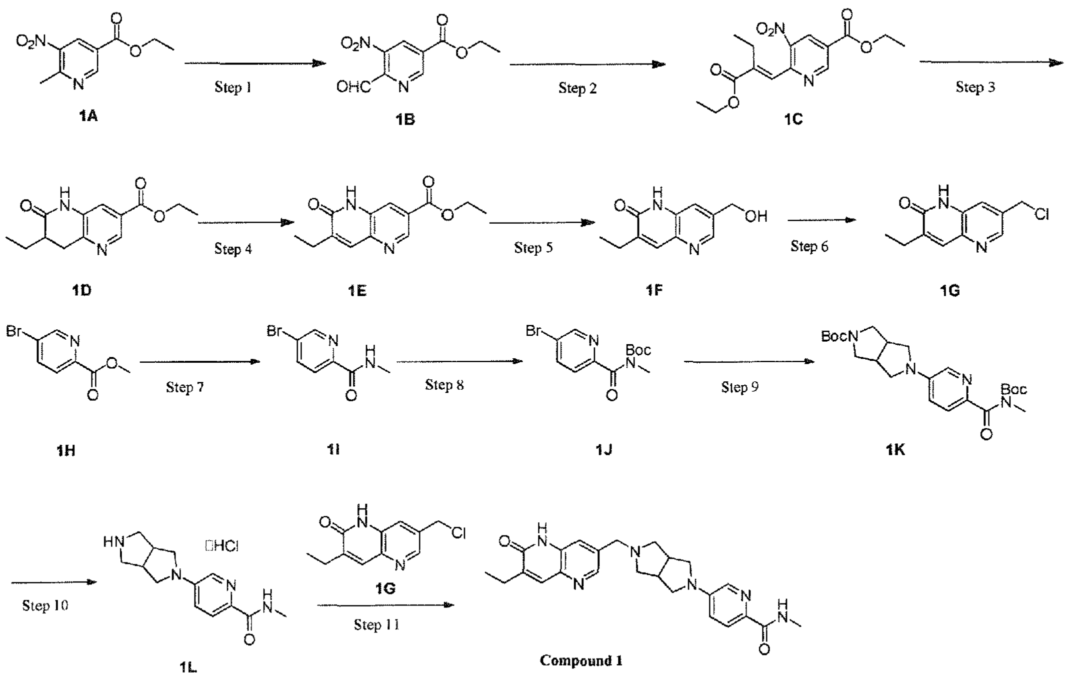


Step 1:

Ethyl 6-methyl-5-nitronicotinate 1A (10 g, 47.6 mmol) and selenium dioxide (21.14 g, 190.5 mmol) were dissolved in 1,4-dioxane (100 mL), and the mixture was refluxed overnight at 100°C. After the reaction was completed, the reaction liquid was filtered through a funnel lined with diatomaceous earth, the diatomaceous earth was washed with ethyl acetate, the filtrate was concentrated, and the resulting residue was separated and purified by silica gel column chromatography

(eluent: ethyl acetate:petroleum ether = 0%-40%), to afford compound 1B (10.104 g, 94.7%) as a yellow oil.
LCMS(ESI) m/z=225.1 $[M+1]^+$

Step 2:

Sodium hydride (2.695 g, 112.3 mmol) was dissolved in anhydrous tetrahydrofuran (100 ml) and stirred at 0°C, and triethyl 2-phosphonobutyrate (28.3 g, 112.3 mmol) was added dropwise. After the dropwise addition was completed, the mixture was stirred at 0°C for 20 min, warmed to 40°C and stirred for 10 min, and then transferred to a dry ice ethanol bath. Compound 1B (10.48 g, 46.8 mmol) was dissolved in anhydrous tetrahydrofuran (100 ml) and added dropwise to a reaction flask. The mixture was kept in the dry ice ethanol bath and stirred for 1 h. After the reaction was completed, the reaction liquid was quenched with saturated ammonium chloride solution (100 ml) and extracted with ethyl acetate (200 ml). The organic phase was separated, and the aqueous phase was extracted with ethyl acetate (200 ml×2). The organic phases were combined, dried over anhydrous sodium sulphate and concentrated, and the resulting residue was purified by silica gel column chromatography (eluent: ethyl acetate:petroleum ether = 0-10%), to afford compound 1C (11.57 g, 76.8%) as a yellow oil, a mixture of two isomers.
LC-MS(ESI) m/z =323.1 $[M+1]^+$

Step 3:

Compound 1C (11.57 g, 35.9 mmol) was dissolved in ethanol (50 ml), and 10% palladium on carbon catalyst (1 g) was added. The mixture was subjected to hydrogen replacement three times, stirred overnight at room temperature, and filtered through a funnel lined with diatomaceous earth, and the diatomaceous earth was washed with anhydrous ethanol. The filtrate was concentrated, to the resulting residue was added a solution of hydrogen chloride in dioxane (60 ml, 4M), and the mixture was stirred at room temperature for 1 h and concentrated. To the resulting residue was added ethyl acetate (50 ml), the mixture was stirred and filtered, and the filter cake was washed with ethyl acetate and dried, to afford compound 1D (4.28 g, 42.0%).
$^1$H NMR (400 MHz, DMSO) δ 10.39 (s, 1H), 8.62 (d, 1H), 7.75 (s, 1H), 4.38 - 4.29 (m, 2H), 3.24 (dd, 1H), 2.97 (dd, 1H), 2.62 - 2.53 (m, 1H), 1.83 - 1.64 (m, 1H), 1.55 - 1.35 (m, 1H), 1.33 (dd, 3H), 0.94 (t, 3H).
Step 4:
Compound 1D (4.28 g, 17.3 mmol) and 2,3-dichloro-5,6-dicyanobenzoquinone (4.309 g, 19.0 mmol) were dissolved in dioxane (86 ml), and the mixture was reacted at 100°C for 3.5 h at reflux. After the reaction was completed, saturated sodium bicarbonate solution (40 ml) and ethyl acetate (120 ml) were added. The organic phase was separated, and the aqueous phase was extracted with ethyl acetate (120 ml×2). The organic phases were combined, dried over anhydrous sodium sulphate and concentrated, and the resulting residue was purified by silica gel column chromatography (eluent: ethyl acetate : petroleum ether = 0-50%), to afford compound 1E (3.375 g, 79.5%).
LC-MS(ESI) m/z =247.1 $[M+1]^+$
Step 5:
Compound 1E (3.375 g, 13.72 mmol) was dissolved in anhydrous tetrahydrofuran (150 ml), and the mixture was stirred at -78°C. Lithium aluminium hydride (1.564 g, 41.16 mmol) was added portionwise, and the mixture was stirred at -78°C for 20 min and then warmed to-40°C and stirred for 20 min. After the reaction was completed, 1M hydrochloric acid was added to adjust the system to a neutral pH, the solvent was removed by distillation under reduced pressure, to the resulting residue was added methanol/dichloromethane (1 : 10, 100 ml) for dissolution, and the mixture was subjected to ultrasonic vibration for 10 min and filtered. The filtrate was collected, the filter cake was redissolved in methanol/dichloromethane (1 : 10, 100 ml), and this process was repeated 8 times. The filtrate was combined and concentrated, to afford compound 1F (2.8 g, 100%).
$^1$H NMR (400 MHz, DMSO-d6) δ 11.86 (s, 1H), 8.37 (d, 1H), 7.72 (d, 1H), 7.62 (d, 1H), 5.44 (t, 1H), 4.61 (d, 2H), 2.57 - 2.51 (m, 2H), 1.18 (t, 3H).

Step 6:

1F (100 mg, 0.49 mmol) was added to dichloromethane (2.5 mL), DMF (1 mL) was added to assist dissolution, thionyl chloride (350 mg, 2.94 mmol) was added dropwise at 0°C, and the mixture was reacted at room temperature for 1 hour. Upon complete depletion of raw materials monitored by LCMS, the reaction liquid was directly spun to dryness, to afford title compound 1G (109 mg, crude) which was used in the next reaction.
LC-MS (ESI): m/z= 223.1, 225.1 $[M+H]^+$.

Step 7:

Methyl 5-bromopicolinate 1H (9 g, 41.66 mmol) was dissolved in 40% methylamine aqueous solution (20 mL), acetonitrile (5 mL) was added, and the mixture was stirred and reacted at 40°C for 2 hours. After the reaction was completed as monitored by TLC, the reaction liquid was spun to dryness to remove the solvent, and the residue was separated by silica gel chromatographic column (100% DCM), to afford compound 1I (8.9 g, 99%).
LC-MS (ESI): m/z= 215.1, 217.0 $[M+H]^+$.

Step 8:

5-Bromo-N-methylpicolinamide 1I (8.9 g, 41.6 mmol) was dissolved in dioxane (100 mL), di-tert-butyl dicarbonate (27.4 g, 125.6 mmol) and 4-dimethylaminopyridine (7.7 g, 62.8 mmol) were added, and the mixture was stirred and reacted at 80°C for 2 hours. After the reaction was completed, the reaction liquid was spun to dryness to remove the solvent, and the residue was separated by silica gel chromatographic column (PE : EA = 1 : 1), to afford product 1J (12 g, 91%).
LC-MS (ESI): m/z= 315.1, 317.0 $[M+H]^+$.

Step 9:

1J (1.7 g, 5.39 mmol) and tert-butyl hexahydropyrrolo[3, 4-C]pyrrole-2(1H)-carboxylate (1.14 g, 5.39 mmol) were dissolved in anhydrous 1,4-dioxane (25 mL), caesium carbonate (3.51 g, 10.78 mmol) and RuPhos-Pd-G2 (0.42 g, 0.54 mmol) were added, and the mixture was stirred and reacted at 100°C under nitrogen protection for 4 hours. The reaction liquid was filtered, and the solid was washed with ethyl acetate. The filtrate was spun to dryness to remove the solvent, and the residue was separated by silica gel chromatographic column (PE : EA = 1 : 2), to afford compound 1K (900 mg, 37.4%).
LC-MS(ESI) m/z=447.3 $[M+1]^+$

Step 10:

1K (900 mg, 2.0 mmol) was dissolved in 5 mL of methanol, a solution of hydrogen chloride in dioxane (10 mL, 4 mol/L) was added, and the mixture was stirred and reacted at room temperature for 2 hours. The reaction liquid was spun to dryness to remove the solvent to afford a crude, and the crude was slurried and purified with ethyl acetate to afford compound 1L (800 mg), which was directly used in the next reaction.
LC-MS(ESI) m/z =247.3 $[M+1]^+$

Step 11:

1G (100 mg, 0.45 mmol) and compound 1L (160 mg, 0.57 mmol) were dissolved in 2 mL of anhydrous DMF, DIEA (1 mL) and potassium iodide (100 mg, 0.60 mmol) were added, and the mixture was stirred and reacted at 80°C for 3 hours. After the reaction was completed, the reaction liquid was spun to dryness to remove the solvent, and the residue was purified by preparative HPLC with preparative HPLC separation methods: 1. Instruments: waters 2767 (preparative liquid phase chromatographic instrument); chromatographic column: SunFire@ PrepC18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 15 mL/min; d. elution time: 20 min, retention time: 8.0 min, to afford compound 1 (20 mg, 10.2%).

$^1$H NMR (400 MHz, DMSO-$d_6$) δ 11.81 (s, 1H), 8.36 (s, 1H), 8.31 (q, 1H), 7.96 (d, 1H),7.82 (d, 1H), 7.72 (s, 1H), 7.59 - 7.54 (m, 1H), 7.06 (dd, 1H), 3.69 (s, 2H), 3.63 - 3.50 (m, 2H), 3.21 (dd, 2H), 2.96 (s, 2H), 2.78 (d, 3H), 2.62 (s, 2H), 2.58 - 2.52 (m, 4H), 1.17 (t, 3H).
LC-MS (ESI): m/z= 433.2 $[M+H]^+$

Example 2

5-(6-((7-ethyl-6-oxo-5,6-dihydro-1,5-naphthyridin-3-yl)methyl)-3,6-diazabicyclo[3.1.1]heptan-3-yl)-N-methylpicolinamide (Compound 2)

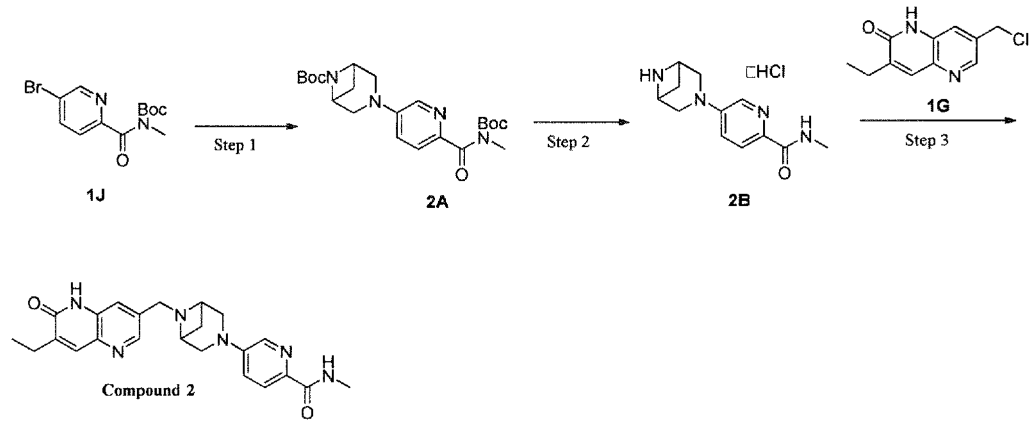


1J 2A 2B 1G

Compound 2

Step 1:

1J (800 mg, 2.54 mmol) and tert-butyl 3,6-diazabicyclo[3.1.1]heptane-6-carboxylate (580 mg, 2.54 mmol) were dissolved in anhydrous 1,4-dioxane (13 mL), caesium carbonate (1.66 g, 5.08 mmol) and RuPhos-Pd-G2 (200 mg, 0.25 mmol) were added, and the mixture was stirred and reacted at 100°C under nitrogen protection for 4 hours. The reaction liquid was filtered, and the solid was washed with ethyl acetate. The filtrate was spun to dryness to remove the solvent, and the residue was separated by silica gel chromatographic column (PE : EA = 1 : 2), to afford compound 2A (530 mg, 48.2%).
LC-MS(ESI) m/z = 433.2 $[M+1]^+$

Step 2:

Compound 2A (530 mg) was dissolved in 5 mL of methanol, a solution of hydrogen chloride in dioxane (10 mL, 4 mol/L) was added, and the mixture was stirred and reacted at room temperature for 2 hours. The reaction liquid was spun to dryness to remove the solvent to afford a crude, and the crude was slurried and purified with ethyl acetate to afford compound 2B (500 mg), which was directly used in the next reaction.
LC-MS(ESI) m/z = 233.3 $[M+1]^+$

Step 3:

1G (100 mg, 0.45 mmol) and compound 2B (160 mg, 0.57 mmol) were dissolved in anhydrous DMF (2 mL), DIEA (1 mL) and potassium iodide (100 mg, 0.60 mmol) were added, and the mixture was stirred and reacted at 80°C for 3 hours. After the reaction was completed, the reaction liquid was spun to dryness to remove the solvent, and the residue was purified by preparative HPLC with preparative HPLC separation methods: 1. Instruments: waters 2767 (preparative liquid phase chromatographic instrument); chromatographic column: SunFire@ PrepC18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 15 mL/min; d. elution time: 20 min, retention time: 7.4 min, to afford compound 2 (50 mg, 26.6%).

$^1H$ NMR (400 MHz, DMSO-$d_6$) δ 8.38 (d, 1H), 8.35 (q, 1H), 8.12 (d, 1H), 7.89 (d, 1H), 7.72 (s, 1H), 7.67 - 7.63 (m, 1H), 7.21 (dd, 1H), 3.73 (d, 2H), 3.63 (d, 4H), 3.44 (d, 2H), 2.80 (d, 3H), 2.57 (d, 1H), 2.53 (d, 2H), 1.84 (s, 7H), 1.63 (d, 1H), 1.17 (t, 3H).
LC-MS (ESI): m/z= 419.2 $[M+H]^+$

Example 3

5-(8-((7-ethyl-6-oxo-5,6-dihydro-1,5-naphthyridin-3-yl)methyl)-3,8-diazabicyclo[3.2.1]octan-3-yl)-N-methylpicolinamide (Compound 3)

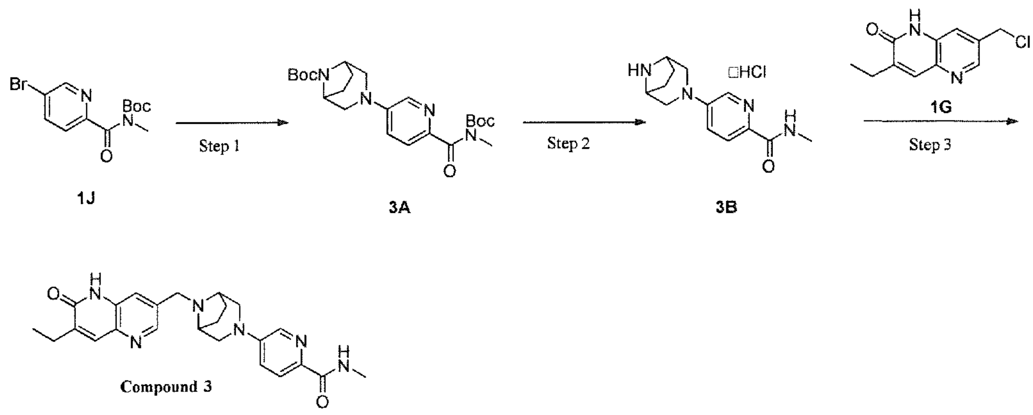


1J 3A 3B

Compound 3

Step 1:

1J (1.04 g, 3.3 mmol) and tert-butyl 3,8-diazabicyclo[3.2.1]octane-8-carboxylate (700 mg, 3.3 mmol) were dissolved in anhydrous 1,4-dioxane (13 mL), caesium carbonate (2.15 g, 6.6 mmol) and RuPhos-Pd-G2 (260 mg, 0.33 mmol) were added, and the mixture was stirred and reacted at 100°C under nitrogen protection for 4 h. The reaction liquid was filtered, and the solid was washed with ethyl acetate. The filtrate was spun to dryness to remove the solvent, and the residue was separated by silica gel chromatographic column (PE : EA = 1 : 2), to afford compound 3A (550 mg, 37.3%).

LC-MS(ESI) m/z = 447.3 $[M+1]^+$

Step 2:

3A (550 mg, 1.23 mmol) was dissolved in methanol (5 mL), a solution of hydrogen chloride in dioxane (10 ml, 4 mol/L) was added, and the mixture was stirred and reacted at room temperature for 2 hours. The reaction liquid was spun to dryness to remove the solvent to afford a crude, and the crude was slurried and purified with ethyl acetate to afford compound 3B (500 mg), which was directly used in the next reaction.

LC-MS(ESI) m/z = 247.2 $[M+1]^+$

Step 3:

1G (100 mg, 0.45 mmol) and 5-(3,8-diazabicyclo[3.2.1]oct-3-yl)-N-methylpicolinamide hydrochloride (140 mg, 0.49 mmol) were dissolved in anhydrous DMF (2 mL), DIEA (1 mL) and potassium iodide (100 mg, 0.60 mmol) were added, and the mixture was stirred and reacted at 80°C for 3 hours. After the reaction was completed, the reaction liquid was spun to dryness to remove the solvent, and the residue was purified by preparative HPLC with preparative HPLC separation methods: 1. Instruments: waters 2767 (preparative liquid phase chromatographic instrument); chromatographic column: SunFire@ PrepC18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 15 mL/min; d. elution time: 20 min, retention time: 7.2 min, to afford compound 3 (50 mg, 25.7%).

$^1$H NMR (400 MHz, DMSO-$d_6$) $\delta$ 12.15 (s, 1H), 10.13 (s, 1H), 8.61 (s, 1H), 8.40 (d, 1H), 8.24 (s, 1H), 7.87 (d, 2H), 7.81 (s, 1H), 7.38 (d, 1H), 4.43 (s, 2H), 4.12 (s, 2H), 3.89 (d, 3H), 3.23 (d, 3H), 2.79 (d, 3H), 2.58 (q, 2H), 2.39 (s, 2H), 2.07 (s, 2H), 1.20 (t, 3H).

LC-MS (ESI): m/z= 433.1 $[M+H]^+$

Example 4

5-(3-((7-ethyl-6-oxo-5,6-dihydro-1,5-naphthyridin-3-yl)methyl)-3,8-diazabicyclo[3.2.1]octan-8-yl)-N-methylpicolinamide (Compound 4)

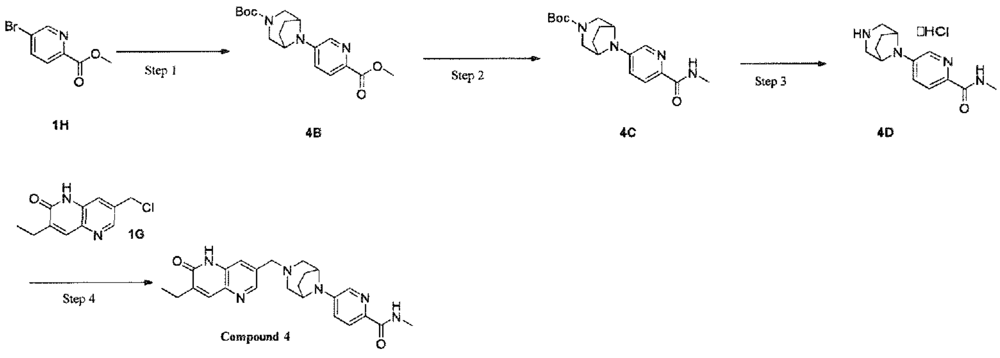

1H 4B 4C 4D 1G Compound 4

Step 1:

Methyl 5-bromopicolinate 1H (500 mg, 2.31 mmol) and tert-butyl 3,8-diazabicyclo[3.2.1]octane-3-carboxylate (590 mg, 2.78 mmol) were dissolved in 1,4-dioxane (10 mL), caesium carbonate (2.26 g, 6.94 mmol) and RuPhos-Pd-G3 (78 mg, 0.09 mmol) were added, and the mixture was reacted overnight at 100°C under nitrogen protection. Subsequently, the reaction liquid was quenched with water (15 mL) and extracted with ethyl acetate (20 mL×3). The organic phases were combined, dried over anhydrous sodium sulphate, filtered, spun to dryness, and separated by silica gel chromatographic column (PE : EA = 1 : 0-1 : 1), to afford title compound 4B (560 mg, 69.7%) as a yellow solid.
LC-MS (ESI): m/z= 348.1 $[M+H]^+$.

Step 2:

4B (560 mg, 1.61 mmol) was dissolved in methanol (5 mL), methylamine aqueous solution (5 mL, 40%) was added, and the mixture was reacted at room temperature for 4 hours. The suspension was concentrated, saturated ammonium chloride solution was added, and the mixture was extracted with dichloromethane. The organic phases were combined, dried over anhydrous sodium sulphate, filtered and spun to dryness, to afford title compound 4C (550 mg, 98.6%).
LC-MS (ESI): m/z= 347.2 $[M+H]^+$.

Step 3:

4C (300 mg, 0.87 mmol) was dissolved in methanol (5 mL), and a solution of hydrogen chloride in dioxane (5 mL, 4M) was added. The mixture was reacted at room temperature for 2 hours and spun to dryness, to afford title compound 4D (240 mg, crude).
LC-MS (ESI): m/z= 247.1 $[M+H]^+$.

Step 4:

Compound 1G (50 mg, 0.22 mmol) and 4D (66 mg, 0.27 mmol) were dissolved in anhydrous acetonitrile (5 mL), potassium iodide (4 mg, 0.02 mmol) and DIPEA (144 mg, 1.12 mmol) were added, and the mixture was subjected to nitrogen replacement and reacted at 80°C for 2 hours. Upon complete depletion of raw materials monitored by LCMS, the system was concentrated, saturated sodium bicarbonate solution (20 mL) was added, and the mixture was extracted with DCM (10 mL × 3). The organic phases were combined, dried over anhydrous sodium sulphate, concentrated, and passed through column (DCM : MeOH = 1 : 0-10 : 1), to afford compound 4 (67 mg, 69.3%).
$^1$H NMR (400 MHz, DMSO-$d_6$) δ 11.84 (s, 1H), 8.33 (d, 1H), 8.32 - 8.27 (m, 1H), 8.15 (d, 1H), 7.79 (d, 1H), 7.73 (s, 1H), 7.65 - 7.60 (m, 1H), 7.28 (dd, 1H), 4.45 (s, 2H), 3.51 (s, 2H), 2.78 (d, 3H), 2.57 - 2.51 (m, 4H overlapped with solvent DMSO peak), 2.42 - 2.35 (m, 2H), 2.09 - 2.01 (m, 2H), 1.96 - 1.86 (m, 2H), 1.18 (t, 3H).
LC-MS (ESI): m/z= 433.2 $[M+H]^+$.

Example 5

5-(5-((7-ethyl-6-oxo-5,6-dihydro-1,5-naphthyridin-3-yl)methyl)-2,5-diazabicyclo[2.2.1]heptan-2-yl)-N-methylpicolinamide (Compound 5)

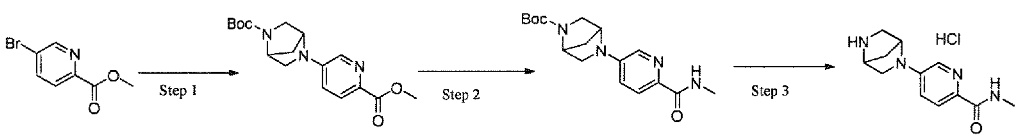


1H 5A 5B 5C

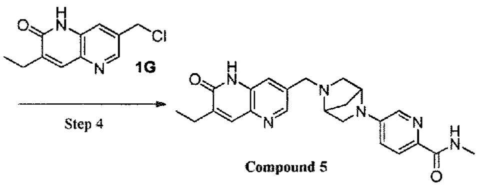


1G

Compound 5

Step 1:

Methyl 5-bromopicolinate 1H (500 mg, 2.31 mmol) and tert-butyl 2,5-diazabicyclo[2.2.1]heptane-2-carboxylate (551 mg, 2.78 mmol) were dissolved in 1,4-dioxane (10 mL), caesium carbonate (2.26 g, 6.94 mmol) and RuPhos-Pd-G3 (78 mg, 0.09 mmol) were added, and the mixture was reacted overnight at 100°C under nitrogen protection. Subsequently, the reaction liquid was quenched with water (15 mL) and extracted with ethyl acetate (20 mL×3). The organic phases were combined, dried over anhydrous sodium sulphate, filtered, spun to dryness, and separated by silica gel chromatographic column (PE : EA = 1 : 0-1 : 1), to afford title compound 5A (710 mg, 92.1%).
LC-MS (ESI): m/z= 334.1 $[M+H]^+$.

Step 2:

5A (710 mg, 2.13 mmol) was dissolved in methanol (5 mL), methylamine aqueous solution (5 mL, 40%) was added, and the mixture was reacted at room temperature for 4 hours. The suspension was concentrated, saturated ammonium chloride solution was added, and the mixture was extracted with dichloromethane. The organic phases were combined, dried over anhydrous sodium sulphate, filtered and spun to dryness, to afford title compound 5B (700 mg, 98.9%).
LC-MS (ESI): m/z= 333.2 $[M+H]^+$.

Step 3:

5B (450 mg, 1.36 mmol) was dissolved in methanol (5 mL), and a solution of hydrogen chloride in dioxane (5 mL, 4M) was added. The mixture was reacted at room temperature for two hours and spun to dryness, to afford title compound 5C (360 mg, crude).
LC-MS (ESI): m/z= 223.1 $[M+H]^+$.

Step 4:

1G (50 mg, 0.22 mmol) and 5C (60 mg, 0.27 mmol) were dissolved in anhydrous acetonitrile (5 mL), potassium iodide (4 mg, 0.02 mmol) and DIPEA (144 mg, 1.12 mmol) were added, and the mixture was subjected to nitrogen replacement and reacted at 80°C for 2 hours. Upon complete depletion of raw materials monitored by LCMS, the system was concentrated, saturated sodium bicarbonate solution (20 mL) was added, and the mixture was extracted with dichloromethane (10 mL × 3). The organic phases were combined, dried over anhydrous sodium sulphate, concentrated, and passed through column (DCM : MeOH = 1 : 0-10 : 1), to afford compound 5 (60 mg, 53.5%).
$^1H$ NMR (400 MHz, DMSO-$d_6$) δ 11.74 (s, 1H), 8.36 (d, 1H), 8.27 (q, 1H), 7.95 (d, 1H), 7.80 (d, 1H), 7.72 (s, 1H), 7.60 (s, 1H), 7.06 (dd, 1H), 4.56 (s, 1H), 3.80 (s, 2H), 3.63 (s, 1H), 3.49 - 3.41 (m, 1H), 3.37 - 3.31 (m, 1H), 2.88 - 2.81 (m, 1H), 2.78 (d, 3H), 2.58 - 2.51 (m, 3H), 2.02 - 1.95 (m, 1H), 1.86 - 1.80 (m, 1H), 1.17 (t, 3H).
LC-MS (ESI): m/z= 419.2 $[M+H]^+$.

Example 6

5-((1-((7-ethyl-6-oxo-5,6-dihydro-1,5-naphthyridin-3-yl)methyl)azetidin-3-yl)amino)-N-methylpicolinamide (Compound 6)

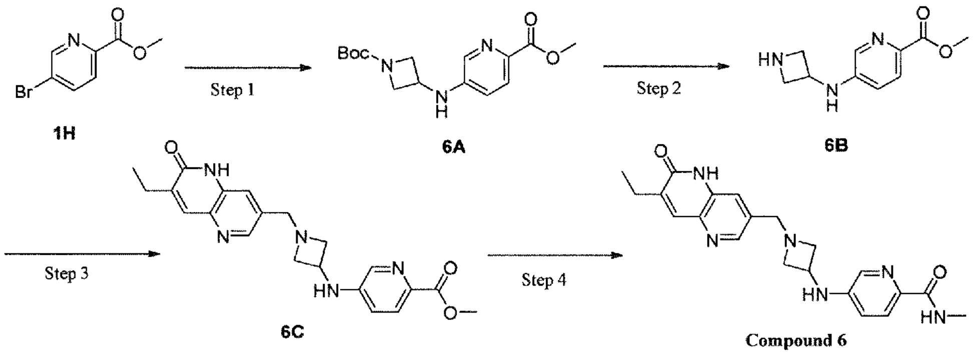

1H 6A 6B 6C Compound 6

Step 1:

Compound 1H (1.0 g, 4.63 mmol), 1-tert-butoxycarbonyl-3-aminocyclobutylamine (0.8 g, 4.63 mmol) and potassium carbonate (1.9 g, 13.89 mmol) were dissolved in N,N-dimethylformamide (20 mL), chloro(2-dicyclohexylphosphino-2',4',6'-triisopropyl-1,1'-biphenyl)[2-(2'-amino-1,1'-biphenyl)]palladium (II) (0.36 g, 0.46 mmol) was added, and under nitrogen protection, the mixture was heated to 95°C under microwave and reacted for 2 hours. The reaction liquid was concentrated and separated by column chromatography (dichloromethane : methanol = 20 : 1), to afford target compound 6A (1.0 g, 70.30%).
LC-MS (ESI): m/z = 308.3 $[M+H]^+$

Step 2:

Compound 6A (1.0 g, 3.15 mmol) was dissolved in a solution of hydrogen chloride in 1,4-dioxane (4M, 20 mL), and the mixture was reacted at room temperature for 4 hours, at which time a large amount of solid was observed to be precipitated out. The reaction liquid was filtered, washed twice with ethyl acetate and dried, to afford compound 6B (0.5 g, 71.20%).
LC-MS (ESI): m/z = 208.3$[M+H]^+$

Step 3:

Compound 6B (0.1 g, 0.45 mmol), intermediate 1G (0.1 g, 0.45 mmol) and potassium iodide (0.1 g, 0.69 mmol) were dissolved in N,N-dimethylformamide (3 mL), N,N-diisopropylethylamine (1 mL) was added, and the mixture was heated to 80°C and reacted for 3 hours. The reaction liquid was concentrated and separated by column chromatography (dichloromethane : methanol = 20 : 1), to afford compound 6C (0.1 g, 60.28%).
LC-MS (ESI): m/z = 394.2 $[M+H]^+$

Step 4:

Compound 6C (0.1 g, 0.25 mmol) was dissolved in methanol (5 mL), methylamine aqueous solution (2 mL) was added, and the mixture was reacted at room temperature for 5 hours. The reaction liquid was concentrated and purified by C-18 reverse phase column chromatography (acetonitrile: water = 30 : 70), to afford compound 6 (8 mg, 4.25%).

LC-MS (ESI): m/z = 393.3 $[M+H]^+$
$^1H$ NMR (400 MHz, DMSO-J6) δ 11.81 (s, 2H), 8.36 - 8.26 (m, 2H), 7.89 (d, 1H), 7.76 - 7.70 (m, 2H), 7.55 (s, 1H), 6.93 - 6.90 (m, 2H), 4.13 - 4.08 (m, 1H), 3.70 - 3.65 (m, 4H), 2.95 (t, 2H), 2.76 (d, 3H), 2.57 - 2.52 (m, 2H), 1.18 (t, 3H).

Example 7

5-((1-((7-ethyl-6-oxo-5,6-dihydro-1,5-naphthyridin-3-yl)methyl)pyrrolidin-3-yl)amino)-N-methylpicolinamide (Compound 7)

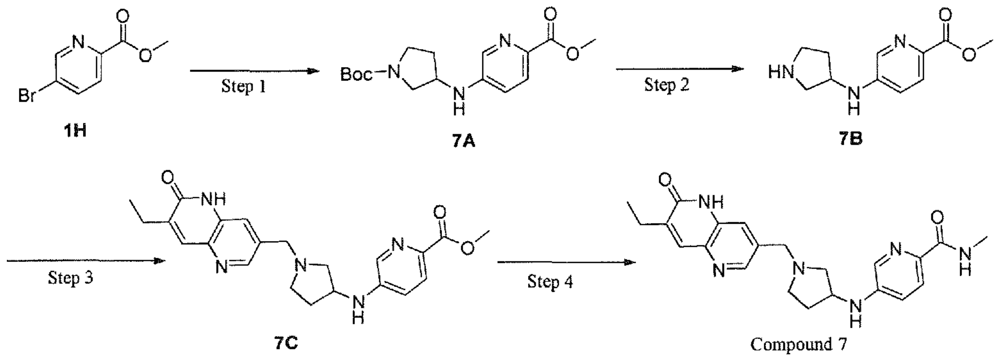


1H, 7A, 7B, 7C, Compound 7

Step 1:

Compound 1H (1.0 g, 4.63 mmol), tert-butyl 3-aminopyrrolidine-1-carboxylate (0.8 g, 4.63 mmol) and potassium carbonate (1.9 g, 13.89 mmol) were dissolved in N,N-dimethylformamide (20 mL), chloro(2-dicyclohexylphosphino-2',4',6'-triisopropyl-1,1'-biphenyl)[2-(2'-amino-1,1'-bipphenyl)]palladium (II) (0.36 g, 0.46 mmol) was added, and under nitrogen protection, the mixture was heated to 95°C under microwave and reacted for 2 hours. The reaction liquid was concentrated and separated by column chromatography (dichloromethane : methanol = 20 : 1), to afford target compound 7A (1.1 g, 77.30%).
LC-MS (ESI): m/z = 322.3 $[M+H]^+$

Step 2:

Compound 7A (1.0 g, 3.11 mmol) was dissolved in a solution of hydrogen chloride in 1,4-dioxane (4M, 20 mL), and the mixture was reacted at room temperature for 4 hours, at which time a large amount of solid was observed to be precipitated out. The reaction liquid was filtered, washed twice with ethyl acetate and dried, to afford compound 7B (0.5 g, 72.66%).
LC-MS (ESI): m/z = 222.1$[M+H]^+$

Step 3:

Compound 7B (0.1 g, 0.45 mmol), intermediate 1G (0.1 g, 0.45 mmol) and potassium iodide (0.1 g, 0.69 mmol) were dissolved in N,N-dimethylformamide (3 mL), N,N-diisopropylethylamine (1 mL) was added, and the mixture was heated to 80°C and reacted for 3 hours. The reaction liquid was concentrated and separated by column chromatography (dichloromethane : methanol = 20 : 1), to afford compound 7C (0.12 g, 65.28%).
LC-MS (ESI): m/z = 408.5 $[M+H]^+$

Step 4:

Compound 7C (0.1 g, 0.25 mmol) was dissolved in methanol (5 mL), methylamine aqueous solution (2 mL) was added, and the mixture was reacted at room temperature for 5 hours. The reaction liquid was concentrated and purified by C-18 reverse phase column chromatography (acetonitrile : water= 30 : 70), to afford compound 7 (40 mg, 39.36%).

LC-MS (ESI): m/z = 407.5 $[M+H]^+$
$^1$H NMR (400 MHz, $CD_3OD$) δ 8.60 (d, 1H), 8.00 - 7.83 (m, 4H), 7.08 - 7.04 (m, 1H), 4.62 (s, 2H), 4.38 (s, 1H), 3.93 - 3.36 (m, 4H), 2.92 (s, 3H), 2.65 - 2.71(m, 3H), 2.15 (s, 1H), 1.28 (t, 3H).

Example 8

5-(6-((7-ethyl-6-oxo-5,6-dihydro-1,5-naphthyridin-3-yl)methyl)-2,6-diazaspiro[3.3]heptan-2-yl)-N-methylpicolinamide (Compound 8)

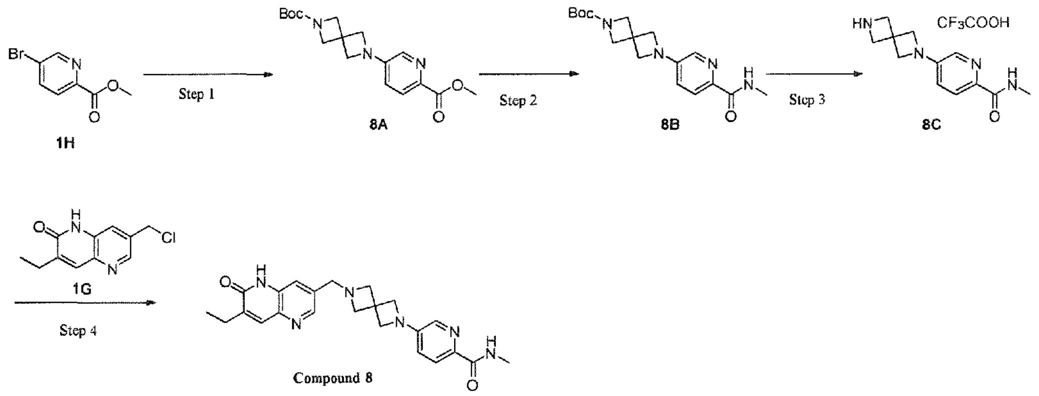


Step 1:

Methyl 5-bromopicolinate 1H (500 mg, 2.31 mmol) and tert-butyl 2,6-diazaspiro[3.3]heptane-2-carboxylate (562 mg, 2.31 mmol) were dissolved in toluene (10 mL), caesium carbonate (3.0 g, 9.24 mmol), triethylamine, BINAP (863 mg, 1.39 mmol) and $Pd_2(dba)_3$ (635 mg, 0.69 mmol) were added, and the mixture was reacted overnight at 100°C under nitrogen protection. Subsequently, the reaction liquid was quenched with water (15 mL) and extracted with ethyl acetate (20 mL × 3). The organic phases were combined, dried over anhydrous $Na_2SO_4$, filtered, spun to dryness, and separated by silica gel chromatographic column (PE : EA = 1 : 0-1 : 1), to afford title compound 8A (590 mg, crude).

LC-MS (ESI): m/z= 334.1 $[M+H]^+$.

Step 2:

8A (590 mg, crude) was dissolved in methanol (5 mL), methylamine aqueous solution (5 mL, 40%) was added, and the mixture was reacted at room temperature for 4 hours. The suspension was concentrated, saturated ammonium chloride solution was added, and the mixture was extracted with dichloromethane. The organic phases were combined, dried over anhydrous sodium sulphate, filtered and spun to dryness, to afford title compound 8B (510 mg, crude).

LC-MS (ESI): m/z= 333.2 $[M+H]^+$.

Step 3:

8B (450 mg, crude) was dissolved in dichloromethane (5 mL), trifluoroacetic acid (1 mL) was added, and the mixture was reacted at room temperature for 1 hour. Water (25 mL) and dichloromethane (10 mL) were added, the organic phase was separated and discarded, and the aqueous phase was spun to dryness, to afford title compound 8C (250 mg, 31.2%, over three steps).

LC-MS (ESI): m/z= 233.1 $[M+H]^+$.

Step 4:

1G (100 mg, 0.45 mmol) and 8C (156 mg, 0.45 mmol) were dissolved in anhydrous acetonitrile (5 mL), potassium iodide (8 mg, 0.05 mmol) and DIPEA (290 mg, 2.25 mmol) were added, and the mixture was subjected to nitrogen replacement and reacted at 80°C for 2 hours. Upon complete depletion of raw materials monitored by LCMS, the system was concentrated, saturated sodium bicarbonate solution (20 mL) was added, and the mixture was extracted with dichloromethane (10 mL × 3). The organic phases were combined, dried over anhydrous sodium sulphate, concentrated, and passed through column (DCM : MeOH = 1 : 0-10 : 1), to afford compound 8 (112 mg, 59.5%).

LC-MS (ESI): m/z= 419.2 $[M+H]^+$.

$^1H$ NMR (400 MHz, DMSO-$d_6$) δ 11.81 (s, 1H), 8.34 (d, 1H), 8.30 (q, 1H), 7.80 (d, 1H), 7.75 (d, 1H), 7.73 (s, 1H), 7.55 (s, 1H), 6.87 (dd, 1H), 4.06 (s, 4H), 3.66 (s, 2H), 3.37 (s, 4H), 2.77 (d, 3H), 2.58 - 2.52 (m, 2H), 1.18 (t, 3H).

Example 9

5-(6-((7-ethyl-6-oxo-5,6-dihydro-1,5-naphthyridin-3-yl)methyl)-2,6-diazaspiro[3.4]octan-2-yl)-N-methylpicolinamide (Compound 9)

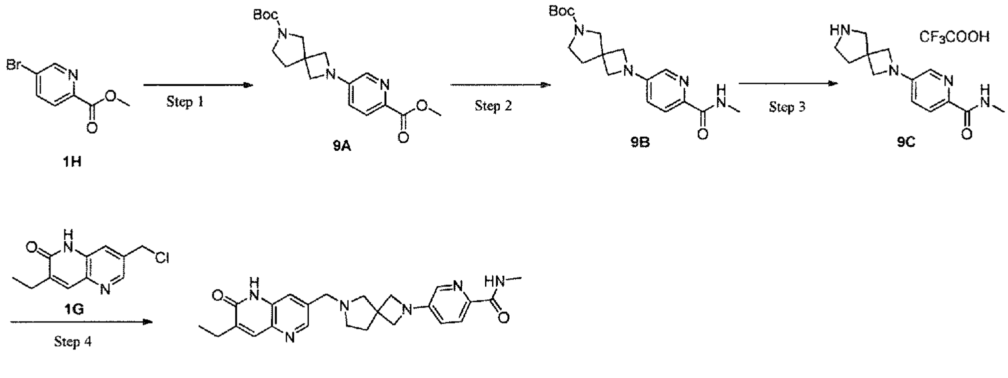


Compound 9

Step 1:

Methyl 5-bromopicolinate 1H (500 mg, 2.31 mmol) and tert-butyl 2,6-diazaspiro[3.4]octane-6-carboxylate (562 mg, 2.31 mmol) were dissolved in toluene (10 mL), caesium carbonate (3.0 g, 9.24 mmol), triethylamine, BINAP (863 mg, 1.39 mmol) and $Pd_2(dba)_3$ (635 mg, 0.69 mmol) were added, and the mixture was reacted overnight at 100°C under nitrogen protection. Subsequently, the reaction liquid was quenched with water (15 mL) and extracted with ethyl acetate (20 mL × 3). The organic phases were combined, dried over anhydrous $Na_2SO_4$, filtered, spun to dryness, and separated by silica gel chromatographic column (PE : EA = 1 : 0-1 : 1), to afford title compound 9A (590 mg, crude) as a yellow solid.
LC-MS (ESI): m/z= 348.1 $[M+H]^+$.

Step 2:

9A (590 mg, crude) was dissolved in methanol (5 mL), methylamine aqueous solution (5 mL, 40%) was added, and the mixture was reacted at room temperature for 4 hours. The suspension was concentrated, saturated ammonium chloride solution was added, and the mixture was extracted with dichloromethane. The organic phases were combined, dried over anhydrous sodium sulphate, filtered and spun to dryness, to afford title compound 9B (510 mg, crude).
LC-MS (ESI): m/z= 347.2 $[M+H]^+$.

Step 3:

9B (450 mg, crude) was dissolved in dichloromethane (5 mL), trifluoroacetic acid (1 mL) was added, and the mixture was reacted at room temperature for 1 hour. Water (25 mL) and dichloromethane (10 mL) were added, the organic phase was separated and discarded, and the aqueous phase was spun to dryness, to afford title compound 9C (250 mg, 31.2%, over three steps).
LC-MS (ESI): m/z= 247.1 $[M+H]^+$.

Step 4:

1G (50 mg, 0.22 mmol) and 9C (65 mg, 0.26 mmol) were dissolved in anhydrous acetonitrile (5 mL), potassium iodide (4 mg, 0.02 mmol) and DIPEA (142 mg, 1.10 mmol) were added, and the mixture was subjected to nitrogen replacement and reacted at 80°C for 2 hours. Upon complete depletion of raw materials monitored by LCMS, the system was concentrated, saturated sodium bicarbonate solution (20 mL) was added, and the mixture was extracted with dichloromethane (10 mL × 3). The organic phases were combined, dried over anhydrous sodium sulphate, filtered, concentrated, and passed through column (DCM : MeOH = 1 : 0-10 : 1), to afford compound 9 (65 mg, 68.3%).
LC-MS (ESI): m/z= 433.2 $[M+H]^+$.
$^1H$ NMR (400 MHz, DMSO-$d_6$) δ 11.81 (s, 1H), 8.38 (d, 1H), 8.31 (q, 1H), 7.79 (d, 1H), 7.76 - 7.72 (m, 2H),

7.60 (s, 1H), 6.86 (dd, 1H), 3.96 - 3.84 (m, 4H), 3.71 (s, 2H), 2.80 - 2.74 (m, 5H), 2.62 - 2.56 (m, 2H), 2.56 - 2.52 (m, 2H), 2.09 (t, 2H), 1.18 (t, 3H).

Example 10

5-(2-((7-ethyl-6-oxo-5,6-dihydro-1,5-naphthyridin-3-yl)methyl)-2,6-diazaspiro[3.4]octan-6-yl)-N-methylpicolinamide (Compound 10)

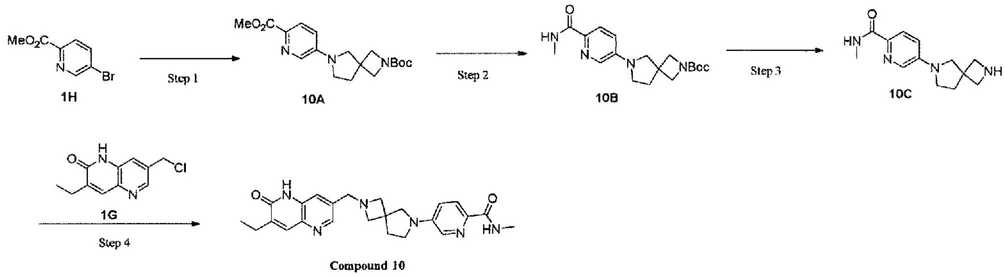


Step 1:

Under nitrogen protection, compound methyl 5-bromopicolinate 1H (1.20 g, 5.55 mmol), tert-butyl 2,6-diazaspiro[3.4]octane-2-carboxylate (1.42 g, 6.67 mmol), tris(dibenzylideneacetone)dipalladium (1.53 g, 1.67 mmol), 1,1'-binaphthyl-2,2'-bisdiphenylphosphine (2.08 g, 3.33 mmol) and caesium carbonate (7.22 g, 22.2 mmol) were all added to a reaction flask, toluene (50 mL) was added, and the mixture was warmed to 100°C and reacted overnight. After the reaction was completed as monitored by TLC, the reaction liquid was filtered through diatomaceous earth and washed with ethyl acetate. The organic phase was concentrated, and the resulting residue was separated and purified by silica gel column chromatography (petroleum ether : ethyl acetate (v/v) = 1 : 1 to 1 : 10), to afford 10A (1.37 g, 49.7%) as a yellow solid.
LC-MS (ESI): m/z =348.2 $[M+H]^+$

Step 2:

Compound 10A (1.37 g, 3.95 mmol) was dissolved in methanol (20 mL), methylamine aqueous solution (20 mL) was added, and the mixture was reacted overnight at room temperature. After the reaction was completed as monitored by LCMS, the reaction liquid was directly concentrated and directly used in the next step without purification.
LC-MS (ESI): m/z =347.1 $[M+H]^+$

Step 3:

Compound 10B (500 mg, crude) was dissolved in dry dichloromethane (16 mL), trifluoroacetic acid (4 mL) was added at room temperature, and the mixture was reacted at room temperature for 3 hours after the addition was completed. After the reaction was completed as monitored by TLC, the reaction liquid was washed with water (10 mL × 4), and the resulting aqueous phase was in turn washed with ethyl acetate (5 mL×1). The aqueous phase was lyophilized, to afford compound 10C (140 mg, 39.3%).
LC-MS (ESI): m/z =247.2 $[M+H]^+$

Step 4:

Compound 1G (100 mg, 0.448 mmol), 10C (121 mg, 0.493 mmol), N,N-diisopropylethylamine (292 mg, 2.24 mmol), and potassium iodide (7.42 mg, 0.0448 mmol) were all added to a reaction tube, dry acetonitrile (5 mL) was added, and the mixture was warmed to 80°C and reacted for about 5 hours. After the reaction was completed as monitored by TLC, water (5 mL) was added, and the mixture was extracted with ethyl acetate (3 mL × 10). The organic phases were combined, dried over anhydrous sodium sulphate, filtered and concentrated, and the resulting crude was separated and purified by thick silica gel plate (dichloromethane/ethyl acetate/methanol = 10 : 1 : 1), to afford compound 10 (86 mg, 44%) as a white solid.

LC-MS (ESI): m/z =433.3 $[M+H]^+$

$^1H$ NMR (400 MHz, Chloroform-d): δ 12.37 (s, 1H), 8.45 (d, 1H), 7.99 (d, 1H), 7.84 (s, 1H), 7.78 (d, 1H), 7.73 (q, 1H), 7.68 (d, 1H), 6.79 (dd, 1H), 3.77 (s, 2H), 3.54 - 3.44 (m, 3H), 3.36 (t, 2H), 3.30 (s, 3H), 2.98 (d, 3H), 2.74 (q, 2H), 2.23 (t, 2H), 1.31 (t, 3H).

Example 11

5-(3-((7-ethyl-6-oxo-5,6-dihydro-1,5-naphthyridin-3-yl)methyl)-3,6-diazabicyclo[3.1.1]heptan-6-yl)-N-methylpicolinamide (Compound 11)

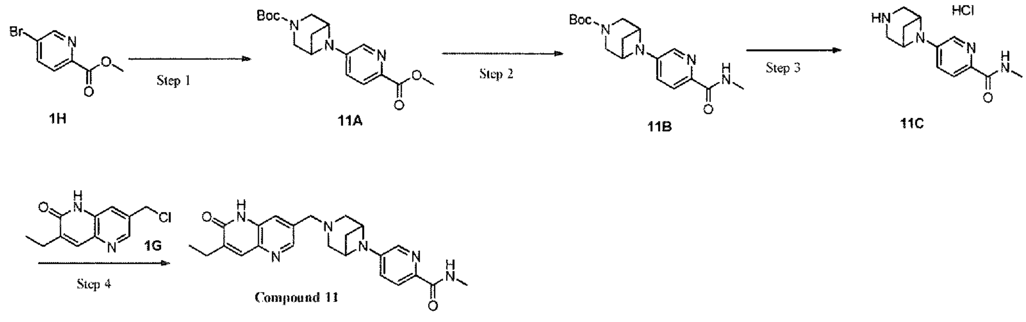


1H 11A 11B 11C 1G Compound 11

Step 1:

Methyl 5-bromopicolinate 1H (544 mg, 2.52 mmol) and tert-butyl 3,6-diazabicyclo[3.1.1]heptane-3-carboxylate (551 mg, 2.78 mmol) were dissolved in 1,4-dioxane (25 mL), caesium carbonate (1.6 g, 5.04 mmol) and RuPhos-Pd-G3 (210 mg, 0.25 mmol) were added, and the mixture was reacted overnight at 100°C under nitrogen protection. Subsequently, the reaction liquid was quenched with water (15 mL) and extracted with ethyl acetate (20 mL×3). The organic phases were combined, dried over anhydrous sodium sulphate, filtered, spun to dryness, and separated by silica gel chromatographic column (PE : EA = 1 : 0-1 : 1), to afford title compound 11A (797 mg, 94.9%) as a light yellow solid.

LC-MS (ESI): m/z= 334.1 $[M+H]^+$.

Step 2:

11A (797 mg, 2.39 mmol) was dissolved in methanol (5 mL), methylamine aqueous solution (5 mL, 40%) was added, and the mixture was reacted at room temperature for 4 hours. The suspension was concentrated, saturated ammonium chloride solution was added, and the mixture was extracted with dichloromethane. The organic phases were combined, dried over anhydrous sodium sulphate, filtered and spun to dryness, to afford title compound 11B (795 mg, 99.9%).

LC-MS (ESI): m/z= 333.2 $[M+H]^+$.

Step 3:

11B (790 mg, 1.36 mmol) was dissolved in methanol (5 mL), and a solution of hydrogen chloride in dioxane (5 mL, 4M) was added. The mixture was reacted at room temperature for two hours and spun to dryness, to afford title compound 11C (780 mg, crude).

LC-MS (ESI): m/z= 223.1 $[M+H]^+$.

Step 4:

1G (50 mg, 0.22 mmol) and 11C (60 mg, 0.27 mmol) were dissolved in anhydrous acetonitrile (5 mL), potassium iodide (4 mg, 0.02 mmol) and DIPEA (144 mg, 1.12 mmol) were added, and the mixture was subjected to nitrogen replacement and reacted at 80°C for 2 hours. Upon complete depletion of raw materials monitored by LCMS, the system was concentrated, saturated sodium bicarbonate solution (20 mL) was added, and the mixture was extracted with dichloromethane (10 mL × 3). The organic phases were combined, dried over anhydrous sodium sulphate, concentrated,

and passed through column (DCM : MeOH = 1 : 0-10 : 1), to afford compound 11 (56 mg, 60.8%).

$^1$H NMR (400 MHz, DMSO-$d_6$) $\delta$ 11.72 (s, 1H), 8.31 (d, I H), 8.07 (d, 1H), 7.82 (d, 1H), 7.78 (d, 1H), 7.68 (d, 1H), 7.43 (d, 1H), 6.98 (dd, 1H), 4.41 (d, 2H), 3.59 (s, 2H), 3.08 (d, 2H), 2.86 - 2.75 (m, 5H), 2.65 - 2.55 (m, 1H), 2.55 - 2.51 (m, 2H), 2.00 - 1.95 (m, 1H), 1.17 (t, 3H).

LC-MS (ESI): m/z= 419.2 [M+H]$^+$.

Example 12

5-(4-((7-ethyl-6-oxo-5,6-dihydro-1,5-naphthyridin-3-yl)methyl)-4,7-diazaspiro[2.5]octan-7-yl)-N-methylpicolinamide (Compound 12)

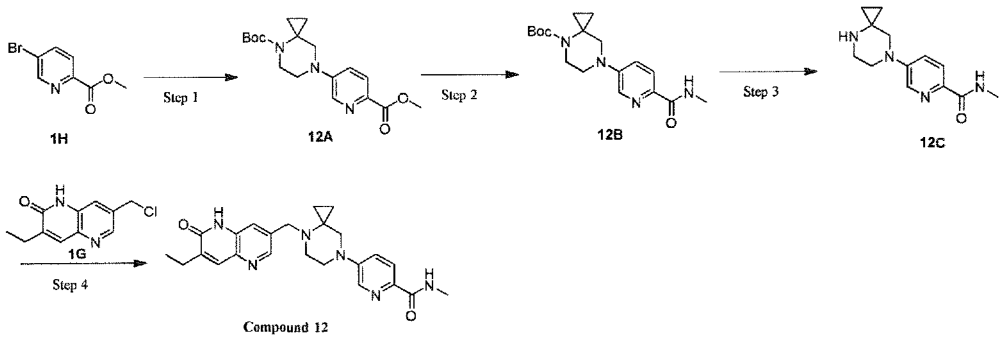

1H 12A 12B 12C
1G
Compound 12

Step 1:

Under nitrogen protection, methyl 5-bromopicolinate 1H (1.5 g, 6.94 mmol) was added to a flask, 1,4-dioxane (10 mL) was added, followed by tert-butyl 4,7-diazaspiro[2.5]octane-4-carboxylate (1.8 g, 8.33 mmol), caesium carbonate (4.5 g, 13.8 mmol) and Ru Phos Pd G3 (230 mg, 0.28 mmol), and the mixture was warmed to 100°C and reacted overnight at this temperature. Upon complete depletion of raw materials monitored by LCMS, the reaction liquid was quenched with water, extracted with ethyl acetate, washed, dried, concentrated and passed through column (PE : EA = 1 : 0-0 : 1), to afford title compound 12A (1.47 g, 61.1%).

LC-MS (ESI): m/z= 348.1 [M+H]$^+$.

Step 2:

Compound 12A (1.2 g, 3.45 mmol) was added to a flask, methylamine aqueous solution (12 mL) and methanol (12 mL) were added, and the mixture was reacted overnight at room temperature. Upon complete depletion of raw materials monitored by LCMS, the reaction liquid was directly concentrated and passed through column (PE : EA = 1 : 0-0 : 1), to afford title compound 12B (1.15 g, 95.8%).

LC-MS (ESI): m/z= 347.1 [M+H]$^+$.

Step 3:

Compound 12B (1.1 g, 3.17 mmol) was added to a flask, trifluoroacetic acid (3 mL) and dichloromethane (9 mL) were added, and the mixture was reacted at room temperature for 2 h. Upon complete depletion of raw materials monitored by LCMS, the reaction liquid was directly concentrated, to afford title compound 12C (1.0 g, crude).

LC-MS (ESI): m/z= 247.2 [M+H]$^+$.

Step 4:

Under nitrogen protection, compound 12C (60 mg, crude) was added to a flask, acetonitrile (2.0 mL) was added, followed by 7-(chloromethyl)-3-ethyl-1,5-naphthyridin-2(1H)-one 1G (37.7 mg, 0.17 mmol), potassium iodide (5.0 mg, 0.03 mmol) and DIEA (131.8 mg, 1.02 mmol), and the mixture was warmed to 80°C and reacted at this temperature for 2 hours. Upon complete depletion of raw materials monitored by LCMS, a product was generated. The reaction liquid was quenched with water, extracted with ethyl acetate, washed, dried, concentrated and passed through column (PE : EA = 1 : 0-0 : 1), to afford title compound 12 (42 mg, 57.5%).

$^1H$ NMR (400 MHz, Chloroform-*d*) δ 11.74 (s, 1H), 8.47 (d, 1H), 8.14 (d, 1H), 8.07 (d, 1H), 7.86 (s, 1H), 7.78 (q, 1H), 7.65 (s, 1H), 7.19 (dd, 1H), 4.04 (s, 2H), 3.34 (t, 2H), 3.18 (s, 2H), 3.08 (t, 2H), 3.01 (d, 3H), 2.77 - 2.70 (m, 2H), 1.32 (t, 3H), 0.88 (d, 2H), 0.71 (d, 2H).

LC-MS (ESI): m/z= 433.2 $[M+H]^+$.

Example 13

5-(1-[(7-ethyl-6-oxo-5,6-dihydro-1,5-naphthyridin-3-yl)methyl]piperidin-4-yl)-N-methylpyridine-2-carboxamide (Compound 13)

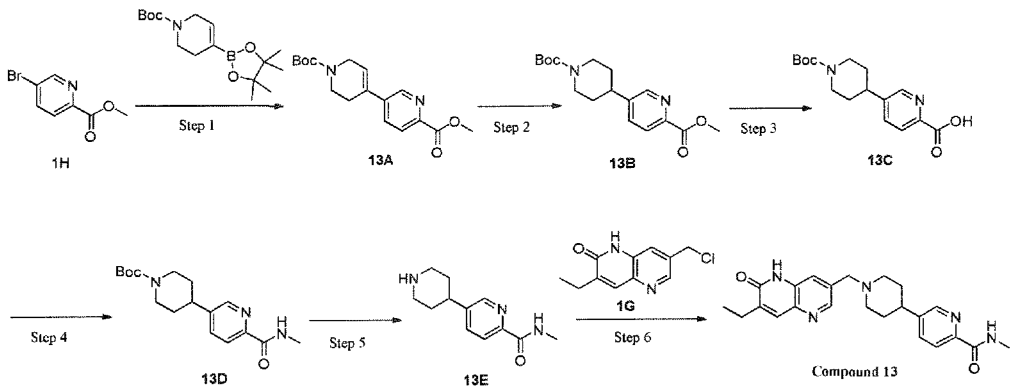


Step 1:

1H (0.7 g, 3.24 mmol), tert-butyl 4-(4,4,5,5-tetramethyl-1,3,2-dioxaborolan-2-yl)-3,6-dihydropyridine-1(2H)-carboxylate (1 g, 3.24 mmol), chloro[2-(dicyclohexylphosphino)-3,6-dimethoxy-2',4',6'-triisopropyl-1, 1'-biphenyl][2-(2-aminoethyl)phenyl]palladium (II) (RuPhos-Pd-G2, 260 mg, 0.32 mmol), and potassium carbonate (0.6 g, 4.44 mmol) were dissolved in anhydrous DMF (30 ml), and the mixture was reacted at 100°C under nitrogen protection for 3 hours. After the reaction was completed as monitored by LCMS, the reaction liquid was spun to dryness to remove the solvent, and the residue was separated by Flash silica gel column (PE : EA 0-100% 10CV, 70%), to afford compound 13A (1 g).

LC-MS (ESI): m/z= 319.2 $[M+H]^+$.

Step 2:

13A (0.3 g, 0.94 mmol) was dissolved in 10 mL of methanol, palladium on carbon (0.4 g) was added, and the mixture was subjected to hydrogen replacement, stirred and reacted at room temperature for 1 hour. After the reaction was completed as monitored by LCMS, the reaction liquid was filtered, and the reaction liquid was spun to dryness to remove the solvent, to afford compound 13B (0.25 g), which was directly used in the next reaction.

LC-MS (ESI): m/z= 321.2 $[M+H]^+$.

Step 3:

13B (0.25 g, 0.78 mmol) was dissolved in water (1 mL) and dioxane (5 mL), sodium hydroxide was added, and the mixture was stirred and reacted at 80°C for 2 hours. After the reaction was completed as monitored by TLC, 0.5M hydrochloric acid was added to adjust to pH 3, and the mixture was extracted twice with ethyl acetate and concentrated, to afford compound 13C (0.25 g), which was directly used in the next reaction.

LC-MS (ESI): m/z= 307.1 $[M+H]^+$.

Step 4:

13C (0.35 g, 1.14 mmol), a solution of methylamine in THF (2M, 1 mL), and diisopropylethylamine (0.44 g, 3.42 mmol) were dissolved in a reaction flask, 2-(7-aza-1H-benzotriazol-1-yl)-1,1,3,3-tetramethyluronium hexafluorophosphate (HATU, 0.65 g, 1.71 mmol) was added, and the mixture was stirred and reacted at 40°C for 4 hours. After the reaction was completed as monitored by LCMS, the reaction liquid was extracted with ethyl acetate/water, and the

organic phase was washed three times with water and concentrated, to afford compound 13D (300 mg), which was directly used in the next reaction.

LC-MS (ESI): m/z= 320.3 [M+H]+.

Step 5:

13D (0.36 g, 1.14 mmol) was dissolved in methanol (2 ml) and a solution of hydrogen chloride in dioxane (4 M, 10 ml), and the mixture was stirred and reacted at room temperature for 1.5 hours. After the reaction was completed as monitored by LCMS, the reaction liquid was spun to dryness to remove the solvent, and the residue was directly used in the next reaction.

LC-MS (ESI): m/z= 220.2 [M+H]+.

Step 6:

13E (100 mg, 0.46 mmol) and 1G (100 mg, 0.46 mmol) were dissolved in anhydrous DMF, 1 mL of triethylamine and 100 mg of potassium iodide were added, and the mixture was stirred and reacted at 80°C for 2 hours. After the reaction was completed as monitored by LC-MS, the reaction liquid was spun to dryness and subjected to preparative HPLC with preparative HPLC separation methods: 1. Instruments: waters 2767 (preparative liquid phase chromatographic instrument); chromatographic column: SunFire@ PrepC18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 15 mL/min; d. elution time: 20 min, retention time: 8.5 min, to afford compound 13 (50 mg, 26.8%).

$^1$H NMR (400 MHz, DMSO-$d_6$) δ 12.18 (s, 1H), 9.61 (s, 1H), 8.67 (q, 2H), 8.56 (s, 1H), 8.51 (d, 1H), 8.00 (d, 1H), 7.81 (s, 2H), 7.78 (s, 1H), 4.52 (d, 3H), 3.21 - 3.07 (m, 1H), 2.81 (d, 3H), 2.58 (q, 3H), 2.33 (s, 1H), 2.08 (d, 1H), 1.20 (t, 4H).

LC-MS (ESI): m/z= 406.1 [M+H]+.

Example 14

1'-((7-ethyl-6-oxo-5,6-dihydro-1,5-naphthyridin-3-yl)methyl)-N-methyl-1',2',3',6'-tetrahydro-[3,4'-bipyridine]-6-carboxamide (Compound 14)

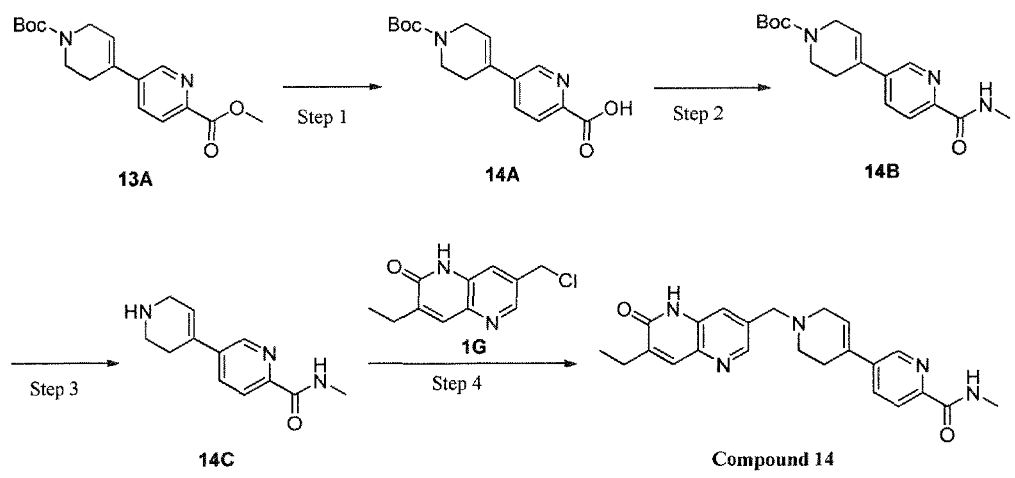


13A 14A 14B 1G 14C Compound 14

Step 1:

13A (0.3 g, 0.94 mmol) was dissolved in dioxane (5 ml) and water (1 mL), sodium hydroxide (0.5 g, 12.5 mmol) was added, and the mixture was stirred and reacted at 60°C for 2 hours. After the reaction was completed as monitored by LCMS, 0.5M hydrochloric acid was added to adjust to pH 3, and the mixture was extracted twice with ethyl acetate and concentrated, to afford compound 14A (0.3 g), which was used in the next reaction.

LC-MS (ESI): m/z= 305.1 [M+H]+.

Step 2:

14A (0.35 g, 1.14 mmol), a solution of methylamine in tetrahydrofuran (2M, 1.5 mL), and diisopropylethylamine (0.44 g, 3.42 mmol) were dissolved in a reaction flask, 2-(7-aza-1H-benzotriazol-1-yl)-1,1,3,3-tetramethyluronium hexafluorophosphate (HATU, 0.65 g, 1.71 mmol) was added, and the mixture was stirred and reacted at 40°C for 4 hours. After the reaction was completed as monitored by LCMS, the reaction liquid was extracted with ethyl acetate/water, and the organic phase was washed three times with water and concentrated, to afford compound 14B (300 mg), which was used in the next reaction.
LC-MS (ESI): m/z= 318.2 $[M+H]^+$.

Step 3:

14B (0.36 g, 1.14 mmol) was dissolved in methanol (2 mL) and a solution of hydrogen chloride in dioxane (4 M, 10 mL), and the mixture was stirred and reacted at room temperature for 1.5 hours. After the reaction was completed as monitored by LCMS, the reaction liquid was spun to dryness to remove the solvent to afford 14C, which was directly used in the next reaction.
LC-MS (ESI): m/z= 218.2 $[M+H]^+$.

Step 4:

14C (100 mg, 0.46 mmol), 1G (100 mg, 0.46 mmol), triethylamine (0.7 g, 6.9 mmol), and potassium iodide (100 mg, 0.69 mmol) were dissolved in anhydrous DMF, and the mixture was stirred and reacted at 80°C for 2 hours. After the reaction was completed as monitored by LCMS, the reaction liquid was spun to dryness to remove DMF, and the residue was subjected to preparative HPLC with preparative HPLC separation methods: 1. Instruments: waters 2767 (preparative liquid phase chromatographic instrument); chromatographic column: SunFire@ PrepC18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 15 mL/min; d. elution time: 20 min, retention time: 8.5 min, to afford compound 14 (20 mg, 10.8%).
$^1$H NMR (400 MHz, DMSO-*d6*) δ 11.83 (s, 1H), 8.69 (d, 2H), 8.43 (s, 1H), 8.00 (s, 2H), 7.77 (s, 1H), 7.65 (s, 1H), 6.42 (s, 1H), 3.73 (s, 2H), 3.17 (s, 1H), 2.82 (d, 4H), 2.69 (d, 2H), 2.64 - 2.51 (m, 4H), 1.19 (t, 3H).
LC-MS (ESI): m/z= 404.1 $[M+H]^+$.

Example 15

5-(2-((7-ethyl-6-oxo-5,6-dihydro-1,5-naphthyridin-3-yl)methyl)-2,7-diazaspiro[3.5]nonan-7-yl)-N-methylpicolinamide (Compound 15)

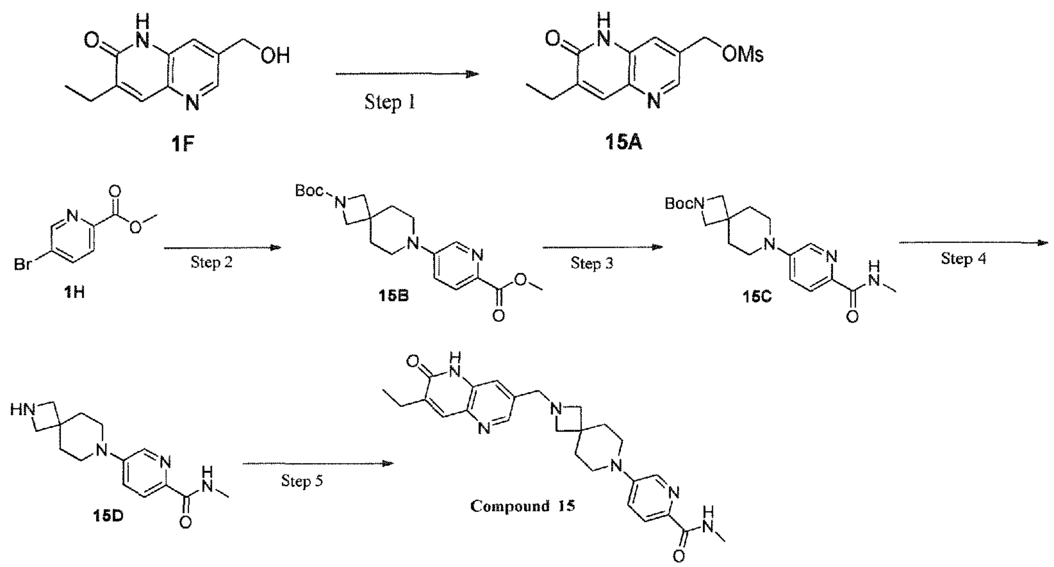


1F 15A 1H 15B 15C 15D Compound 15

Step 1:

Compound 1F (0.4 g, 1.96 mmol) was dissolved in dichloromethane (10 mL), triethylamine (0.4 g, 3.92 mmol) and methanesulfonyl chloride (0.27 g, 2.35 mmol) were successively added, and the mixture was reacted at room temperature for 2 h, concentrated and separated by column chromatography (dichloromethane : methanol = 20 : 1), to afford 15A (0.22 g, 39.76%).
LC-MS (ESI): m/z = 283.1$[M+H]^+$

Step 2:

Compound 1H (1.0 g, 4.63 mmol), 2-tert-butoxycarbonyl-2,7-diazaspiro[3.5]nonane (1.05 g, 4.63 mmol) and potassium carbonate (1.9 g, 13.89 mmol) were dissolved in N,N-dimethylformamide (20 mL), chloro(2-dicyclohexylphosphino-2',4',6'-triisopropyl-1,1'-biphenyl)[2-(2'-amino-1,1'-biphenyl)]palladium (II) (0.36 g, 0.46 mmol) was added, and under nitrogen protection, the mixture was heated to 120°C and reacted for 8 hours. The reaction liquid was concentrated and separated by column chromatography (dichloromethane : methanol = 20 : 1), to afford target compound 15B (1.2 g, 71.71%).
LC-MS (ESI): m/z =362.2$[M+H]^+$

Step 3:

Compound 15B (0.1 g, 0.28 mmol) was dissolved in 5 mL of methanol, methylamine aqueous solution (0.11 mL) was added, and the mixture was reacted at room temperature for 5 hours. The reaction liquid was concentrated to afford compound 15C (0.09 g, 89.17%).
LC-MS (ESI): m/z = 361.1$[M+H]^+$

Step 4:

Compound 15C (0.1 g, 0.28 mmol) was dissolved in a solution of hydrogen chloride in 1,4-dioxane (4M, 1.4 mL), and the mixture was reacted at room temperature for 4 hours. Upon complete depletion of raw materials monitored by TLC (dichloromethane : methanol = 20 : 1), a large amount of solid was observed to be precipitated out, and the reaction liquid was filtered, washed twice with ethyl acetate and dried, to afford compound 15D (0.06 g, 82.31%).

Step 5:

Compound 15D (0.05 g, 0.19 mmol), 15A (0.05 g, 0.18 mmol) and potassium iodide (0.1 g, 0.69 mmol) were dissolved in N,N-dimethylformamide (3 mL), N,N-diisopropylethylamine (1 mL) was added, and the mixture was heated to 80°C and reacted for 3 hours. The reaction liquid was concentrated and separated by column chromatography (dichloromethane : methanol = 20 : 1), to afford compound 15 (0.03 g, 37.32%).

LC-MS (ESI): m/z = 447.5$[M+H]^+$
$^1$H NMR (400 MHz, $CDCl_3$) $\delta$ 8.43 (d, 1H), 8.14 (d, 1H), 8.03 (d, 1H), 7.79 - 7.69 (m, 2H), 7.21 - 7.18 (m, 1H), 4.10 (s, 2H), 3.35 - 3.20 (m, 8H), 3.00 (d , 3H), 2.66 (d, 2H), 2.04 (s, 4H), 1.28 (t, 3H).

Example 16

3-ethy1-7-((3-(6-(5-methyl-1,3,4-oxadiazol-2-yl)pyridin-3-yl)-3,8-diazabicyclo[3.2.1]octan-8-yl)methyl)-1,5-naphthyridin-2(1H)-one (Compound 16)

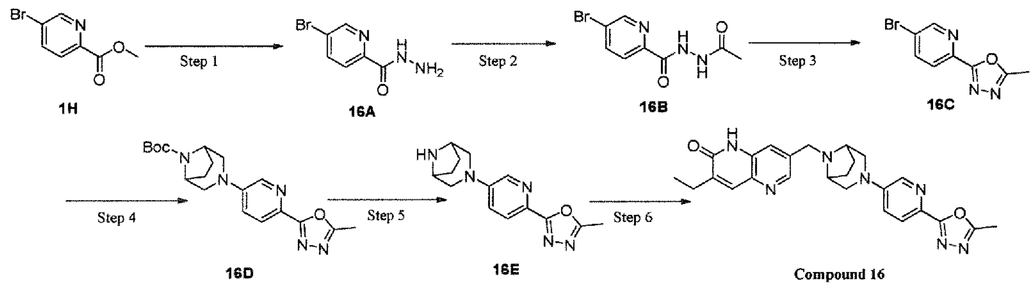


Step 1:

Compound 1H (2.0 g, 9.26 mmol) and hydrazine hydrate (2.9 g, 46.3 mmol) were dissolved in methanol (50 mL), and the mixture was reacted at 25°C for 2 hours. Upon complete depletion of raw materials monitored by TLC (dichloromethane : methanol = 20 : 1), the reaction liquid was concentrated, 50 mL of water was added, at which time a large amount of solid was observed to be precipitated out, and the mixture was filtered and dried, to afford target compound 16A (1.9 g, 94.97%).

Step 2:

Compound 16A (1 g, 4.63 mmol) and triethylamine (1.4 g, 13.89 mmol) were added to dichloromethane (15 mL), acetic anhydride (0.51 g, 5.09 mmol) was added dropwise at 25°C, and the mixture was reacted and stirred for 1.5 hours. Subsequently, the reaction liquid was poured into ice water, and the solid was filtered off, washed with water and dried, to afford compound 16B (1.1 g, 92.06%).

Step 3:

Compound 16B (1 g, 3.87 mmol) was dissolved in dichloromethane (10 mL), triethylamine (2.6 g, 25.70 mmol) and p-toluenesulfonyl chloride (0.9 g, 4.72 mmol) were then added, and the reaction liquid was stirred at room temperature for 1.5 h. After the reaction was completed as monitored by TLC, saturated sodium bicarbonate was added, and the mixture was extracted with dichloromethane. The organic phases were combined, dried over anhydrous sodium sulphate, concentrated, and separated by column chromatography (dichloromethane : methanol = 20 : 1), to afford compound 16C (0.8 g, 87.19%).
LC-MS (ESI): m/z = 241.1 $[M+H]^+$

Step 4:

Compound 16C (0.8 g, 3.33 mmol), tert-butyl 3,8-diazabicyclo[3.2.1]octane-8-carboxylate (0.71 g, 3.33 mmol) and potassium carbonate (1.38 g, 9.99 mmol) were dissolved in N,N-dimethylformamide (5 mL), and under nitrogen protection, (chloro(2-dicyclohexylphosphino-2',4',6'-triisopropyl-1,1'-biphenyl)[2-(2'-amino-1,1'-biphenyl)]palladium (II)) (0.26 g, 0.33 mmol) was added, and under nitrogen protection, the mixture was reacted at 120°C for 8 h, concentrated, and separated by column chromatography (dichloromethane : methanol = 10 : 1), to afford compound 16D (0.8 g, 64.68%).
LC-MS (ESI): m/z = 372.4 $[M+H]^+$

Step 5:

Compound 16D (0.5 g, 1.35 mmol) was dissolved in a solution of hydrogen chloride in 1,4-dioxane (4M, 5 mL), and the mixture was reacted at room temperature for 4 hours. Upon complete depletion of raw materials monitored by TLC (dichloromethane : methanol = 20 : 1), a large amount of solid was observed to be precipitated out, and the reaction liquid was filtered, washed twice with ethyl acetate and dried, to afford compound 16E (0.28 g, 76.44%).

Step 6:

Compound 16E (0.13 g, 0.49 mmol), 1G (0.1 g, 0.49 mmol), potassium iodide (0.12 g, 0.73 mmol) and N,N-diisopropylethylamine (0.95 g, 7.35 mmol) were dissolved in anhydrous DMF (2 mL), and the mixture was stirred and reacted at 80°C for 3 h. After the reaction was completed as monitored by LC-MS, the reaction liquid was spun to dryness

to remove the solvent, and the residue was separated by column chromatography (dichloromethane : methanol = 10 : 1), to afford compound 16 (0.05 g, 22.30%).

LC-MS (ESI): m/z = 458.5[M+H]$^+$

$^1$H NMR (400 MHz, DMSO-*d6*) δ 11.80 (s, 1H), 8.44 (d, 1H), 8.31 (d, 1H), 7.91 (d, 1H), 7.75 (s, 2H), 7.35 - 7.30 (m, 1H), 3.72 (s, 2H), 3.60 (d, 2H), 3.35 (s, 2H), 3.06 (d, 2H), 2.59 - 2.52 (m, 5H), 2.10 - 2.01 (m, 2H), 1.71 (d, 2H), 1.24 (s, 1H), 1.18 (t, 3H).

Example 17:

5-((1-((7-ethyl-6-oxo-5,6-dihydro-1,5-naphthyridin-3-yl)methyl)piperidin-4-yl)amino)-N-methylpicolinamide (Compound 17)

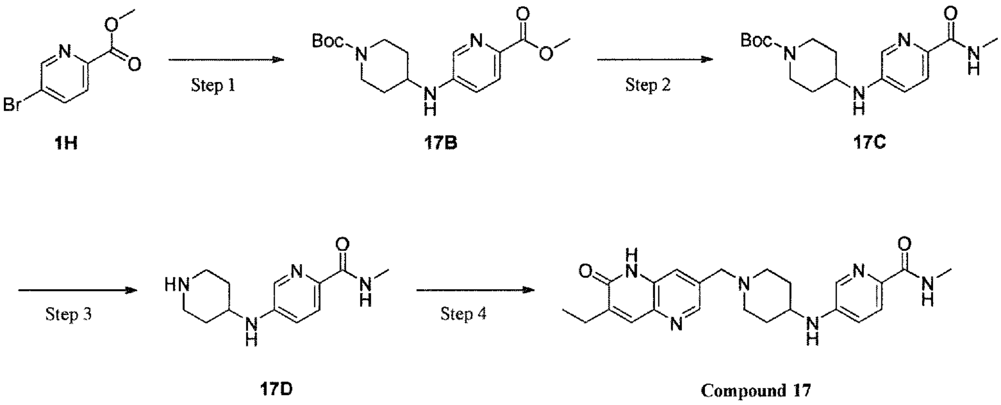


1H 17B 17C 17D Compound 17

Step 1:

Methyl 5-bromopyridine-2-carboxylate 1H (2.00 g, 9.26 mmol), 1-Boc-4-aminopiperidine (2.23 g, 11.11 mmol), caesium carbonate (6.03 g, 18.52 mmol) and 4,5-bisdiphenylphosphine-9,9-dimethylxanthene (0.54 g, 0.93 mmol) were successively added to dry toluene (150 mL) solution, and the mixture was subjected to nitrogen replacement three times. Tris(dibenzylideneacetone)dipalladium (0.42 g, 0.46 mmol) was added, and the mixture was subjected to nitrogen replacement again three times, and then warmed to 100°C and reacted for 18 hours. After the reaction was completed, the reaction liquid was filtered, and the filtrate was concentrated to dryness to afford a crude product. The crude product was purified by column chromatography (DCM : MeOH (v/v) = 100-10 : 1), to afford intermediate 17B (2.80 g, 90.15%).
LC-MS (ESI): m/z = 336.2[M+H]$^+$

Step 2:

Intermediate 17B (2.50 g, 7.45 mmol) was added to acetonitrile (50 mL) solution, followed by 40% methylamine aqueous solution (50 mL), and the mixture was stirred overnight while the temperature was controlled at 30°C. The reaction liquid was concentrated and purified by column chromatography (DCM : MeOH (v/v) = 100-10 : 1), to afford intermediate 17C (2.23 g, 89.51%).
LC-MS (ESI): m/z = 335.1[M+H]$^+$

Step 3:

Intermediate 17C (2.60 g, 7.77 mmol) was dissolved in dichloromethane solution (20 mL), and a solution of hydrogen chloride in ethyl acetate (2 mol/L, 20 mL) was slowly added dropwise while the temperature was controlled at 30°C. The mixture was stirred for 5 hours and then filtered, and the filter cake was washed with petroleum ether, to afford intermediate 17D (2.23 g, 83.51%).
LC-MS (ESI): m/z = 236.2[M+H]$^+$

Step 4:

Compound 17D (310 mg, 0.9 mmol) and 1G (200 mg, 0.9 mmol) were added to acetonitrile solution (20 mL), followed by potassium iodide (15 mg, 0.09 mmol) and N,N-diisopropylethylamine (1 mL), and the mixture was warmed to 80°C and reacted for 2 hours. The reaction liquid was concentrated to dryness, methanol was added for dissolution, and the mixture was separated and purified by reverse phase column (C18 spherical 20-35 nm 100A 120 g; water : acetonitrile (v/v) = 98 : 2-70 : 30), to afford compound 17 (154 mg, 36.68%).

LC-MS (ESI): m/z = 421.2 $[M+H]^+$
$^1H$ NMR (400 MHz, DMSO-$d_6$) δ 12.17 (s, 1H), 9.81 (s, 1H), 8.54 (d, 1H), 8.26 (dd, 1H), 8.05 - 7.69 (m, 4H), 7.06 (dd, 1H), 6.60 (s, 1H), 4.48 (s, 3H), 3.63 - 3.11 (m, 3H), 2.76 (d, 3H), 2.63 - 2.54 (m, 2H), 2.26 - 1.48 (m, 4H), 1.20 (t, 3H).

Examples 18 and 19

(S)-5-((1-((7-ethyl-6-oxo-5,6-dihydro-1,5-naphthyridin-3-yl)methyl) pyrrolidin-3-yl)amino)-N-methylpicolinamide and (R)-5-((1-((7-ethyl-6-oxo-5,6-dihydro-1,5-naphthyridin-3-yl)methyl)pyrrolidin-3-yl)amino)-N-methylpicolinamide (Compound 18 and Compound 19)

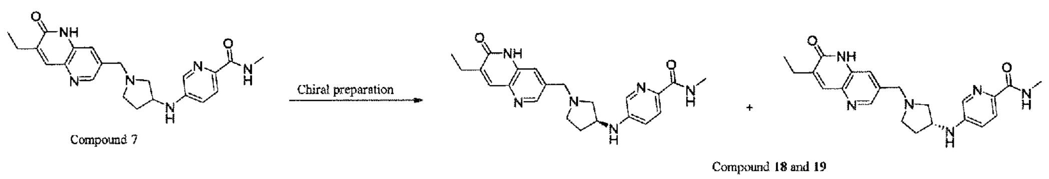


Chiral preparation:

Compound 7 (190 mg) was subjected to chiral resolution, to afford compound 18 (55 mg) and compound 19 (55 mg).

Preparation method:

Instrument: Waters 150 MGM; column: Chiralpak Column; mobile phase: A, $CO_2$ B, ethanol and acetonitrile (0.1% $NH_3$•$H_2O$); gradient: 60% B gradient elution; flow rate: 100 mL/min; column temperature: 35°C; wavelength: 220 nm; cycle time: 3.8 min; sample preparation: sample concentration: 2 mg/mL, acetonitrile solution; sample injection: 1.5 ml/injection. After separation, the fractions were dried on a rotary evaporator at the bath temperature of 40°C to afford P1 (retention time: 0.961 minutes, compound 18) and P2 (retention time: 1.734 minutes, compound 19).

Compound 18: LC-MS (ESI): m/z = 407.0 $[M+H]^+$
Compound 19: LC-MS (ESI): m/z = 407.1 $[M+H]^+$

Example 20

1'-((7-ethyl-6-oxo-5,6-dihydro-1,5-naphthyridin-3-yl)methyl)-N-(1-methyl-1H-pyrazol-4-yl)-1',2',3',6'-tetrahydro-[3,4'-bipyridine]-6-carboxamide (Compound 20)

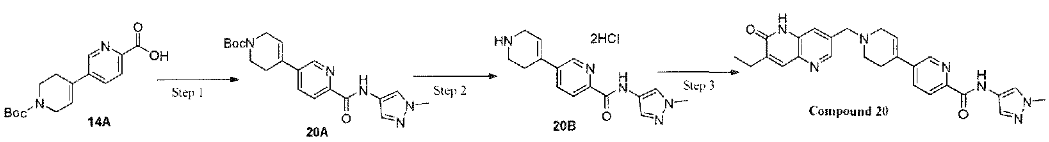

Step 1:

N,N,N',N'-Tetramethylchlorourea hexafluorophosphate (2.90 g, 10.35 mmol) was added to dichloromethane (50 mL) solution, N-methylimidazole (1.13 g, 13.80 mmol) was slowly added, and the mixture was stirred for 15 min. Subsequently, intermediate 14A (2.10 g, 6.90 mmol) and 1-methyl-1H-pyrazol-4-amine (0.80 g, 8.28 mmol) were successively added, and the mixture was kept at 25°C and reacted for 5 hours. After the reaction was completed, the reaction liquid was washed with water (50 mL×3), the organic phase was dried over anhydrous sodium sulphate and concentrated to dryness, and the residue was separated and purified by silica gel column (dichloromethane : methanol = 100%-85%), to afford intermediate 20A (2.10 g, yield: 79.94%).
LC-MS (ESI): m/z = 384.1 $[M+H]^+$

Step 2:

Intermediate 20A (1.80 g, 4.69 mmol) was added to a solution of hydrogen chloride in dioxane (4.0 mol/L, 30 mL), and the mixture was reacted at room temperature for 5 hours. The reaction liquid was concentrated to dryness, isopropanol (5 mL) solution and ethyl acetate (10 mL) solution were added, and the mixture was stirred for 1 hour and filtered, to afford intermediate 20B (1.20 g, yield: 71.83%).
LC-MS (ESI): m/z = 284.1 $[M+H]^+$

Step 3:

Compound 20B (530 mg, 1.49 mmol) and 1G (300 mg, 1.35 mmol) were added to a mixed solution of acetonitrile (50 mL) and N,N-dimethylformamide (10 mL), potassium iodide (220 mg, 1.35 mmol) and N,N-diisopropylethylamine (1.50 mL) were then added, and the mixture was warmed to 80°C and reacted for 2 hours. After the reaction was completed, the reaction liquid was concentrated to dryness, spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 5%-50%, elution time: 15 min, flow rate: 12 mL/min, and column temperature: 30°C), to afford title compound 20 (210 mg, yield: 33.13%, retention time: about 5.40 min).

LC-MS (ESI): m/z = 470.1 $[M+H]^+$
$^1$H NMR (400 MHz, DMSO-$d_6$) δ11.82 (s, 1H), 10.82 (s, 1H), 8.75 (t, 1H), 8.42 (d, 1H), 8.12 - 8.01 (m, 3H), 7.74 (d, 2H), 7.65 (d, 1H), 6.46 (d, 1H), 3.78 (d, 5H), 3.18 (d, 2H), 2.73 (t, 2H), 2.62 - 2.52 (m, 4H), 1.19 (t, 3H).

Example 21

5-(4-(((7-ethyl-6-oxo-5,6-dihydro-1,5-naphthyridin-3-yl)methyl)amino) piperidin-1-yl)-N-methylpicolinamide (Compound 21)

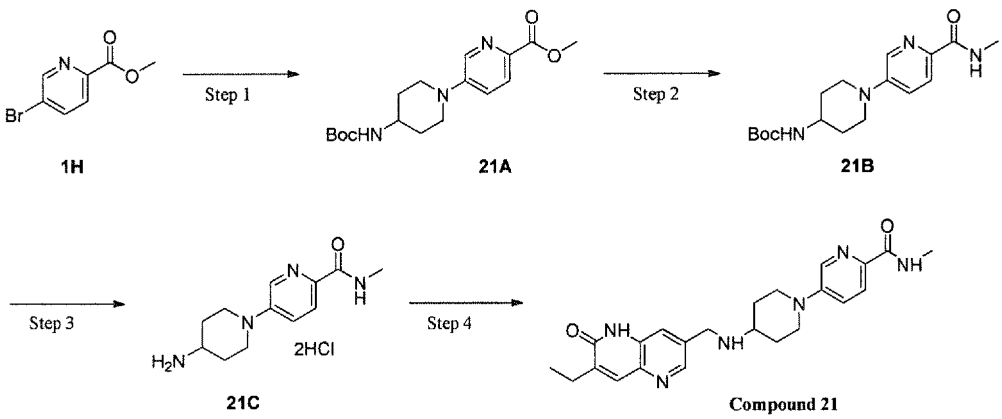


1H 21A 21B

21C Compound 21

Step 1:

Methyl 5-bromopyridine-2-carboxylate 1H (2.00 g, 9.29 mmol), 4-tert-butoxycarbonyl aminopiperidine (2.23 g, 11.15 mmol) and caesium carbonate (6.05 g, 18.58 mmol) were successively added to 1,4-dioxane (250 mL) solution, and the mixture was subjected to nitrogen replacement three times. 4,5-Bisdiphenylphosphine-9,9-dimethylxanthene (0.54 g, 0.93 mmol) and tris(dibenzylideneacetone)dipalladium (0.43 g, 0.46 mmol) were added, and the mixture was subjected to nitrogen replacement again three times, and then warmed to 100°C and reacted for 4 hours. After the reaction was completed, the reaction liquid was filtered, the filtrate was concentrated to dryness, and the residue was purified by silica gel column (dichloromethane : methanol = 100%-90%), to afford compound 21A (2.90 g, yield: 93.07%).
LC-MS (ESI): m/z = 336.2 $[M+H]^+$

Step 2:

Intermediate 21A (2.90 g, 8.65 mmol) was added to acetonitrile (40 mL) solution, followed by 40% methylamine aqueous solution (20 mL), and the mixture was reacted at 30°C for 5 hours. After the reaction was completed, the reaction liquid was concentrated to dryness, to afford crude product 21B (2.30 g, yield: 79.51%).
LC-MS (ESI): m/z = 335.2 $[M+H]^+$

Step 3:

Intermediate 21B (2.30 g, 6.88 mmol) was added to a solution of hydrogen chloride in dioxane (4.0 mol/L, 30 mL), and the mixture was reacted at room temperature for 5 hours. The reaction liquid was concentrated to dryness, isopropanol (5 mL) solution and ethyl acetate (10 mL) solution were added, and the mixture was stirred for 1 hour and filtered, to afford intermediate 21C (1.74 g, yield: 82.33%).
LC-MS (ESI): m/z = 235.2 $[M+H]^+$

Step 4:

Compound 21C (170 mg, 0.55 mmol) and 1G (100 mg, 0.45 mmol) were added to a mixed solution of acetonitrile (10 mL) and N,N-dimethylformamide (1 mL), potassium iodide (75 mg, 0.45 mmol) and N,N-diisopropylethylamine (0.50 mL) were then added, and the mixture was warmed to 80°C and reacted for 2 hours. After the reaction was completed, the reaction liquid was concentrated to dryness, spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 5%-50%, elution time: 15 min, flow rate: 12 mL/min, and column temperature: 30°C), to afford title compound 21 (30 mg, yield: 15.85%, retention time: about 5.5 min).

LC-MS (ESI): m/z = 421.2 $[M+H]^+$
$^1H$ NMR (400 MHz, DMSO-$d_6$) $\delta$ 11.80 (s, 1H), 8.42 (s, 1H), 8.35 (d, 1H), 8.25 (d, 1H), 7.80 (d, 1H), 7.73 (s, 1H), 7.65 (s, 1H), 7.38 (dd, 1H), 3.84 (d, 4H), 2.92 (t, 2H), 2.78 (d, 3H), 2.69 -2.61 (m, 2H), 2.55 (d, 2H), 1.92 (d, 2H), 1.37 (dd, 2H), 1.18 (t, 3H).

Example 22

N-(1'-((7-ethyl-6-oxo-5,6-dihydro-1,5-naphthyridin-3-yl)methyl)-1',2',3',6'-tetrahydro-[3,4'-bipyridin]-6-yl)cyclopropane-carboxamide (Compound 22)

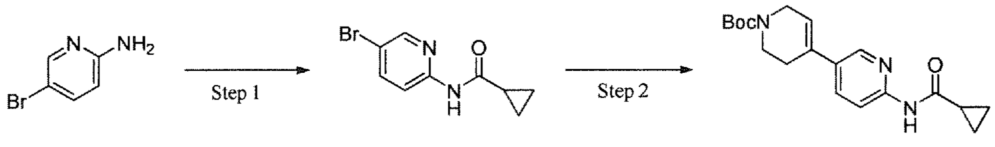


22A 22B 22C

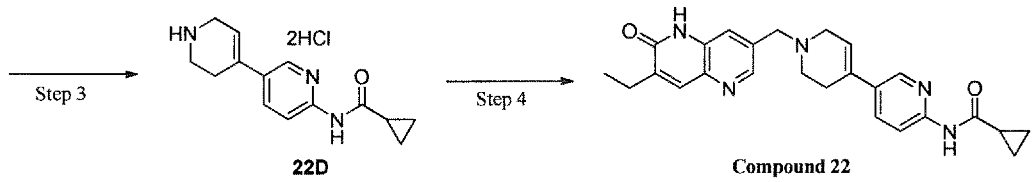


22D Compound 22

Step 1:

N,N,N',N'-Tetramethylchlorourea hexafluorophosphate (3.89 g, 13.87 mmol) was added to dichloromethane (100 mL) solution, N-methylimidazole (1.90 g, 23.12 mmol) was slowly added, and the mixture was stirred for 15 min. Subsequently, intermediate 22A (2.00 g, 11.56 mmol) and cyclopropylcarboxylic acid (0.99 g, 11.50 mmol) were successively added, and the mixture was kept at 25°C and reacted for 5 hours. After the reaction was completed, the reaction liquid was washed with water (50 mL×3), the organic phase was dried over anhydrous sodium sulphate and concentrated to dryness, and the residue was separated and purified by silica gel column (dichloromethane : methanol = 100%-85%), to afford intermediate 22B (2.70 g, yield: 96.88%).
LC-MS (ESI): m/z = 243.0 $[M+H]^+$

Step 2:

Intermediate 22B (3.00 g, 12.44 mmol), N-Boc-1,2,5,6-tetrahydropyridine-4-boronic acid pinacol ester (5.77 g, 18.66 mmol) and potassium carbonate (3.44 g, 24.88 mmol) were successively added to N,N-dimethylformamide (150 mL) solution, and the mixture was subjected to nitrogen replacement three times. Chloro(2-dicyclohexylphosphino-2',6'-di-isopropoxy-1,1'-biphenyl)(2-amino-1,1'-biphenyl-2-yl)palladium (II) (0.97 g, 1.24 mmol) was added, and the mixture was subjected to nitrogen replacement again three times, and then warmed to 110°C and reacted for 4 hours. After the reaction was completed, the reaction liquid was cooled to room temperature, filtered and concentrated to dryness, and the resulting residue was purified by silica gel column (dichloromethane : methanol = 100%-90%), to afford compound 22C (4.10 g, yield: 95.97%).
LC-MS (ESI): m/z = 344.2 $[M+H]^+$

Step 3:

Intermediate 22C (3.50 g, 10.19 mmol) was added to a solution of hydrogen chloride in dioxane (4.0 mol/L, 30 mL), and the mixture was reacted at room temperature for 5 hours. The reaction liquid was concentrated to dryness, isopropanol (5 mL) solution and ethyl acetate (20 mL) solution were added, and the mixture was stirred for 1 hour and filtered, to afford intermediate 22D (2.20 g, yield: 88.74%).
LC-MS (ESI): m/z = 244.2 $[M+H]^+$

Step 4:

Compound 22D (83 mg, 0.26 mmol) and 1G (50 mg, 0.22 mmol) were added to a mixed solution of acetonitrile (15 mL) and N,N-dimethylformamide (5 mL), potassium iodide (37 mg, 0.22 mmol) and N,N-diisopropylethylamine (0.50 mL) were then added, and the mixture was warmed to 80°C and reacted for 2 hours. After the reaction was completed, the reaction liquid was concentrated to dryness, spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 5%-50%, elution time: 15 min, flow rate: 12 mL/min, and column temperature: 30°C), to afford title compound 22 (10 mg, yield: 10.58%, retention time: about 4.70 min).

LC-MS (ESI): m/z = 430.2 $[M+H]^+$
$^1H$ NMR (400 MHz, DMSO-$d_6$) δ 11.80 (s, 1H), 10.76 (s, 1H), 8.39 (dd, 2H), 8.02 (d, 1H), 7.88 - 7.69 (m, 2H), 7.64

(d, 1H), 6.28 - 6.13 (m, 1H), 3.71 (s, 2H), 3.11 (dd, 2H), 2.68 (t, 2H), 2.55 (td, 4H), 2.10 - 1.95 (m, 1H), 1.19 (t, 3H), 0.87 - 0.71 (m, 4H).

Example 23

5-(3-(((7-ethyl-6-oxo-5,6-dihydro-1,5-naphthyridin-3-yl)methyl)amino) azetidin-1-yl)-N-methylpicolinamide (Compound 23)

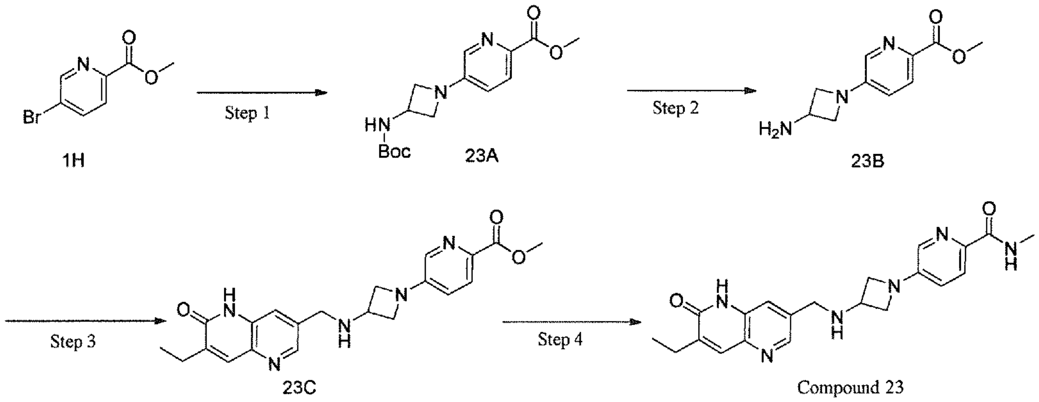


Step 1:

Compound 1H (1.0 g, 4.63 mmol), tert-butyl azetidin-3-yl carbamate (0.8 g, 4.63 mmol) and potassium carbonate (1.9 g, 13.89 mmol) were dissolved in N,N-dimethylformamide (20 mL), chloro(2-dicyclohexylphosphino-2',4',6'-triisopropyl-1,1'-biphenyl)[2-(2'-amino-1,1'-biphenyl)]palladium (II) (0.36 g, 0.46 mmol) was added, and under nitrogen protection, the mixture was heated to 120°C and reacted for 5 hours. The reaction liquid was concentrated and separated by column chromatography (dichloromethane : methanol = 20 : 1), to afford target compound 23A (1.1 g, 77.30%).
LC-MS (ESI): m/z = 308.3 $[M+H]^+$

Step 2:

Compound 23A (0.5 g, 1.63 mmol) was dissolved in a solution of hydrogen chloride in 1,4-dioxane (4M, 10 mL), and the mixture was reacted at room temperature for 4 hours, at which time a large amount of solid was observed to be precipitated out. The reaction liquid was filtered, washed twice with ethyl acetate and dried, to afford compound 23B (0.3 g, 88.81%).
LC-MS (ESI): m/z = 208.3 $[M+H]^+$

Step 3:

Compound 23B (0.11 g, 0.49 mmol), 1G (0.1 g, 0.49 mmol) and potassium iodide (0.12 g, 0.73 mmol) were dissolved in N,N-dimethylformamide (2 mL), N,N-diisopropylethylamine (1.22 mL) was added, and the mixture was heated to 80°C and reacted for 3 hours. The reaction liquid was concentrated and separated by column chromatography (dichloromethane : methanol = 20 : 1), to afford compound 23C (0.05 g, 25.94%).
LC-MS (ESI): m/z = 394.2 $[M+H]^+$

Step 4:

Compound 23C (0.1 g, 0.25 mmol) was dissolved in methanol (3 mL), methylamine aqueous solution (1 mL) was added, and the mixture was reacted at room temperature for 5 hours. The reaction liquid was concentrated and purified by C-18 reverse phase column chromatography (acetonitrile : water = 30 : 70), to afford compound 23 (50 mg, 4.25%).

LC-MS (ESI): m/z = 393.3 $[M+H]^+$
$^1$H NMR (400 MHz, $CD_3OD$) δ 8.50 (d, 1H), 7.88 - 7.81 (m, 2H), 7.79 - 7.72 (m, 2H), 6.86 (dd, 1H), 4.23 (t, 2H),

3.97 - 3.89 (m, 3H), 3.76 (dd, 2H), 2.92 (s, 3H), 2.66 (q, 2H), 1.28 (t, 3H).

Example 24

5-((1-((7-ethyl-6-oxo-5,6-dihydro-1,5-naphthyridin-3-yl)methyl)pyrrolidin-3-yl)amino)-N-methylpicolinamide (Compound 24)

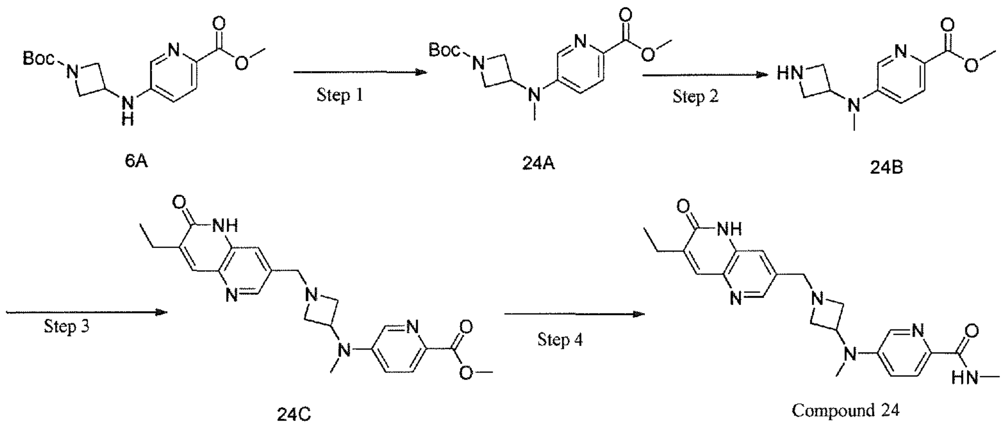


6A 24A 24B 24C Compound 24

Step 1:

Compound 6A (0.01 g, 0.033 mmol), potassium iodide (5 mg, 0.033 mmol) and potassium carbonate (0.014 g, 0.099 mmol) were dissolved in N,N-dimethylformamide (3 mL), the mixture was reacted at room temperature for 16 hours, and the reaction liquid was concentrated and separated by column chromatography (dichloromethane : methanol = 20 : 1), to afford target compound 24A (0.01 g, 94.29%).
LC-MS (ESI): m/z = 322.3$[M+H]^+$

Step 2:

Compound 24A (0.52 g, 1.63 mmol) was dissolved in a solution of hydrogen chloride in 1,4-dioxane (4M, 10 mL), and the mixture was reacted at room temperature for 4 hours, at which time a large amount of solid was observed to be precipitated out. The reaction liquid was filtered, washed twice with ethyl acetate and dried, to afford compound 24B (0.3 g, 83.18%).

Step 3:

Compound 24B (0.11 g, 0.49 mmol), 1G (0.11 g, 0.49 mmol) and potassium iodide (0.12 g, 0.73 mmol) were dissolved in N,N-dimethylformamide (2 mL), N,N-diisopropylethylamine (1.22 mL) was added, and the mixture was heated to 80°C and reacted for 3 hours. The reaction liquid was concentrated and separated by column chromatography (dichloromethane : methanol = 20 : 1), to afford compound 24C (0.05 g, 25.04%).
LC-MS (ESI): m/z = 408.5 $[M+H]^+$

Step 4:

Compound 24C (0.05 g, 0.12 mmol) was dissolved in methanol (5 mL), methylamine aqueous solution (2 mL) was added, and the mixture was reacted at room temperature for 5 hours. The reaction liquid was concentrated and purified by C-18 reverse phase column chromatography (acetonitrile : water = 30 : 70), to afford compound 24 (20 mg, 19.90%).

LC-MS (ESI): m/z = 407.5 $[M+H]^+$
$^1H$ NMR (400 MHz, $CD_3OD$) δ 8.47 (d, 1H), 8.15 (s, 1H), 8.08 (d, 1H), 7.89 (d, 1H), 7.82 (s, 1H), 7.71 (s, 1H), 7.16 (dd, 1H), 4.43 (t, 1H), 4.02 - 3.83 (m, 4H), 3.43 (dd, 2H), 3.04 (s, 3H), 2.92 (s, 3H), 2.73 - 2.60 (m, 2H), 1.28 (t, 3H).

Example 25:

N-cyclopropyl-1'-((7-ethyl-6-oxo-5,6-dihydro-1,5-naphthyridin-3-yl)methyl)-1',2',3',6'-tetrahydro-[3,4'-bipyridine]-6-carboxamide (Compound 25)

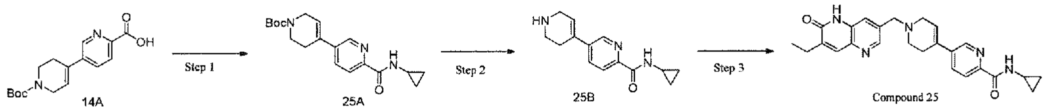


Step 1:

14A (0.5 g, 1.64 mmol) was dissolved in N,N-dimethylformamide (10 mL), diisopropylethylamine (1.27 g, 9.84 mmol) and 2-(7-aza-1H-benzotriazol-1-yl)-1,1,3,3-tetramethyluronium hexafluorophosphate (0.75 g, 1.97 mmol) were then added, and the mixture was stirred at room temperature for half an hour. Cyclopropylamine (0.14 g, 2.46 mmol) was added, and the mixture was stirred for another 1 hour. Water was added for dilution, and the mixture was extracted with ethyl acetate (10 ml × 3), washed with saturated brine (50 mL × 3). dried over anhydrous sodium sulphate, concentrated, and subjected to column chromatography, to afford product 25A (0.3 g, 53%).
LCMS m/z=344.3 $[M+1]^+$

Step 2:

25A (0.30 g, 0.87 mmol) was dissolved in dichloromethane (8 mL), trifluoroacetic acid (3.06 g, 26.84 mmol) was then added, and the mixture was stirred at room temperature for 3 hours. After the reaction was completed, the reaction liquid was concentrated under reduced pressure, to afford crude product 25B (0.22 g).
LCMS m/z=244.1 $[M+1]^+$

Step 3:

25B (0.1 g, 0.41 mmol) was dissolved in acetonitrile (6mL) solution, 1G (0.091 g, 0.41 mmol) and potassium iodide (0.014 g, 0.082 mmol) were added, and the mixture was stirred at 80°C for 6 hours. The reaction liquid was spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 10%-70%, elution time: 15 min, retention time: 10 min, flow rate: 12 mL/min, and column temperature: 30°C), to afford title compound 25 (62 mg, yield: 35%).

LCMS m/z =430.1 $[M+1]^+$
$^1$H NMR (400 MHz, DMSO-$d_6$) δ 11.81 (s, 1H), 8.82 - 8.58 (m, 2H), 8.41 (d, 1H), 8.01 - 7.87 (m, 2H), 7.75 (s, 1H), 7.64 (d, 1H), 6.51 - 6.32 (m, 1H), 3.72 (s, 2H), 3.16 (q, 2H), 2.94 - 2.84 (m, 1H), 2.71 (t, 2H), 2.60 - 2.51 (m, 4H), 1.18 (t, 3H), 0.73 - 0.63 (m, 4H).

Example 26:

1'-((7-ethyl-6-oxo-5,6-dihydro-1,5-naphthyridin-3-yl)methyl)-N-((1R,2S)-2-fluorocyclopropyl)-1',2',3',6'-tetrahydro-[3,4'-bipyridine]-6-carboxamide (Compound 26)

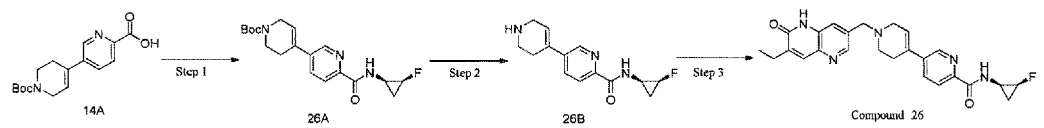


Step 1:

14A (0.5 g, 1.64 mmol) was dissolved in dichloromethane (30 mL), (1R,2S)-2-fluorocyclopropan-1-amine, 4-methylbenzene-1-sulfonic acid (0.81 g, 3.28 mmol), 1-methyl-1H-imidazole (0.27 g, 3.28 mmol) and [chloro(dimethylamino)methylene]-dimethylazane hexafluorophosphate (0.55 g, 1.97 mmol) were added, and the mixture was stirred at room temperature for 2 hours, concentrated under reduced pressure and purified by column chromatography, to afford product 26A (0.23 g, 39%).
LCMS m/z =362.1[M+1]$^+$

Step 2:

Compound 26A (0.23 g, 0.64 mmol) was dissolved in dichloromethane (8 mL), trifluoroacetic acid (3.06 g, 26.84 mmol) was added, and the mixture was stirred at room temperature for 3 hours and concentrated under reduced pressure, to afford crude product 26B (0.2 g).
LCMS m/z =262.2 [M+1]$^+$

Step 3:

Compound 26B (0.17 g, 0.65 mmol), 1G (0.17 g, 0.78 mmol), diisopropylethylamine (0.34 g, 2.6 mmol) and potassium iodide (0.011 mg, 0.065 mmol) were dissolved in acetonitrile (10 mL), and the mixture was stirred at 80°C for 6 hours. The reaction liquid was spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 10%-70%, elution time: 15 min, retention time: 10.5 min, flow rate: 12 mL/min, and column temperature: 30°C), to afford title compound 26 (49 mg, yield: 17%).

LCMS M/Z (ESI): m/z=448.3 [M+1]$^+$
$^1$H NMR (400 MHz, DMSO-$d_6$) δ 11.79 (s, 1H), 8.73 - 8.69 (m, 1H), 8.65 (d, 1H), 8.41 (d, 1H), 8.04 - 7.96 (m, 2H), 7.75 (s, 1H), 7.64 (d, 1H), 6.42 (d, 1H), 4.96 -4.59 (m, 1H), 3.72 (s, 2H), 3.35 - 3.20 (m, 2H), 3.16 (q, 2H), 2.71 (t, 2H), 2.56 (t, 2H), 2.53 (d, 1H), 1.44 - 1.30 (m, 1H), 1.28 - 1.22 (m, 1H), 1.18 (t, 3H).

Example 27

3-ethyl-7-((4-(2-methylimidazo[1,2-a]pyrazin-6-yl)-3,6-dihydropyridin-1(2H)-yl)methyl)-1,5-naphthyridin-2(1H)-one (Compound 27)

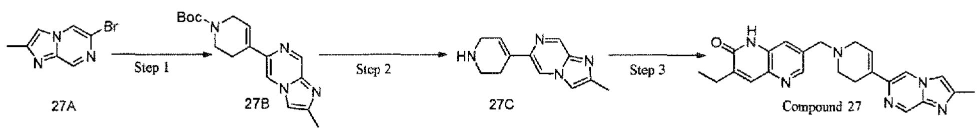


Step 1:

27A (0.5 g, 2.36 mmol), N-Boc-1,2,5,6-tetrahydropyridine-4-boronic acid pinacol ester (0.88 g, 2.83 mmol) and potassium carbonate (0.65 g, 4.72 mmol) were successively added to a solution of 1,4-dioxane (10 mL) and water (2 mL), and the mixture was subjected to nitrogen replacement three times. Chloro(2-dicyclohexylphosphino-2',6'-di-isopropoxy-1,1'-biphenyl)(2-amino-1,1'-biphenyl-2-yl)palladium (II) (0.18 g, 0.23 mmol) was added, and the mixture was subjected to nitrogen replacement again three times, and then warmed to 100°C and reacted for 4 hours. After the reaction was completed, the reaction liquid was cooled to room temperature, concentrated, and separated by column chromatography (dichloromethane : methanol = 50 : 1), to afford compound 27B (0.6 g, 80.87%).
LC-MS (ESI): m/z = 315.3 [M+H]$^+$

Step 2:

Compound 27B (0.5 g, 0.63 mol) was dissolved in methanol (5 mL) solution, a solution of hydrogen chloride in methanol (4 mol/L, 5 mL) was slowly added dropwise, and the mixture was stirred overnight at 30°C. After the reaction was completed, the reaction liquid was concentrated to dryness, ethyl acetate (10 mL) was added, followed by triethylamine (1 mL), and the mixture was stirred for another 1 hour, concentrated and separated by column chromatography (dichloromethane : methanol = 10 : 1), to afford compound 27C (0.25 g, 73.38%).

Step 3:

Compound 27C (0.2 g, 0.93 mmol), intermediate 1G (0.21 g, 0.93 mmol), potassium iodide (0.15 g, 0.93 mmol) and N,N-diisopropylethylamine (0.62 mL) were added to N,N-dimethylformamide (10 mL) mixed solution, and the mixture was warmed to 80°C and reacted for 2 hours. After the reaction was completed, the reaction liquid was concentrated and purified by C-18 reverse phase column chromatography (acetonitrile : water = 30 : 70), to afford compound 27 (50 mg, 13.42%).

LC-MS (ESI): m/z = 401.2 $[M+H]^+$
$^1$H NMR (400 MHz, DMSO-*d6*) δ 11.83 (s, 1H), 8.86 (s, 1H), 8.50 (s, 1H), 8.42 (s, 1H), 7.82 (s, 1H), 7.76 (s, 1H), 7.66 (s, 1H), 6.75 (s, 1H), 3.74 (s, 2H), 3.18 (s, 2H), 2.73 (s, 2H), 2.59 - 2.52 (m, 4H), 2.39 (s, 3H), 1.19 (t, 3H).

Example 28

5-(4-((7-ethyl-6-oxo-5,6-dihydro-1,5-naphthyridin-3-yl)methylene)piperidin-1-yl)-N-methylpicolinamide (Compound 28)

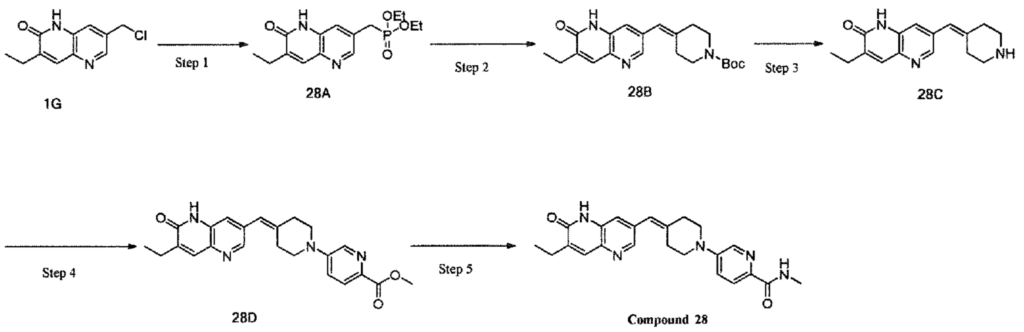


Step 1:

Intermediate 1G (5 g, 22.45 mmol) was added to triethyl phosphite (50 mL), and the mixture was stirred and warmed to 150°C in an oil bath to gradually dissolve the raw materials. After the reaction was completed as monitored by LCMS and TLC, the reaction liquid was cooled and concentrated under reduced pressure, to afford crude (8 g), which was directly used in the next reaction.
LC-MS (ESI): m/z= 325.1 $[M+H]^+$.

Step 2:

NaH (2.96 g, 74.07 mmol) was added to DMF (10 mL), and the mixture was stirred. A solution of 28A (8 g, 24.69 mmol, containing triethyl phosphite) in DMF (50 mL) was added dropwise at 0°C, and the mixture was continuously stirred for 30 minutes. Subsequently, tert-butyl 4-oxopiperidine-1-carboxylate (9.8 g, 49.38 mmol) was dissolved in DMF (20 mL) and added dropwise to the reaction, and the mixture was quenched with water (300 mL) after 4 hours and extracted with ethyl acetate (100 mL × 3). The organic phase was dried over anhydrous sodium sulphate, filtered, concentrated, and separated by silica gel chromatographic column (EA : PE = 0-100%), to afford title compound 28B (4.4 g, yield over two steps: 53.1%).
LC-MS (ESI): m/z= 370.2 $[M+H]^+$.

Step 3:

28B (1.2 g, 3.25 mmol) was dissolved in dichloromethane (12 mL), trifluoroacetic acid (4 mL) was added dropwise, and the mixture was stirred for 2 hours, concentrated, and separated by C18 reverse phase column (5‰ $NH_3$•$H_2O$/$H_2O$:MeOH), to afford title compound 28C (600 mg, 68.6%).
LC-MS (ESI): m/z= 270.1 $[M+H]^+$.

Step 4:

28C (600 mg, 2.22 mmol) was dissolved in dimethyl sulfoxide (6 mL), DIPEA (1.4 g, 11.1 mmol) and methyl 5-fluoropicolinate (689 mg, 4.44 mmol) were added, and the mixture was reacted at 120°C for 4 hours. Subsequently, water (60 mL) was added for dilution, and the mixture was extracted with ethyl acetate (20 mL × 3). The organic phase was dried over anhydrous sodium sulphate, filtered, concentrated, and separated by silica gel chromatographic column (DCM : MeOH = 1 : 10), to afford title compound 28D (800 mg, 89.1%).
LC-MS (ESI): m/z= 405.3 $[M+H]^+$.

Step 5:

28D (200 mg, 0.50 mmol) was added to methanol and methylamine aqueous solution (1 : 1, 10 mL), the mixture was reacted and stirred for 4 hours and concentrated, and then 100 mg of the concentrated liquid was purified by preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5%-50%; c. flow rate: 12 mL/min; d. elution time: 20 min, and the preparative liquid was concentrated and lyophilized, to afford compound 28 (40 mg, 40.1%).
$^1$H NMR (400 MHz, DMSO-$d_6$) δ 11.78 (s, 1H), 8.39 - 8.32 (m, 2H), 8.30 (d, 1H), 7.84 (d, 1H), 7.73 (s, 1H), 7.54 (d, 1H), 7.42 (dd, 1H), 6.48 (s, 1H), 3.52 (dt, 4H), 2.79 (d, 3H), 2.60 (t, 2H), 2.58 - 2.51 (m, 4H, overlapped with solvent DMSO peak), 1.18 (t, 3H).
LC-MS (ESI): m/z= 404.2 $[M+H]^+$.

Example 29

5-(4-((7-ethyl-6-oxo-5,6-dihydro-1.5-naphthyridin-3-yl)methyl)piperidin-1-yl)-N-methylpicolinamide (Compound 29)

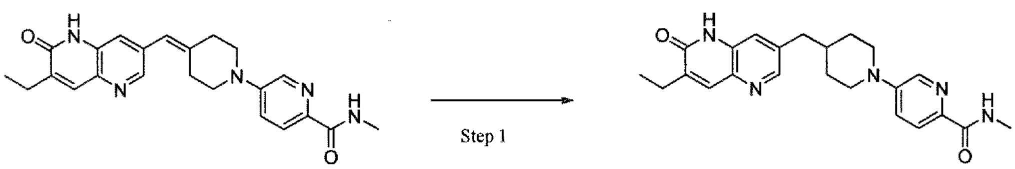


Compound 28 Compound 29

Step 1:

Compound 28 (100 mg, 0.25 mmol) was dissolved in methanol (10 mL), palladium on carbon (30 mg, 10%) was added, and the mixture was reacted and stirred in hydrogen environment for 12 hours, filtered, concentrated, and purified by preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5%-50%; c. flow rate: 12 mL/min; d. elution time: 20 min, and the preparative liquid was concentrated and lyophilized, to afford compound 29 (45 mg, 44.8%).
$^1$H NMR (400 MHz, DMSO-$d_6$) δ 11.80 (s, 1H), 8.37 - 8.29 (m, 2H), 8.24 (d, 1H), 7.80 (d, 1H), 7.73 (s, 1H), 7.44 (d, 1H), 7.36 (dd, 1H), 3.90 (d, 2H), 2.84 - 2.73 (m, 5H), 2.66 (d, 2H), 2.57 - 2.51 (m, 2H), 1.87 - 1.73 (m, 1H), 1.72 - 1.62 (m, 2H), 1.37 - 1.23 (m, 2H), 1.18 (t, 3H).
LC-MS (ESI): m/z= 406.2 $[M+H]^+$.

Example 30

N-(1'-((7-ethyl-6-oxo-5,6-dihydro-1,5-naphthyridin-3-yl)methyl)-1',2',3',6'-tetrahydro-[3,4'-bipyridin]-6-yl)-1-metllyl-1H-pyrazole-4-carboxamide (Compound 30)

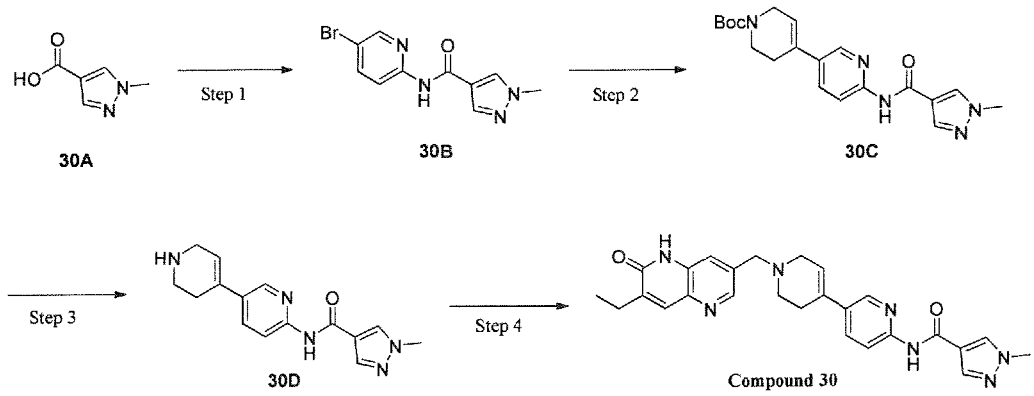


Step 1:

1-Methylpyrazole-4-carboxylic acid 30A (2.00 g, 15.83 mmol) was placed in dichloromethane (30 mL) solution, N,N-dimetliylfonnamide (0.5 mL) was added, and the mixture was purged with nitrogen and then cooled to 0-10°C. Oxalyl chloride (2.21g, 17.41 mmol) was slowly added dropwise, and the mixture was reacted at room temperature for 3 hours after the dropwise addition was completed. The reaction liquid was concentrated to dryness, dichloromethane (30 mL) was added, and the mixture was concentrated to dryness again, diluted with dichloromethane (10 mL), and added dropwise to a solution of 5-bromo-2-aminopyridine (2.74 g, 15.83 mmol) in dichloromethane (30 mL) while the temperature was controlled at 0-10°C. After the reaction was completed following 30 minutes, the reaction liquid was washed with water (50 mL×3), the organic phase was concentrated, and the residue was purified by silica gel column (methanol : dichloromethane = 0-15%), to afford compound 30B (3.4 g, yield: 76.40%).
LC-MS (ESI): m/z = 282.1 $[M+H]^+$

Step 2:

Intermediate 30B (2.60 g, 9.25 mmol), N-Boc-1,2,5,6-tetrahydropyridine-4-boronic acid pinacol ester (3.43 g, 11.10 mmol) and potassium carbonate (2.56 g, 18.5 mmol) were successively added to N,N-dimethylformamide (50 mL) solution, and the mixture was subjected to nitrogen replacement three times. Chloro(2-dicyclohexylphosphino-2',6'-diisopropoxy-1,1'-biphenyl)(2-amino-1,1'-biphenyl-2-yl)palladium (II) (0.72 g, 0.93 mmol) and water (0.42 g, 23.13 mmol) were added, and the mixture was subjected to nitrogen replacement again three times, and then warmed to 110°C and reacted for 4 hours. After the reaction was completed, the reaction liquid was cooled to room temperature, filtered and concentrated to dryness, and the resulting residue was purified by silica gel column (dichloromethane : methanol = 100%-90%), to afford compound 30C (3.40 g, yield: 95.86%).
LC-MS (ESI): m/z = 384.1 $[M+H]^+$

Step 3:

Intermediate 30C (2.60 g, 6.78 mmol) was added to methanol (30 mL) solution, a solution of hydrogen chloride in dioxane (4.0 mol/L, 30 mL) was slowly added dropwise, and the mixture was reacted at room temperature for 5 hours. The reaction liquid was concentrated to dryness, isopropanol (5 mL) solution and ethyl acetate (20 mL) solution were added, and the mixture was stirred for 1 hour and filtered, to afford intermediate 30D (2.37 g, yield: 98.13%).
LC-MS (ESI): m/z = 284.1 $[M+H]^+$

Step 4:

Intermediate 30D (760 mg, 2.12 mmol), 1G (500 mg, 1.93 mmol), potassium iodide (0.32 g, 1.93 mmol) and N,N-diisopropylethylamine (1.60 mL) were added to a mixed solution of acetonitrile (20 mL) and N,N-dimethylformamide

(4 mL), and the mixture was warmed to 80°C and reacted for 2 hours. After the reaction was completed, the reaction liquid was concentrated to 10 mL, water (30 mL) was added, the mixture was stirred for 1 hour and filtered, and the filter cake was washed with water (10mL), to afford a crude compound. The crude was refluxed with acetonitrile (30mL) for 2 hours, cooled to room temperature and filtered, to afford compound 30 (320 mg, 3531%).

LC-MS (ESI): m/z = 470.1 $[M+H]^+$
$^1H$ NMR (400 MHz, DMSO-$d_6$) δ 11.81 (s, 1H), 10.47 (s, 1H), 8.42 (q, 3H), 8.16 - 8.11 (m, 2H), 7.87 - 7.75 (m, 2H), 7.67 - 7.63 (m, 1H), 6.23 (s, 1H), 3.88 (s, 3H), 3.72 (s, 2H), 3.13 (d, 2H), 2.70 (t, 2H), 2.59 - 2.51 (m, 4H), 1.19 (t, 3H).

Example 31

N-cyclopropyl-5-(4-((7-ethyl-6-oxo-5,6-dihydro-1,5-naphthyridin-3-yl)methylene)piperidin-1-yl)picolinamide (Compound 31)

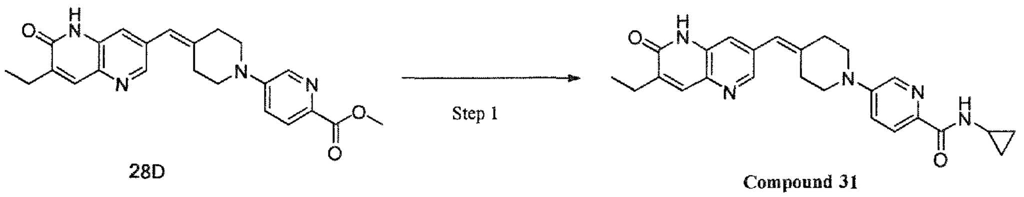


28D

Compound 31

Step 1:

28D (100 mg, 0.26 mmol) was added to cyclopropylamine (1 mL), and the mixture was reacted at 58°C in a sealed tube for 24 hours. After the reaction was completed, the reaction liquid was concentrated and purified by preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 12 mL/min; d. elution time: 20 min, and $NaHCO_3$ was added to the preparative liquid to adjust to pH 7-8, and then the mixture was extracted with DCM and concentrated, to afford compound 31 (50 mg, 47.1%).

$^1H$ NMR (400 MHz, DMSO-$d_6$) δ 11.78 (s, 1H), 8.35 (d, 1H), 8.31 (d, 1H), 8.27 (d, 1H), 7.84 (d, 1H), 7.73 (s, 1H), 7.56 - 7.50 (m, 1H), 7.43 (dd, 1H), 6.48 (s, 1H), 3.51 (dt, 4H), 2.90 - 2.79 (m, 1H), 2.60 (t, 2H), 2.57 - 2.51 (m, 4H), 1.18 (t, 3H), 0.71 - 0.58 (m, 4H).

LC-MS (ESI): m/z= 430.2 $[M+H]^+$.

Example 32

5-(4-((7-ethyl-6-oxo-5,6-dihydro-1,5-naphthyridin-3-yl)methylene)piperidin-1-yl)-N-(1-methyl-1H-pyrazol-4-yl)picolinamide (Compound 32)

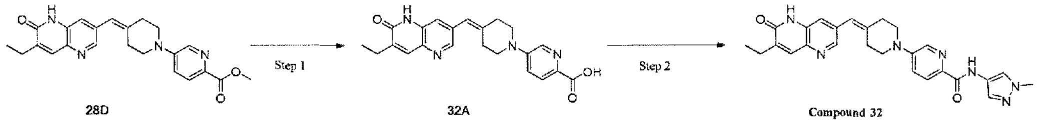


28D

32A

Compound 32

Step 1:

28D (500 mg, 1.24 mmol) was dissolved in methanol (2.5 mL), an aqueous solution (2.5 mL) of lithium hydroxide monohydrate (260 mg, 6.18 mmol) was added dropwise, and the mixture was reacted at room temperature for 1 h. After the reaction was completed as monitored by LCMS, the reaction liquid was subjected to rotary evaporation to remove the organic phase, 1N hydrochloric acid was used to adjust to pH 6-7, and the precipitated product was filtered off, to afford title compound 32A (450 mg, 93.2%).

LC-MS (ESI): m/z= 391.1 $[M+H]^+$.

Step 2:

32A (100 mg, 0.26 mmol), 1-methyl-1H-pyrazol-4-amine hydrochloride (68 mg, 0.51 mmol), HATU (195 mg, 0.51 mmol) and DIPEA (165 mg, 1.28 mmol) were added to DMF (5 mL), and the mixture was stirred overnight at room temperature and purified by preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% TFA); b. gradient elution, mobile phase A: 5%-50%; c. flow rate: 12 mL/min; d. elution time: 20 min, and $NaHCO_3$ was added to the preparative liquid to adjust to pH 7-8, and then the mixture was extracted with dichloromethane and concentrated, to afford compound 32 (30 mg, 24.9%).

$^1H$ NMR (400 MHz, DMSO-$d_6$) δ 11.79 (s, 1H), 10.47 (s, 1H), 8.37 - 8.33 (m, 2H), 8.02 (s, 1H), 7.92 (d, 1H), 7.73 (s, 1H), 7.70 - 7.68 (m, 1H), 7.54 (d, 1H), 7.48 (dd, 1H), 6.50 (s, 1H), 3.81 (s, 3H), 3.56 (dt, 4H), 2.62 (t, 2H), 2.58 - 2.52 (m, 4H), 1.19 (t, 3H).

LC-MS (ESI): m/z= 470.2 $[M+H]^+$.

Example 33

3-ethyl-7-((4-(2-(trifluoromethyl)imidazo[1,2-a]pyrazin-6-yl)-3,6-dihydropyridin-1(2H)-yl)methyl)-1,5-naphthyridin-2(1H)-one (Compound 33)

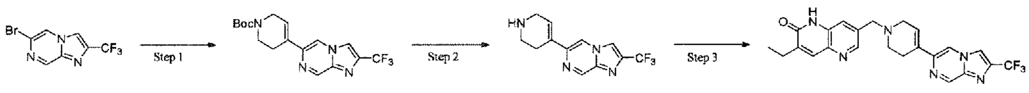


33A 33B 33C Compound 33

Step 1:

33A (280 mg, 1.05 mmol), N-Boc-1,2,5,6-tetrahydropyridine-4-boronic acid pinacol ester (0.49 g, 1.58 mmol) and potassium carbonate (0.29 g, 2.10 mmol) were successively added to N,N-dimethylformamide (20 mL) solution, and the mixture was subjected to nitrogen replacement three times. Chloro(2-dicyclohexylphosphino-2',6'-di-isopropoxy-1,1'-biphenyl)(2-amino-1,1'-biphenyl-2-yl)palladium (II) (41 mg, 0.053 mmol) and water (47 mg, 2.63 mmol) were added, and the mixture was subjected to nitrogen replacement again three times, and then warmed to 110°C and reacted for 4 hours. After the reaction was completed, the reaction liquid was cooled to room temperature, ethyl acetate (100 mL) was added, and the mixture was stirred for 15 minutes and filtered. The filtrate was washed with water (200 mL×3), the organic phase was dried over anhydrous sodium sulphate and concentrated to dryness, and the residue was purified by silica gel column (methanol : dichloromethane (v/v) = 0 : 1-1 : 10), to afford compound 33B (350 mg, yield: 90.49%). LC-MS (ESI): m/z = 369.50 $[M+H]^+$

Step 2:

33B (320 mg, 0.87 mmol) was added to a solution of hydrogen chloride in dioxane (4.0 mol/L, 20 mL), and the mixture was reacted at room temperature for 5 hours. The reaction liquid was concentrated to dryness, isopropanol (2 mL) solution and ethyl acetate (10 mL) solution were added, and the mixture was stirred for 1 hour and filtered, to afford intermediate 33C (225 mg, yield: 84.88%).
LC-MS (ESI): m/z = 269.30 $[M+H]^+$

Step 3:

33C (130 mg, 0.43 mmol) and 1G (100 mg, 0.39 mmol) were added to a mixed solution of acetonitrile (20 mL) and N,N-dimethylformamide (2 mL), potassium iodide (65 mg, 0.39 mmol) and N,N-diisopropylethylamine (1.00 mL) were then added, and the mixture was warmed to 80°C and reacted for 2 hours. After the reaction was completed, the reaction liquid was concentrated to dryness, and the residue was separated and purified by reverse phase column (C18 spherical 20-35 nm 100A 120 g; acetonitrile: water (v/v) = 5%-60%), to afford title compound 33 (55 mg, yield: 31.03%).

LC-MS (ESI): m/z = 455.30 $[M+H]^+$
$^1$H NMR (400 MHz, DMSO-$d_6$) δ 11.84 (s, 1H), 9.19 (s, 1H), 8.60 (d, 2H), 8.42 (d, 1H), 7.76 (s, 1H), 7.65 (d, 1H), 6.86 (d, 1H), 3.74 (s, 2H), 3.20 (d, 2H), 2.74 (t, 2H), 2.54 (d, 4H), 1.19 (t, 3H).

Example 34

3-ethyl-7-((4-(2-ethylimidazo[1,2-a]pyrazin-6-yl)-3,6-dihydropyridin-1(2H)-yl)methyl)-1,5-naphthyridin-2(111)-one (Compound 34)

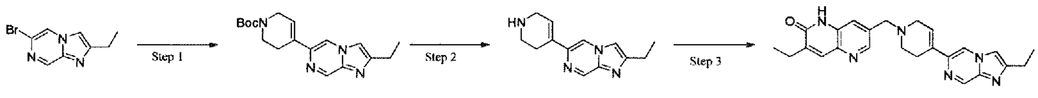


34A 34B 34C Compound 34

Step 1:

34A (200 mg, 0.88 mmol), N-Boc-1,2,5,6-tetrahydropyridine-4-boronic acid pinacol ester (0.41 g, 1.32 mmol) and potassium carbonate (0.24 g, 1.76 mmol) were successively added to N,N-dimethylformamide (20 mL) solution, and the mixture was subjected to nitrogen replacement three times. Chloro(2-dicyclohexylphosphino-2',6'-di-isopropoxy-1,1'-biphenyl)(2-amino-1,1'-biphenyl-2-yl)palladium (II) (34 mg, 0.044 mmol) and water (40 mg, 2.20 mmol) were added, and the mixture was subjected to nitrogen replacement again three times, and then warmed to 110°C and reacted for 4 hours. After the reaction was completed, the reaction liquid was cooled to room temperature, ethyl acetate (100 mL) was added, and the mixture was stirred for 15 minutes and filtered. The filtrate was washed with water (200 mL×3), the organic phase was dried over anhydrous sodium sulphate and concentrated to dryness, and the residue was purified by silica gel column (methanol: dichloromethane (v/v) = 0 : 1-1 : 10), to afford compound 34B (220 mg, yield: 76.12%).
LC-MS (ESI): m/z = 329.40 $[M+H]^+$

Step 2:

34B (220 mg, 0.69 mmol) was added to a solution of hydrogen chloride in dioxane (4.0 mol/L, 20 mL), and the mixture was reacted at room temperature for 5 hours. The reaction liquid was concentrated to dryness, isopropanol (2 mL) solution and ethyl acetate (10 mL) solution were added, and the mixture was stirred for 1 hour and filtered, to afford intermediate 34C (94 mg, yield: 51.46%).
LC-MS (ESI): m/z = 229.30 $[M+H]^+$

Step 3:

34C (94 mg, 0.37 mmol) and 1G (95 mg, 0.37 mmol) were added to a mixed solution of acetonitrile (20 mL) and N,N-dimethylformamide (2 mL), potassium iodide (55 mg, 0.37 mmol) and N,N-diisopropylethylamine (1.00 mL) were then added, and the mixture was warmed to 80°C and reacted for 2 hours. After the reaction was completed, the reaction liquid was concentrated to dryness, and the residue was separated and purified by reverse phase column (C18 spherical 20-35 nm 100A 120 g; acetonitrile : water (v/v) = 5%-60%), to afford title compound 34 (45 mg, yield: 32.37%).

LC-MS (ESI): m/z = 415.20 $[M+H]^+$
$^1$H NMR (400 MHz, DMSO-$d_6$) δ 11.84 (s, 1H), 8.88 (t, 1H), 8.49 (d, 1H), 8.42 (d, 1H), 7.84 (s, 1H), 7.76 (s, 1H), 7.66 (d, 1H), 6.75 (d, 1H), 3.73 (s, 2H), 3.17 (d, 2H), 2.81 - 2.68 (m, 4H), 2.55 (td, 4H), 1.23 (dt, 6H).

Example 35

7-((4-(2-(difluoromethyl)imidazo[1,2-a]pyrazin-6-yl)-3,6-dihydropyridin-1(2H)-yl)methyl)-3-ethyl-1,5-naphthyridin-2(1H)-one (Compound 35)

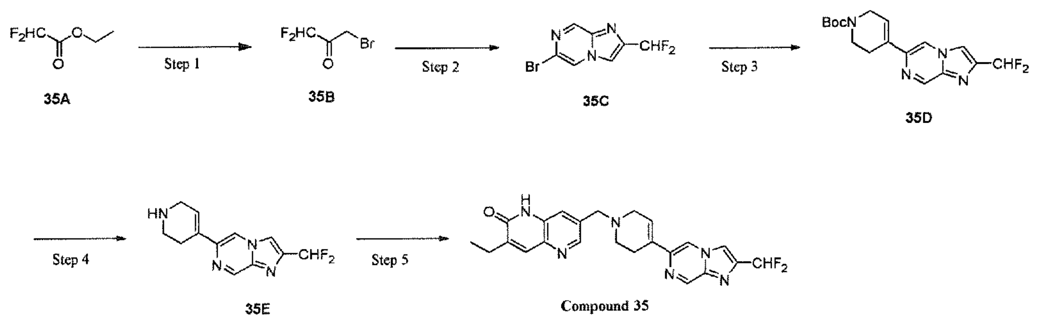


35A 35B 35C 35D 35E Compound 35

Step 1:

35A (5 g, 40.27 mmol) was dissolved in anhydrous tetrahydrofuran, and the mixture was subjected to nitrogen replacement three times. Dibromomethane (14 g, 80.54 mmol) was added, the reaction system was cooled to -78°C, methyllithium (80.54 mmol) was added dropwise, and the mixture was reacted at -78°C for 2 h. After the reaction was completed, saturated ammonium chloride solution was added at 0°C to quench the reaction, and the mixture was extracted with ethyl acetate (50 ml×3). The organic phases were combined, dried over anhydrous sodium sulphate, filtered and concentrated, and the residue was separated and purified by flash column chromatography (eluent: EA/PE = 0%-20%), to afford compound 35B (5.01 g, 72%).
LC-MS (ESI): m/z=173.2[M+1]$^+$

Step 2:

35B (1 g, 5.78 mmol), 5-bromopyrazin-2-amine (0.33 g, 1.90 mmol), and N,N-dimethylacetamide (5 mL) were added to a sealed tube, and the mixture was stirred at 100°C for 16 h. After the reaction was completed, the reaction system was directly subjected to preparative HPLC with preparative HPLC separation methods: 1. Instruments: waters 2767 (preparative liquid phase chromatographic instrument); chromatographic column: SunFire@ Prep C18 (19 mm × 250 mm) 2. The sample was filtered with a 0.45 μm filter to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 40%-70%; c. flow rate: 12 mL/min; retention time: 10.0 min, to afford 35C (250 mg, 53%).
LC-MS (ESI): m/z=248.0[M+1]$^+$

Step 3:

35C (280 mg, 1.13 mmol), N-Boc-1,2,5,6-tetrahydropyridine-4-boronic acid pinacol ester (0.52 g, 1.69 mmol) and potassium carbonate (0.31 g, 2.26 mmol) were successively added to N,N-dimethylformamide (20 mL) solution, and the mixture was subjected to nitrogen replacement three times. Chloro(2-dicyclohexylphosphino-2',6'-di-isopropoxy-1,1'-biphenyl)(2-amino-1,1'-biphenyl-2-yl)palladium (II) (44 mg, 0.056 mmol) and water (51 mg, 2.82 mmol) were added, and the mixture was subjected to nitrogen replacement again three times, and then warmed to 110°C and reacted for 4 hours. After the reaction was completed, the reaction liquid was cooled to room temperature, ethyl acetate (100 mL) was added, and the mixture was stirred for 15 minutes and filtered. The filtrate was washed with water (200 mL×3), the organic phase was dried over anhydrous sodium sulphate and concentrated to dryness, and the residue was purified by silica gel column (methanol: dichloromethane (v/v) = 0 : 1-1 : 10), to afford compound 35D (300 mg, yield: 75.78%).
LC-MS (ESI): m/z = 351.40 [M+H]$^+$

Step 4:

35D (300 mg, 0.86 mmol) was added to a solution of hydrogen chloride in dioxane (4.0 mol/L, 20 mL), and the mixture was reacted at room temperature for 5 hours. The reaction liquid was concentrated to dryness, isopropanol (2 mL) solution and ethyl acetate (10 mL) solution were added, and the mixture was stirred for 1 hour and filtered, to afford intermediate 35E (194 mg, yield: 78.68%).
LC-MS (ESI): m/z = 251.30 [M+H]$^+$

Step 5:

35E (110 mg, 0.43 mmol) and 1G (100 mg, 0.39 mmol) were added to a mixed solution of acetonitrile (20 mL) and N,N-dimethylformamide (2 mL), potassium iodide (65 mg, 0.39 mmol) and N,N-diisopropylethylamine (1.00 mL) were then added, and the mixture was warmed to 80°C and reacted for 2 hours. After the reaction was completed, the reaction liquid was concentrated to dryness, and the residue was separated and purified by reverse phase column (C18 spherical 20-35 nm 100A 120 g; acetonitrile : water (v/v) = 5%-60%), to afford title compound 35 (58 mg, yield: 34.07%).

LC-MS (ESI): m/z = 437.20 $[M+H]^+$

$^1$H NMR (400 MHz, DMSO-$d_6$) δ 11.84 (s, 1H), 9.11 (t, 1H), 8.60 (d, 1H), 8.42 (d, 1H), 8.36 (d, 1H), 7.76 (d, 1H), 7.66 (d, 1H), 7.23 (t, 1H), 6.83 (s, 1H), 3.74 (s, 2H), 3.19 (d, 2H), 2.74 (t, 2H), 2.59 - 2.52 (m, 4H), 1.19 (t, 3H).

Biological test

1. PARP-1 Enzyme Activity Test Experiment

PARP-1 chemical fluorescence detection kit was purchased from BPS Bioscience. The histone solution in the kit was diluted 5X with 1X PBS, and 25 μL of the diluted histone solution was added to a microwell plate and incubated overnight at 4°C. After the incubation, the plate was washed three times with PBST (0.05% Tween-20). 100 μL of the blocking solution was added to the microwell plate and incubated at 25°C for 90 minutes. After the incubation, the plate was washed three times with PBST. 2.5 μL of compounds at different concentrations diluted in test buffer and 12.5 μL of substrate mixed solution (1.25 μL 10X PARP test buffer; 1.25 μL 10X PARP test mixed solution: 2.5 μL Activated DNA, 7.5 μL double-distilled water) were added to the microwell plate. The PARP-1 enzyme was diluted to 2 ng/μL, 10 μL of the diluent was added to the microwell plate, and the reaction system was incubated at 25°C for 60 minutes.

After the incubation, the plate was washed three times with PBST. Streptavidin-HRP was diluted 50X with a blocking solution, and 25 μL of the diluent was added to the microwell plate and incubated at 25°C for 30 minutes. After the incubation, the plate was washed three times with PBST. ELISA ECL substrate A and substrate B were mixed at a ratio of 1 : 1 (v/v), 50 μL of the mixture was added to the microwell plate, and the chemiluminescence value was read.

The inhibition rate was calculated according to formula 1, where RLUsample was the readout of the compound well, RLUmax was the readout of the solvent control well, and RLUmin was the readout of the control well without the PARP-1 enzyme. Curve fitting was performed by four parameters (log(inhibitor) vs. response -- Variable slope) using GraphPad Prism software, and the IC50 value was calculated.

$$\text{Inhibition\%} = (1-(\text{RLUsample}-\text{RLUmin})/(\text{RLUmax}-\text{RLUmin})) \times 100\% \text{ (formula 1)}$$

Test results: the compound of the present invention had a significant inhibitory effect on PARP-1 enzyme activity in vitro, and the IC50 value of the example compounds on PARP-1 enzyme activity was less than 100 μM. The test results of some examples were as shown in Table 1.

Table 1 PARP-1 enzyme activity

| Compound | $IC_{50}$ (nM) |
|---|---|
| Compound 1 | 0.8 |
| Compound 3 | 1.6 |
| Compound 6 | 0.51 |
| Compound 7 | 2.8 |
| Compound 8 | 0.78 |
| Compound 13 | 0.79 |
| Compound 14 | 0.59 |
| Compound 19 | 0.36 |
| Compound 20 | 0.26 |
| Compound 22 | 0.31 |

(continued)

| Compound | $IC_{50}$ (nM) |
|---|---|
| Compound 24 | 0.66 |
| Compound 25 | 0.36 |
| Compound 26 | 0.41 |
| Compound 27 | 0.41 |
| Compound 28 | 0.33 |
| Compound 29 | 0.95 |
| Compound 30 | 0.26 |
| Compound 32 | 0.19 |
| Compound 33 | 0.71 |

Conclusion: the compound of the present invention has a significant inhibitory effect on PARP-1 enzyme activity in vitro.

2. MDA-MB-436 Cell Activity Test Experiment

Breast tumour cells MDA-MB-436 were purchased from ATCC, the culture medium was Leibovitz's L-15 +10% FBS, and the cells were cultured in a $GO_2$-free incubator at 37°C. On day 1, the cells in the exponential growth phase were collected, and the cell suspension was adjusted to 4000 cells/135 μL with the culture medium. 135 μL of the cell suspension was added to each well of a 96-well cell culture plate and incubated overnight. On day 2, compounds at different concentrations were added, and the plate was placed in the incubator and incubated for 7 days. After the incubation was completed, according to operation instructions for a CellTiter-Glo kit (Promega, G7573), 75 μL of CTG solution, which was already pre-melted and equilibrated to room temperature, was added to each well, and the mixture was uniformly mixed for 2 min using a microplate shaker. The plate was placed at room temperature for 10 min, and then fluorescence signal values were measured using an Envision2104 plate reader (PerkinElmer). The inhibition rate was calculated using formula (1), where $RLU_{compound}$ was the readout of the drug treated group, $RLU_{control}$ was the average value of the vehicle control group, and $RLU_{blank}$ was the average value of the cell-free well. The $IC_{50}$ value was calculated using GraphPad Prism software.

$$\text{Inh.\%} = (1 - (RLU_{compound} - RLU_{blank}) / (RLU_{control} - RLU_{blank})) \times 100\% \text{ (formula 1)}$$

Test results: the compounds of the present invention had a significant inhibitory effect on MDA-MB-436 cells, the IC50 value of the compounds on MDA-MB-436 cells was less than 100 nM, the IC50 value of some excellent compounds on MDA-MB-436 cells was less than 10 nM, and the IC50 value of more excellent compounds on MDA-MB-436 cells was less than 1 nM; and the inhibitory rate of the compounds on breast tumour cells MDA-MB-436 was greater than 70%, the inhibition rate of some excellent compounds was greater than 85%, and the inhibition rate of more excellent compounds was greater than 90%. The results of some specific compounds were as shown in Table 2.

Table 2 MDA-MB-436 cell activity

| Compound | $IC_{50}$ (nM) | Max inh.% 10 μM |
|---|---|---|
| 14 | 3.2 | 89.1 |
| 20 | 0.7 | 91.85 |
| 22 | 2.2 | 89.07 |
| 26 | 1.0 | 90.91 |
| 30 | 0.7 | 91.85 |
| 32 | 13.8 | 99.1 |

(continued)

| Compound | $IC_{50}$ (nM) | Max inh.% 10 μM |
|---|---|---|
| 33 | 1.9 | 77.6 |

Conclusion: the compound of the present invention has good inhibitory activity on breast tumour cells MDA-MB-436.

3. PARP2 and PARP7 Enzyme Activity Test Experiment

PARP2 and PARP7 chemical fluorescence detection kits were both purchased from BPS Bioscience. The histone solution in the kit was diluted 5X with 1X PBS, and 25 μL of the diluted histone solution was added to a microwell plate and incubated overnight at 4°C. After the incubation, the plate was washed three times with PBST (0.05% Tween-20). 100 μL of the blocking solution was added to the microwell plate and incubated at 25°C for 90 minutes. After the incubation, the plate was washed three times with PBST. 2.5 μL of compound 4 diluted in test buffer and 5 μL of substrate mixed solution were added to the microwell plate. 5 μL of the diluted PARP enzyme was added to the microwell plate, and the reaction system was incubated at 25°C for 60 minutes.

After the incubation, the plate was washed three times with PBST. Streptavidin-HRP was diluted 50X with a blocking solution, and 25 μL of the diluent was added to the microwell plate and incubated at 25°C for 30 minutes. After the incubation, the plate was washed three times with PBST. ELISA ECL substrate A and substrate B were mixed at a ratio of 1 : 1 (v/v), 25 μL of the mixture was added to the microwell plate, and the chemiluminescence value was read.

The inhibition rate was calculated according to formula $[(1-(RLU_{sample}-RLU_{min})/(RLU_{max}-RLU_{min})) \times 100\%]$, where $RLU_{sample}$ was the readout of the compound well, $RLU_{max}$ was the readout of the solvent control well, and $RLU_{min}$ was the readout of the control well without the PARP1 enzyme. Curve fitting was performed by four parameters (log(inhibitor) vs. response -- Variable slope) using GraphPad Prism software, and the $IC_{50}$ value was calculated.

Test results: compound 14 of the present invention had a weak inhibitory effect on PARP2 enzyme activity in vitro, and its corresponding $IC_{50}$ value was 31 nM; and compound 14 had a weak inhibitory effect on PARP7 enzyme activity in vitro, and its corresponding $IC_{50}$ value was 40 nM. The specific test results were as shown in Table 3.

Table 3 PARP2 and PARP7 enzyme activity

| Compound | PARP enzyme | $IC_{50}$ (nM) |
|---|---|---|
| 14 | PARP2 | 31 |
| 14 | PARP7 | 40 |

Conclusion: the inhibitory effect of compound 14 of the present invention on PARP2 and PARP7 enzyme activities in vitro is much weaker than the inhibitory effect on PARP1, indicating that it has good PARP1 inhibitory selectivity.

4. MDA-MB-231 Cell Proliferation Inhibition Test

Human breast cancer MDA-MB-231 cells purchased from ATCC were placed in complete DMEM medium (supplemented with 10% foetal bovine serum and 1% double antibody) and cultured at 37°C and 5% $CO_2$. The cells in the exponential growth phase were collected, and the cell suspension was adjusted to 1500 cells/135 μL with the culture medium. 135 μL of the cell suspension was added to each well of a 96-well cell culture plate and incubated overnight. On day 2, compounds at different concentrations were added, and the plate was placed in the incubator and incubated for 7 days. After the incubation was completed, according to operation instructions for a CellTiter-Glo kit (Promega, Cat# G7573), 75 μL of CTG solution, which was already pre-melted and equilibrated to room temperature, was added to each well, and the mixture was uniformly mixed for 2 min using a microplate shaker. The plate was placed at room temperature for 10 min, and then fluorescence signal values were measured using an Envision 2104 plate reader (PerkinElmer). The cell proliferation inhibition rate was calculated according to the formula $[(1-(RLU_{compound} - RLU_{blank}) / (RLU_{control} - RLU_{blank})) \times 100\%]$. The $IC_{50}$ value was obtained by four-parameter nonlinear fitting using GraphPad Prism software.

Table 4 MDA-MB-231 cell inhibitory activity

| Compound | $IC_{50}$ (μM) | Max inh.% 10 μM |
|---|---|---|
| 14 | 21.8 | 59.16 |

Conclusion: the compound of the present invention has weak inhibitory activity on BRCA WT cells MDA-MB-231, indicating that it has good cell selectivity.

5. Pharmacokinetic Test among Animals of Varying Genera

5.1 Pharmacokinetic test in rats

5.1.1 Experimental animals: male SD rats, about 220 g, 6-8 weeks old, 6 rats/compound, purchased from Chengdu Ddossy Experimental Animals Co., Ltd.

5.1.2 Experimental design: on the day of the test, 6 SD rats were randomly grouped according to their body weight; the animals were fasted with water available for 12 to 14 h one day before the administration, and were fed 4 h after the administration.

Table 5. Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | Compound 14 | 2.5 | 0.5 | 5 | Plasma | Intravenously |
| G2 | 3 | | 10 | | 10 | Plasma | Intragastrically |

Notes: Solvent for intravenous administration: 5% DMA + 5% Solutol + 90% Saline; Solvent for intragastric administration: 5% DMSO + 30% PEG400 + 65% (20% SBE-CD) (DMA: dimethylacetamide; Solutol: polyethylene glycol-15-hydroxystearate; Saline: physiological saline; MC: methylcellulose)

Before and after the administration, 0.15 ml of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous administration group and intragastric administration group were: 0, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

Table 6. Pharmacokinetic parameters of test compounds in plasma of rats

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 14 | i.v. (2.5 mg/kg) | 0.494 | 0.164 | 84150 | - |
| | i.g. (10 mg/kg) | - | - | 248362 | 73.8 |
| -: not applicable. | | | | | |

5.2 Pharmacokinetic test in mice

5.2.1 Experimental animals: male ICR mice, 20-25 g, 18 mice/compound. purchased from Chengdu Ddossy Experimental Animals Co., Ltd.

5.2.2 Experimental design: on the day of the test, 18 ICR mice were randomly grouped according to their body weights. the animals were fasted with water available for 12 to 14 h one day before the administration, and were fed 4 h after the administration.

Table 7. Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 9 | Compound 14 | 2.5 | 0.5 | 5 | Plasma | Intravenously |
| G2 | 9 | | 10 | | 10 | Plasma | Intragastrically |
| Notes: Solvent for intravenous administration: 5% DMA + 5% Solutol + 90% Saline; Solvent for intragastric administration: 5% DMSO + 30% PEG400 + 65% (20% SBE-CD) (DMA: dimethylacetamide; Solutol: polyethylene glycol-15-hydroxystearate; Saline: physiological saline; MC: methylcellulose) | | | | | | | |

Before and after the administration, 0.06 mL of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous group and intragastric administration group were: 0 min, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

Table 8. Pharmacokinetic parameters of test compounds in plasma of mice

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 14 | i.v. (2.5 mg/kg) | 0.164 | 0.112 | 219576 | - |
| | i.g. (10 mg/kg) | - | - | 828551 | 94.3 |
| -: not applicable. | | | | | |

Conclusion: compound 14 has good pharmacokinetic characteristics in rats and mice.

5. Mouse MDA-MB-436 Subcutaneous in vivo Transplanted Tumour Model

Human breast cancer MDA-MB-436 cells were placed in Leibovitz's L-15 medium (supplemented with 10 μg/mL insulin, 16 μg/mL glutathione, 10% foetal bovine serum and 1% double antibody) and cultured at 37°C. Conventional digestion treatment with trypsin was carried out twice a week for passage. When the cell saturation was 80%-90%, and the number reached the requirement, the cells were collected, counted, and inoculated. BALB/c nude mice (from Beijing Vital River Laboratory Animal Technology Co., Ltd.) were subcutaneously inoculated with 0.2 mL ($10 \times 10^6$) of MDA-MB-436 cells (plus matrigel, with the volume ratio of 1 : 1) on the right back. When the average tumour volume reached about 180 $mm^3$, grouping and administration were performed (marked as Day 0). The solvent group was given 5% DMSO, 30% PEG400 and 65% of 20% sulfobutyl-β-cyclodextrin solution, and the administration group was given compound 14 (Day 0-Day 10: 1 mg/kg; Day 11-Day 28: 0.1 mg/ kg). The administration frequency was once a day, the administration cycle was 29 days, and the drug withdrawal observation period was set to 14 days. After grouping, the tumour diameter was measured twice a week with a vernier calliper. The formula for calculating the tumour volume was: $V = 0.5 \times a \times b^2$, where a and b represented the long and short diameters of the tumour, respectively. The tumour inhibitory effect of compound 14 was evaluated by TGI (%) = [(1-(average tumour volume at the end of administration in the treatment group - average tumour volume at the beginning of administration in the treatment group))/(average tumour volume at the end of treatment in the solvent control group - average tumour volume at the beginning of treatment in the solvent control group)] × 100%. The tumour growth curve and the animal body weight change curve were as shown in Figure 1 and Figure 2, respectively.

Test results: after 28 days of administration, the TGI of the group given compound 14 was 121 %; after the drug withdrawal, the tumour of the animals in the group given compound 14 did not grow again; there was no significant decrease in the body weight of the animals in the group given compound 14.

Conclusion: in the mouse MDA-MB-436 subcutaneous in vivo transplanted tumour model, compound 14 of the present invention has good efficacy in inhibiting tumour growth and inducing tumour regression, and is well tolerated.

## Claims

1. A compound represented by formula (1), (I-1) or (1-2), or a stereoisomer, solvate, or pharmaceutically acceptable salt thereof,

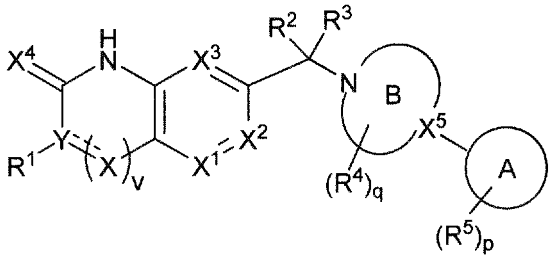

(I)

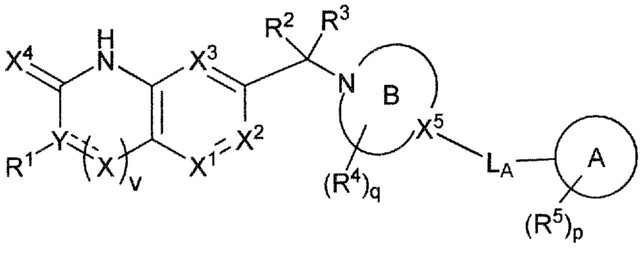

(I-1)

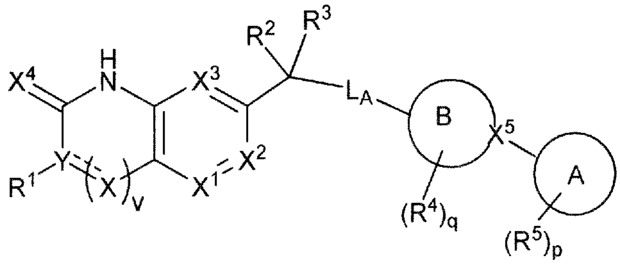

(I-2)

**characterized in that** each X is independently selected from $CR^x$, $C(R^x)_2$, O, N or $NR^x$;
Y is selected from N, C or CH;

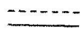

represents a single bond or a double bond, provided that when



represents a single bond, X is selected from $C(R^x)_2$, O or $NR^x$;
v is selected from 1, 2 or 3;
$X^1$, $X^2$ and $X^3$ are each independently selected from N or $CR^x$;
$X^4$ is selected from O or S;
$X^5$ is independently selected from N, C or $CR^x$;
each $R^x$ is independently selected from H, D, halogen, cyano, amino, hydroxyl, $-SF_5$, $C_{1-6}$ alkyl, halo $C_{1-6}$ alkyl, halo $C_{1-6}$ alkoxy, deuterated $C_{1-6}$ alkyl, deuterated $C_{1-6}$ alkoxy, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl); or two $R^x$ on the same carbon atom together form =O;
$R^1$ is selected from halogen, nitro, cyano, amino, hydroxyl, $-SF_5$, $C_{1-6}$ alkyl, $C_{1-6}$ alkoxy, $C_{2-6}$ alkenyl, $C_{2-6}$ alkynyl, $C_{1-6}$ alkyl-O-$C_{1-6}$ alkyl, $-(CH_2)_r-C_{3-12}$ cycloalkyl or $-(CH_2)_r$-(3- to 12-membered heterocycloalkyl), wherein the alkyl, alkoxy, alkenyl, alkynyl, cycloalkyl or heterocycloalkyl is optionally further substituted with 1-3 groups selected from D, halogen, cyano, amino, hydroxyl, $C_{1-6}$ alkyl or $C_{1-6}$ alkoxy;
each r is independently selected from 0, 1, 2 or 3; p is selected from 0, 1, 2 or 3;
$R^2$ and $R^3$ are each independently selected from H, D, halogen, cyano, amino, hydroxyl, $C_{1-6}$ alkyl-O-$C_{1-6}$ alkyl, hydroxy $C_{1-6}$ alkyl, $C_{1-6}$ alkoxy, halo $C_{1-6}$ alkyl, halo $C_{1-6}$ alkoxy, deuterated $C_{1-6}$ alkyl, deuterated $C_{1-6}$ alkoxy or $C_{1-6}$ alkyl; or $R^2$ and $R^3$ together with the carbon atom to which they are attached form $C_{3-5}$ cycloalkyl or 4- to 5-membered heterocycloalkyl;
ring B is piperazinyl, and q is selected from 1 or 2; or
ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6- to 8-membered saturated bridged heterocycle containing 1-4 nitrogen atoms, 5-to 10-membered saturated fused heterocycle containing 1-4 nitrogen atoms, or 5- to 11-membered saturated spiro heterocycle containing 1-4 nitrogen atoms, and q is selected from 0, 1, 2 or 3;
each $R^4$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy; or two $R^4$ on the same carbon atom together with the carbon atom to which they are attached form =O or $C_{3-5}$ cycloalkyl;

each $R^5$ is independently selected from D, halogen, cyano, amino, hydroxyl, - $SF_5$, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

$L_A$ is selected from a bond, -NH-, -$NR^{a1}$-, -O-, -S-, -S(=O)-, -$S(=O)_2$-, -NH-C(=O)-, -C(=O)-NH-, $C_{1-6}$ alkyl, $C_{1-6}$ alkoxy, halo $C_{1-6}$ alkyl, halo $C_{1-6}$ alkoxy, deuterated $C_{1-6}$ alkyl or deuterated $C_{1-6}$ alkoxy;

ring A is selected from 5- to 6-membered monocyclic heteroaromatic ring containing 1-5 nitrogen, oxygen or sulphur atoms, wherein the heteroaromatic ring is further substituted with 1-3 substituents selected from $R_a$; or ring A is selected from 7- to 10-membered bicyclic heteroaromatic ring containing 1-5 nitrogen, oxygen or sulphur atoms, or 7- to 10-membered bicyclic aromatic ring, wherein the heteroaromatic ring or aromatic ring is optionally further substituted with 1-3 substituents selected from $R_b$;

each $R_a$ is independently selected from -C(O)N$(R^{a1})_2$, -$NR^{a1}$C(O)$OR^{a1}$, - $NR^{a1}$C(O)$R^{a1}$, -$NR^{a1}$C(O)N$(R^{a1})_2$, -C(=S)N$(R^{a1})_2$, -$S(O)_2$N$(R^{a1})_2$, 5- to 6-membered monocyclic heteroaryl containing 1-5 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, -$NHC_{1-6}$ alkyl, -N($C_{1-6}$ alkyl$)_2$, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy;

each $R_b$ is independently selected from -C(O)N$(R^{a1})_2$, -$NR^{a1}$C(O)$OR^{a1}$, - $NR^{a1}$C(O)N$(R^{a1})_2$, -C(=S)N$(R^{a1})_2$, -$S(O)_2$N$(R^{a1})_2$, =O, D, halogen, cyano, hydroxyl, amino, -$NHC_{1-6}$ alkyl, -N($C_{1-6}$ alkyl$)_2$, $C_{1-6}$ alkyl, $C_{3-12}$ cycloalkyl, 3- to 12-membered heterocycloalkyl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy;

each $R^{a1}$ is independently selected from H, D, $C_{1-6}$ alkyl, $C_{3-12}$ cycloalkyl, 3- to 12-membered heterocycloalkyl, 5- to 8-membered heteroaryl, $C_{1-6}$ alkoxy, $C_{1-6}$ alkyl-O-$C_{1-6}$ alkyl, halo $C_{1-6}$ alkyl, halo $C_{1-6}$ alkoxy, deuterated $C_{1-6}$ alkyl or deuterated $C_{1-6}$ alkoxy, wherein the cycloalkyl, heterocycloalkyl, or heteroaryl is optionally further substituted with 1-3 substituents selected from D, halogen, cyano, hydroxyl, amino, $C_{1-6}$ alkyl, halo $C_{1-6}$ alkyl, $C_{1-6}$ alkoxy, halo $C_{1-6}$ alkoxy, deuterated $C_{1-6}$ alkyl or deuterated $C_{1-6}$ alkoxy; or two $R^{a1}$ together with the nitrogen atom form 4- to 6-membered heterocycloalkyl;

alternatively, $L_A$ is selected from a bond, and the carbon atom to which $R^3$ is attached and the linking site of ring B directly form a double bond;

unless otherwise specified, the above-mentioned heterocycloalkane, heterocycloalkyl, heteroaryl, or heteroaromatic ring contains 1-5 heteroatoms selected from nitrogen, oxygen or sulphur.

2. The compound, or the stereoisomer, solvate, or pharmaceutically acceptable salt thereof according to claim 1, having a structure of formula (II), (II-a), (II-b), (III), (III-a), (III-b), (IV), (IV-a), (IV-b) or (V):

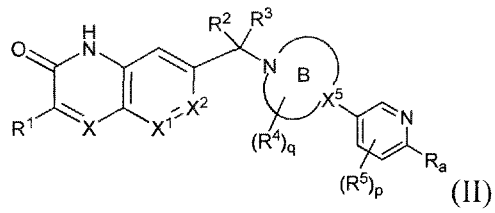
(II)

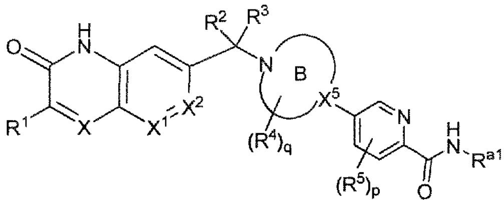
(II-a)

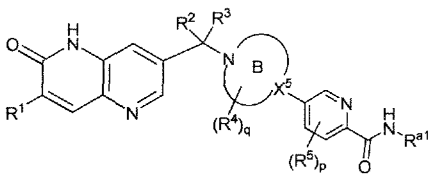
(II-b)

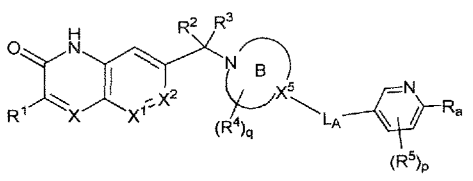
(III)

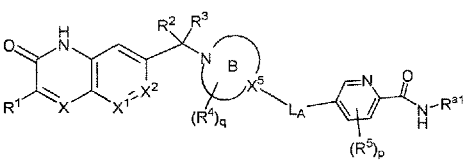
(III-a)

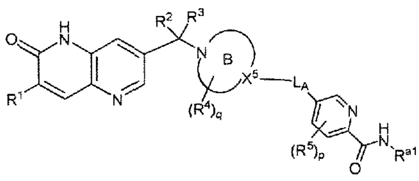
(III-b)

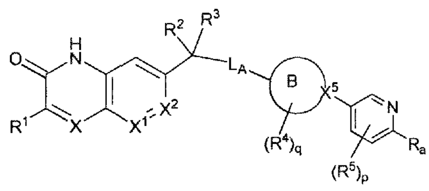 (IV) 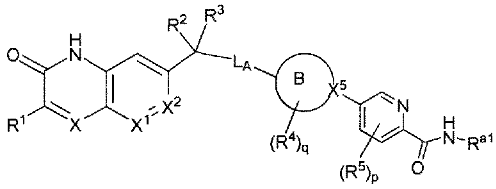 (IV-a)

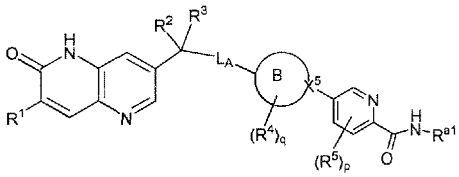 (IV-b) 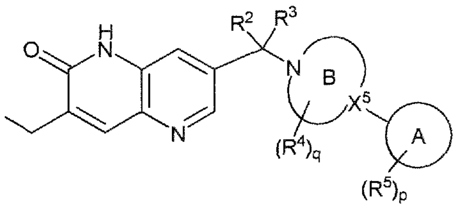 (V)

**characterized in that** X is selected from $CR^x$ or N;

$R^x$ is independently selected from H, D, halogen, cyano, amino, hydroxyl, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl or $-(CH_2)_r-$(4- to 6-membered monocyclic heterocycloalkyl);

$X^3$ is independently selected from N, C or CH;

$L_A$ is selected from a bond, -NH-, -N($C_{1-4}$ alkyl)-, -O-, -S-, -S(=O)-, -S(=O)$_2$- or $C_{1-4}$ alkyl;

alternatively, $L_A$ is selected from a bond, and the carbon atom to which $R^3$ is attached and the linking site of ring B directly form a double bond.

3. The compound, or the stereoisomer, solvate, or pharmaceutically acceptable salt thereof according to claim 2, **characterized in that**

X and $X_2$ are selected from C;

$X_1$ is selected from N;

$R^1$ is selected from ethyl;

$L_A$ is selected from a bond, -NH- or -N($C_{1-2}$ alkyl)-;

alternatively, $L_A$ is selected from a bond, and the carbon atom to which $R^3$ is attached and the linking site of ring B directly form a double bond;

$R^2$ and $R^3$ are each independently selected from H, D, F, Cl, deuterated $C_{1-2}$ alkyl or $C_{1-2}$ alkyl; or $R^2$ and $R^3$ together with the carbon atom to which they are attached form $C_{3-4}$ cycloalkyl;

each $R^4$ is independently selected from D, F, Cl, cyano, amino, hydroxyl, $-SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy; or two $R^4$ on the same carbon atom together with the carbon atom to which they are attached form =O or $C_{3-4}$ cycloalkyl;

each $R^5$ is independently selected from D, F, Cl, cyano, amino, hydroxyl, $-SF_5$, $C_{1-2}$ alkyl, $C_{1-2}$ alkoxy, halo $C_{1-2}$ alkyl, halo $C_{1-2}$ alkoxy, deuterated $C_{1-2}$ alkyl or deuterated $C_{1-2}$ alkoxy;

ring A is selected from 7- to 10-membered bicyclic heteroaromatic ring containing 1-5 nitrogen, oxygen or sulphur atoms, wherein the heteroaromatic ring is optionally further substituted with 1-3 substituents selected from D, F, Cl, cyano, hydroxyl, amino, $C_{1-2}$ alkyl, $C_{3-4}$ cycloalkyl, 4- to 5-membered heterocycloalkyl, halo $C_{1-2}$ alkyl, halo $C_{1-2}$ alkoxy, deuterated $C_{1-2}$ alkyl or deuterated $C_{1-2}$ alkoxy;

$R_a$ is selected from $-C(O)N(R^{a1})_2$, $-NR^{a1}C(O)R^{a1}$, or 5- to 6-membered monocyclic heteroaryl containing 1-5 nitrogen, oxygen or sulphur atoms, wherein the heteroaryl is optionally further substituted with 1-3 substituents selected from D, F, Cl, cyano, hydroxyl, amino, $C_{1-2}$ alkyl, halo $C_{1-2}$ alkyl, $C_{1-2}$ alkoxy, halo $C_{1-2}$ alkoxy, deuterated $C_{1-2}$ alkyl or deuterated $C_{1-2}$ alkoxy;

each $R^{a1}$ is independently selected from H, D, $C_{1-2}$ alkyl, $C_{3-5}$ cycloalkyl, 4- to 5-membered heterocycloalkyl, 5- to 6-membered heteroaryl, halo $C_{1-2}$ alkyl, or deuterated $C_{1-2}$ alkyl, wherein the cycloalkyl, heterocycloalkyl, or heteroaryl is optionally further substituted with 1-3 substituents selected from D, F, Cl, cyano, hydroxyl, amino, $C_{1-2}$ alkyl, deuterated $C_{1-2}$ alkyl or deuterated $C_{1-2}$ alkoxy.

4. The compound, or the stereoisomer, solvate, or pharmaceutically acceptable salt thereof according to any one of claims 1-3, **characterized in that**

ring B is piperazinyl, and q is selected from 1 or 2; or

ring B is selected from piperidyl, 4-membered saturated monocyclic heterocycloalkane containing 1-2 nitrogen atoms, 5-membered saturated monocyclic heterocycloalkane containing 1-2 nitrogen atoms, 5- to 6-membered partially unsaturated monocyclic heterocycloalkane containing 1-2 nitrogen atoms, 6-membered saturated bridged heterocycle containing 1-2 nitrogen atoms, 7-membered saturated bridged heterocycle containing 1-2 nitrogen atoms, 8-membered saturated bridged heterocycle containing 1-2 nitrogen atoms, 7-membered saturated fused heterocycle containing 1-2 nitrogen atoms, 8-membered saturated fused heterocycle containing 1-2 nitrogen atoms, 9-membered saturated fused heterocycle containing 1-2 nitrogen atoms, 10-membered saturated fused heterocycle containing 1-2 nitrogen atoms, 7-membered saturated spiro heterocycle containing 1-2 nitrogen atoms, 8-membered saturated spiro heterocycle containing 1-2 nitrogen atoms, 9-membered saturated spiro heterocycle containing 1-2 nitrogen atoms, 10-membered saturated spiro heterocycle containing 1-2 nitrogen atoms or 11-membered saturated spiro heterocycle containing 1-2 nitrogen atoms, and q is selected from 0, 1, 2 or 3.

**5.** The compound, or the stereoisomer, solvate, or pharmaceutically acceptable salt thereof according to any one of claim 1, **characterized in that**

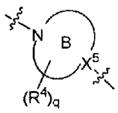

is selected from

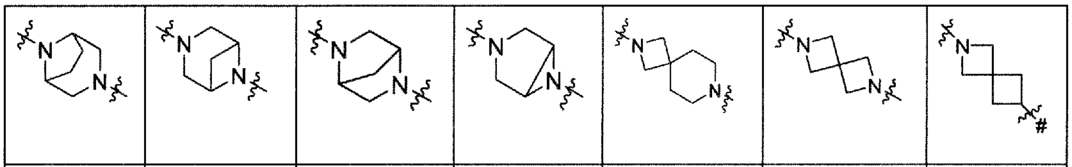

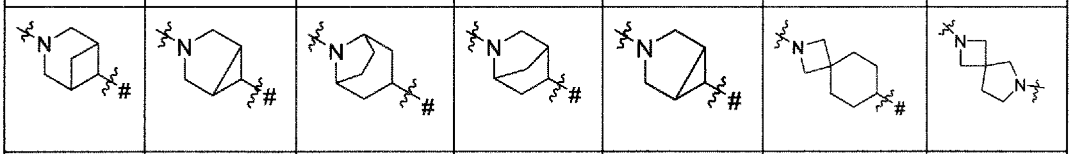

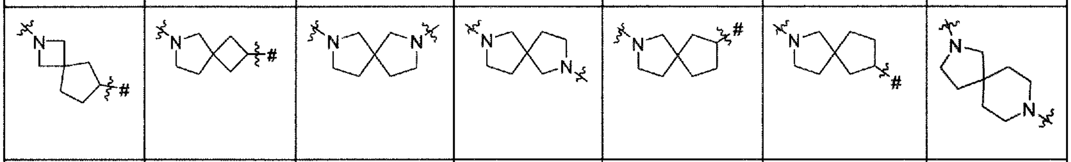

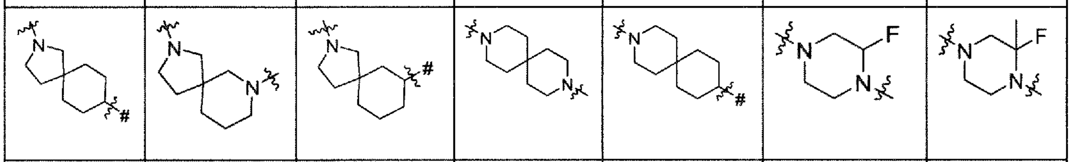

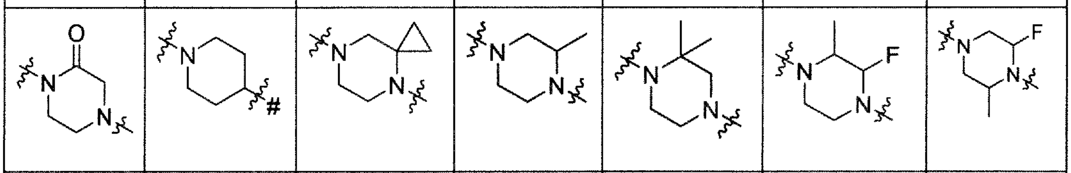

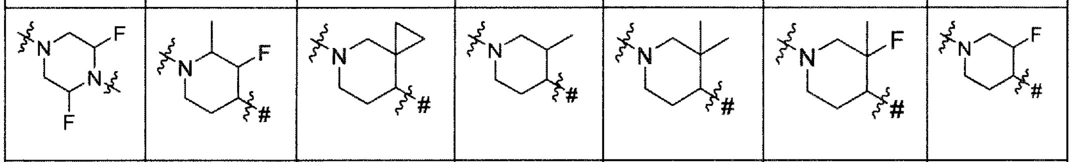

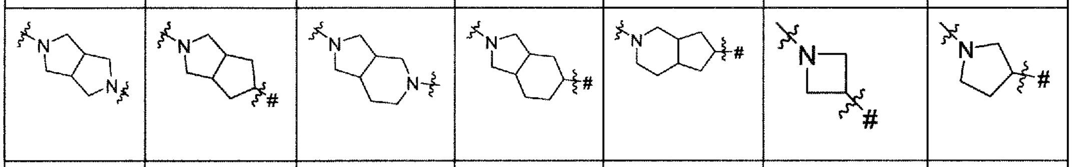

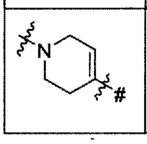 ;

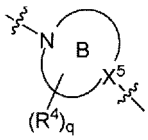

is selected from

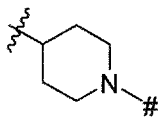 or 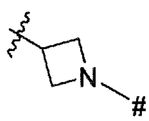 ;

# represents position $X^5$ in ring B, wherein groups with undefined connection positions can be connected at both ends.

**6.** The compound, or the stereoisomer, solvate, or pharmaceutically acceptable salt thereof according to claim 1 or 2, **characterized in that**

$R^1$ is selected from halogen, nitro, cyano, amino, hydroxyl, $-SF_5$, $C_{1-4}$ alkyl, $C_{1-4}$ alkoxy, $C_{2-4}$ alkenyl, $C_{2-4}$ alkynyl, $C_{1-4}$ alkyl-O-$C_{1-4}$ alkyl, $-(CH_2)_r-C_{3-6}$ monocyclic cycloalkyl, $-(CH_2)_r-C_{5-9}$ spiro cycloalkyl, $-(CH_2)_r$-(4- to 6-membered monocyclic heterocycloalkyl) or $-(CH_2)_r$-(5- to 9-membered spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino, hydroxyl, $C_{1-3}$ alkyl or $C_{1-3}$ alkoxy;

each $R_a$ is independently selected from $-C(O)N(R^{a1})_2$, $-NR^{a1}C(O)OR^{a1}$, - $NR^{a1}C(O)R^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-4}$ alkyl, halo $C_{1-4}$ alkyl, $C_{1-4}$ alkoxy, halo $C_{1-4}$ alkoxy, deuterated $C_{1-4}$ alkyl, or deuterated $C_{1-4}$ alkoxy;

each r is independently selected from 0, 1 or 2;

each $R^{a1}$ is independently selected from $C_{14}$ alkyl, $C_{3-5}$ cycloalkyl, $C_{5-9}$ spiro cycloalkyl, 4- to 6-membered heterocycloalkyl, 5- to 9-membered spiro heterocycloalkyl, 5- to 6-membered heteroaryl, $C_{1,4}$ alkoxy, $C_{1-2}$ alkyl-O-$C_{1-2}$ alkyl, halo $C_{1-4}$ alkyl, halo $C_{1-4}$ alkoxy, deuterated $C_{1-4}$ alkyl or deuterated $C_{1-4}$ alkoxy, wherein the cycloalkyl, heterocycloalkyl, or heteroaryl is optionally further substituted with 1-3 substituents selected from D, halogen, $C_{1-4}$ alkyl, halo $C_{1-4}$ alkyl, $C_{1-4}$ alkoxy, halo $C_{1-4}$ alkoxy, deuterated $C_{1-4}$ alkyl or deuterated $C_{1-4}$ alkoxy;

$R^2$ and $R^3$ are each independently selected from H, D, halogen, cyano, amino, hydroxyl, $C_{1-2}$ alkyl-O-$C_{1-2}$ alkyl, hydroxy $C_{1-3}$ alkyl, $C_{1-3}$ alkoxy, halo $C_{1-4}$ alkyl, halo $C_{1-4}$ alkoxy, deuterated $C_{1-4}$ alkyl, deuterated $C_{1-4}$ alkoxy or $C_{1-4}$ alkyl; or $R^2$ and $R^3$ together with the carbon atom to which they are attached form 3-membered cycloalkyl, 4-membered cycloalkyl, 5-membered cycloalkyl, 4-membered heterocycloalkyl, or 5-membered heterocycloalkyl;

each $R^4$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy; or two $R^4$ on the same carbon atom together with the carbon atom to which they are attached form =O;

each $R^5$ is independently selected from D, halogen, cyano, amino, hydroxyl, $C_{1-4}$ alkyl, $C_{1-4}$ alkoxy, halo $C_{1-4}$ alkyl, halo $C_{1-4}$ alkoxy, deuterated $C_{1-4}$ alkyl, or deuterated $C_{1-4}$ alkoxy;

p is selected from 0, 1 or 2.

**7.** The compound, or the stereoisomer, solvate, or pharmaceutically acceptable salt thereof according to claim 1 or 2, **characterized in that**

$R^1$ is selected from cyano, $C_{1-2}$ alkyl, $C_{1-2}$ alkoxy, $C_{2-3}$ alkenyl, $C_{2-3}$ alkynyl, $C_{1-2}$ alkyl-O-$C_{1-2}$ alkyl, $-(CH_2)_r-C_{3-4}$ monocyclic cycloalkyl, $-(CH_2)_r-C_{5-7}$ spiro cycloalkyl, $-(CH_2)_r$-(4-membered monocyclic heterocycloalkyl), or $-(CH_2)_r$-(5- to 7-membered spiro heterocycloalkyl), wherein the alkyl, alkoxy, alkenyl, alkynyl, cycloalkyl, or heterocycloalkyl is optionally further substituted with 1-3 groups selected from D, F, Cl, cyano, amino or hydroxyl;

each $R_a$ is independently selected from $-C(O)N(R^{a1})_2$, $-NR^{a1}C(O)OR^{a1}$, - $NR^{a1}C(O)R^{a1}$, $-NR^{a1}C(O)N(R^{a1})_2$, $-C(=S)N(R^{a1})_2$, $-S(O)_2N(R^{a1})_2$, 5- to 6-membered monocyclic heteroaryl containing 1-4 nitrogen, oxygen or sulphur atoms, 4- to 7-membered monocyclic heterocycloalkyl containing 1-4 nitrogen, oxygen or sulphur atoms, or 3- to 7-membered monocyclic cycloalkyl, wherein the heteroaryl, heterocycloalkyl, or cycloalkyl is optionally further substituted with 1-3 substituents selected from D, halogen, cyano, hydroxyl, amino, $-NHC_{1-2}$ alkyl, $-N(C_{1-2}$ alkyl$)_2$, $C_{1-2}$ alkyl, halo $C_{1-2}$ alkyl, $C_{1-2}$ alkoxy, halo $C_{1-2}$ alkoxy, deuterated $C_{1-2}$ alkyl, or deuterated $C_{1-2}$ alkoxy;

each r is independently selected from 0 or 1;

p is selected from 0 or 1.

**8.** The compound, or the stereoisomer, solvate, or pharmaceutically acceptable salt thereof according to claim 1 or 2, **characterized in that**

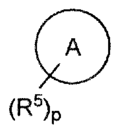

is selected from

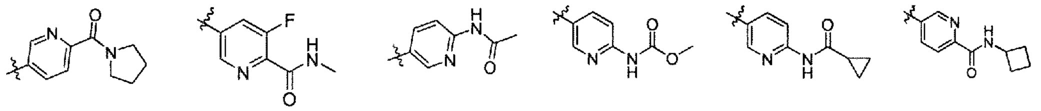

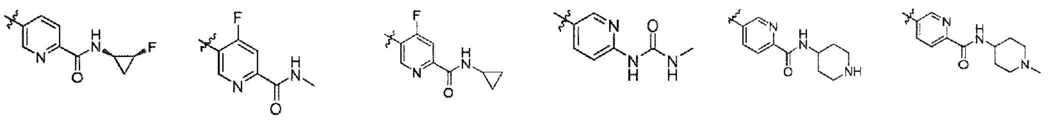

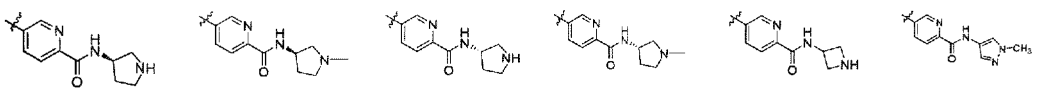

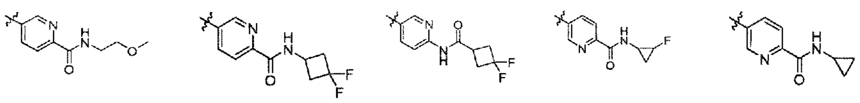

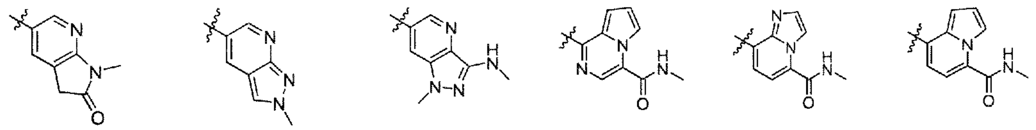

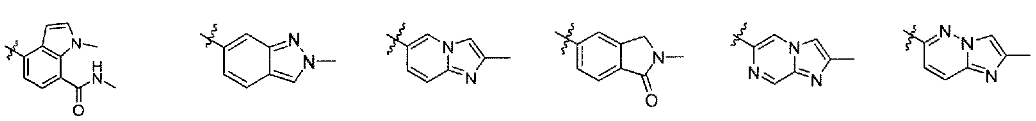

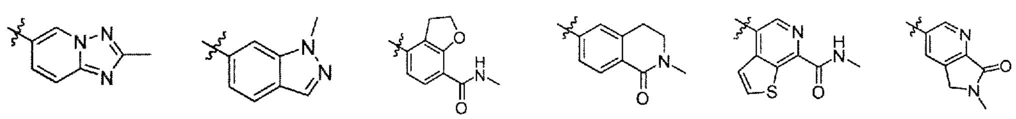

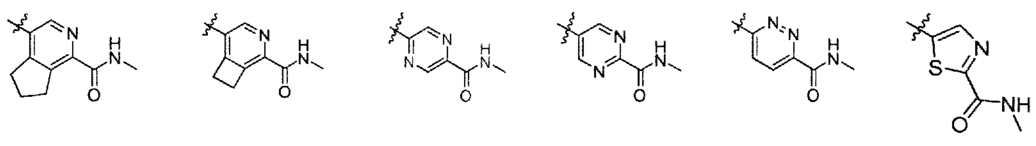

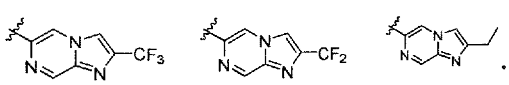
.

9. The compound, or the stereoisomer, solvate, or pharmaceutically acceptable salt thereof according to claim 1, having a structure of formula (VI) or (VII):

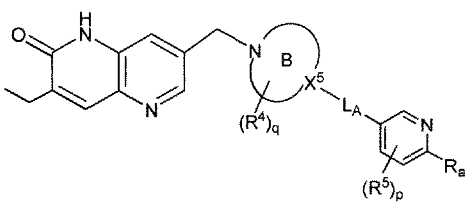
(VI)

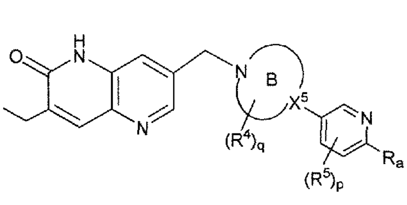
(VII)

**characterized in that** $R_a$ is selected from $-C(O)NHR^{a1}$ or $-NHC(O)R^{a1}$;

$L_A$ is selected from a bond, NH or $-N(CH_3)-$;

$X^5$ is independently selected from N, C or CH;

each $R^{a1}$ is independently selected from $C_{1-2}$ alkyl, $C_{3-5}$ cycloalkyl, 5- to 6-membered heteroaryl, halo $C_{1-2}$ alkyl or deuterated $C_{1-2}$ alkyl, wherein the cycloalkyl or heteroaryl is optionally further substituted with 1-3 substituents selected from D, F, Cl, cyano, hydroxyl, amino, $C_{1-2}$ alkyl, deuterated $C_{1-2}$ alkyl or deuterated $C_{1-2}$ alkoxy;

each $R^4$ is independently selected from D, F, Cl, cyano, $C_{1-2}$ alkyl, halo $C_{1-2}$ alkyl or deuterated $C_{1-2}$ alkyl; or two $R^4$ on the same carbon atom together with the carbon atom to which they are attached form 3- to 4-membered cycloalkyl;

each $R^5$ is independently selected from D, F, Cl, cyano, $C_{1-2}$ alkyl, halo $C_{1-2}$ alkyl or deuterated $C_{1-2}$ alkyl;

q is selected from 0, 1 or 2; p is selected from 0 or 1;

ring B is selected from piperidyl, 6-membered partially unsaturated monocyclic heterocycloalkane containing 1-2 nitrogen atoms, 6-membered saturated bridged heterocycle containing 1-2 nitrogen atoms, 7-membered saturated bridged heterocycle containing 1-2 nitrogen atoms, 8-membered saturated bridged heterocycle containing 1-2 nitrogen atoms, 8-membered saturated fused heterocycle containing 1-2 nitrogen atoms, 9-membered saturated fused heterocycle containing 1-2 nitrogen atoms, 10-membered saturated fused heterocycle containing 1-2 nitrogen atoms, 7-membered saturated spiro heterocycle containing 1-2 nitrogen atoms, 8-membered saturated spiro heterocycle containing 1-2 nitrogen atoms, 9-membered saturated spiro heterocycle containing 1-2 nitrogen atoms, 10-membered saturated spiro heterocycle containing 1-2 nitrogen atoms or 11-membered saturated spiro heterocycle containing 1-2 nitrogen atoms.

**10.** The compound, or the stereoisomer, solvate, or pharmaceutically acceptable salt thereof according to claim 9, **characterized in that**

each $R^{a1}$ is independently selected from methyl, ethyl, cyclopropyl, cyclobutyl, pyrazolyl, imidazolyl, thiazolyl, $CH_2F$, $CHF_2$, $CF_3$, $CH_2D$, $CHD_2$ or $CD_3$, wherein the cyclopropyl, cyclobutyl, pyrazolyl, imidazolyl, or thiazolyl is optionally further substituted with 1-3 substituents selected from D, F, Cl, cyano, hydroxyl, amino, methyl, $CH_2F$, $CHF_2$, $CF_3$, $-OCH_2D$, $-OCHD_2$, or $-OCD_3$;

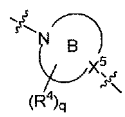

is selected from

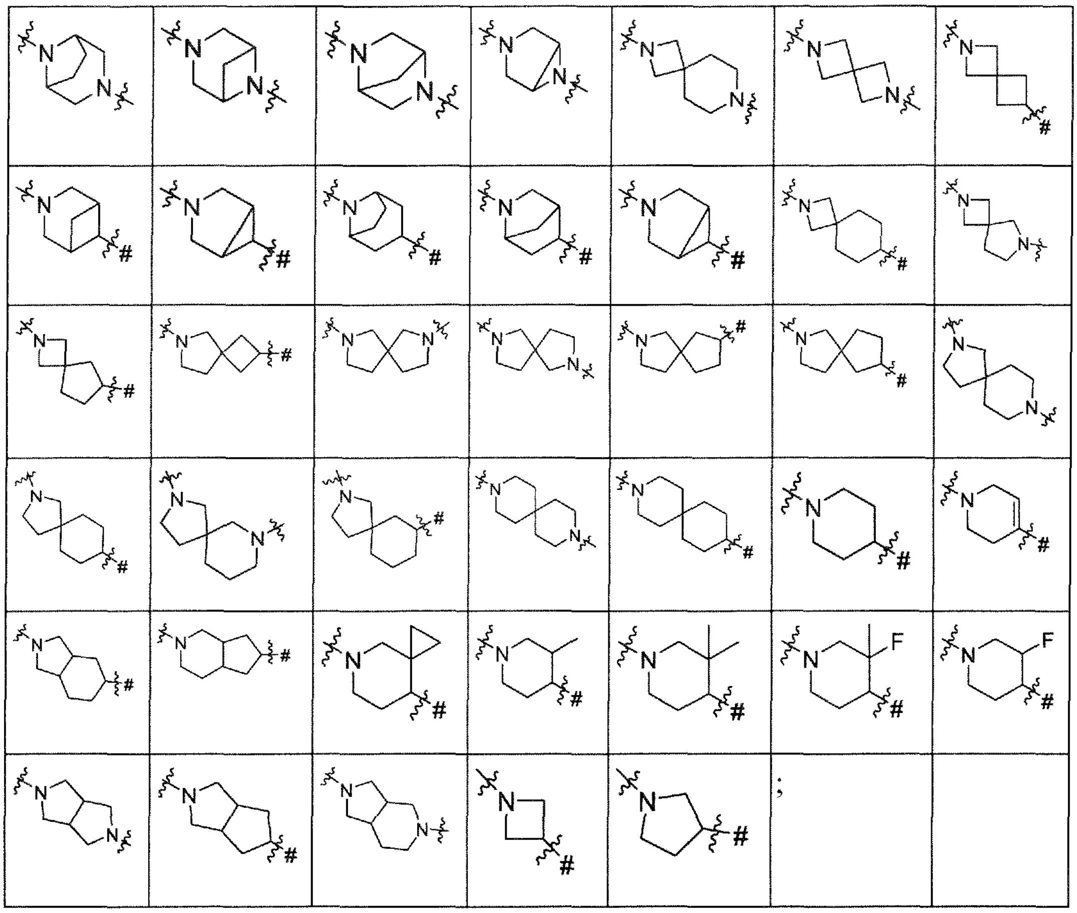

;

p is selected from 0.

11. The compound, or the stereoisomer, solvate, or pharmaceutically acceptable salt thereof according to claim 1, **characterized in that** the compound is selected from one of the following structures:

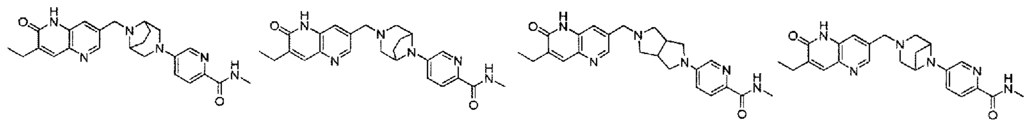

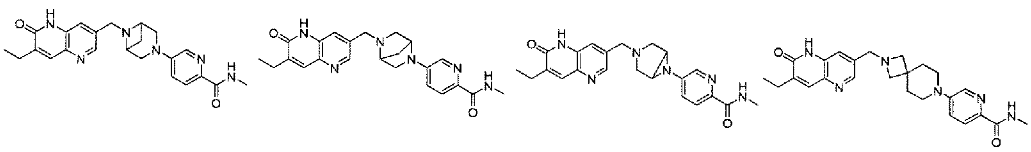

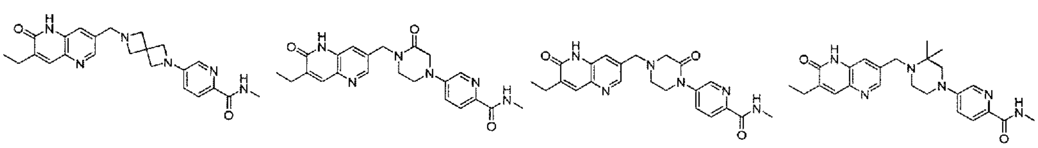

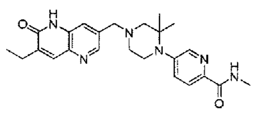
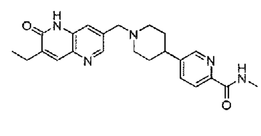
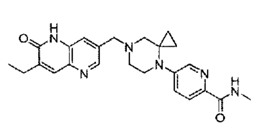
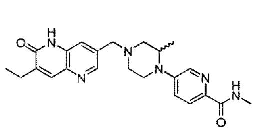
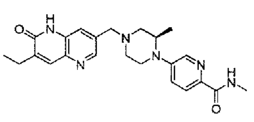
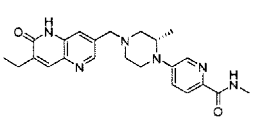
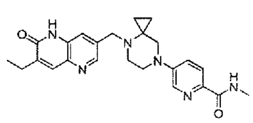
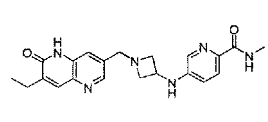
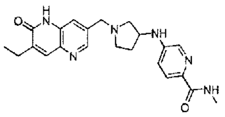
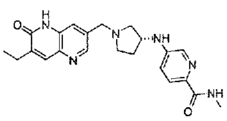
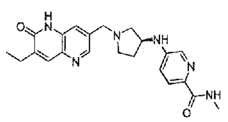
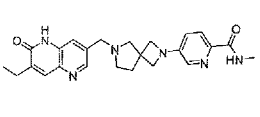
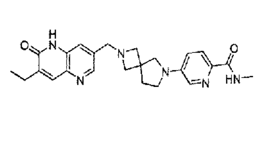
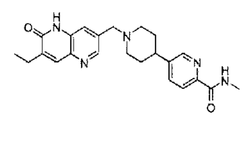
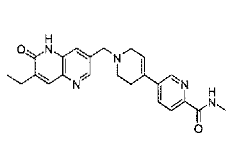
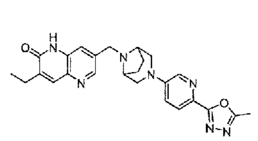
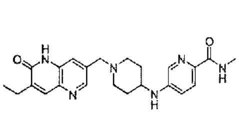
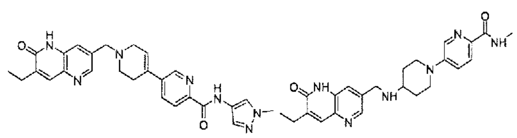
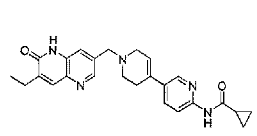
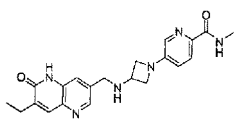
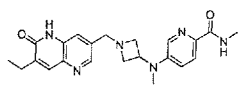
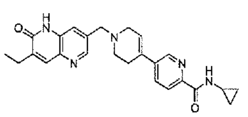
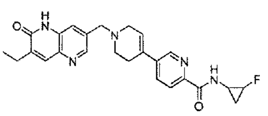
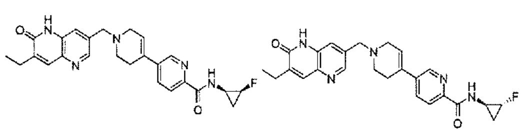
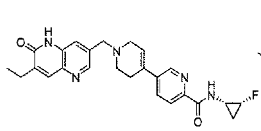
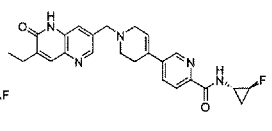
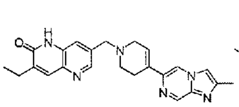
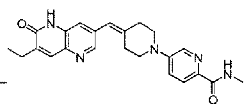
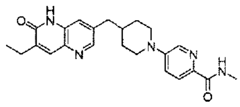
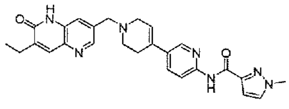
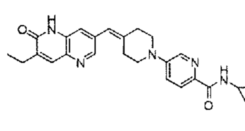
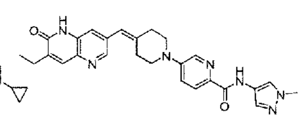
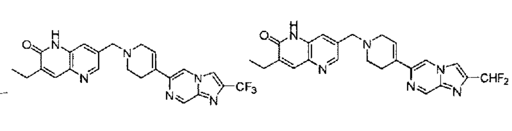
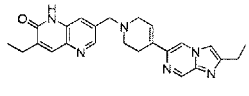
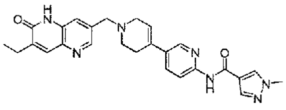

12. A pharmaceutical composition or pharmaceutical preparation, **characterized in that** the pharmaceutical composition or pharmaceutical preparation comprises the compound, or the stereoisomer, solvate, or pharmaceutically acceptable salt thereof according to any one of claims 1-11, and a pharmaceutically acceptable excipient and/or carrier.

**13.** The pharmaceutical composition or pharmaceutical preparation according to claim 12, **characterized in that** the pharmaceutical composition or pharmaceutical preparation comprises 1-1500 mg of the compound, or the stereoisomer, solvate, or pharmaceutically acceptable salt thereof according to any one of claims 1-11, and a pharmaceutically acceptable carrier and/or excipient.

**14.** Use of the compound, or the stereoisomer, solvate, or pharmaceutically acceptable salt thereof according to any one of claims 1-11, or the composition according to claim 12 in the preparation of a drug for treating/preventing a PARP-1-mediated disease.

**15.** The use according to claim 14, **characterized in that** the PARP-1-mediated disease is cancer.

**16.** A method for treating a disease in a mammal, **characterized in that** the method comprises administering to a subject a therapeutically effective amount of the compound, or the stereoisomer, solvate, or pharmaceutically acceptable salt thereof according to any one of claims 1-11, wherein the therapeutically effective amount is preferably 1-1500 mg, and the disease is preferably cancer.

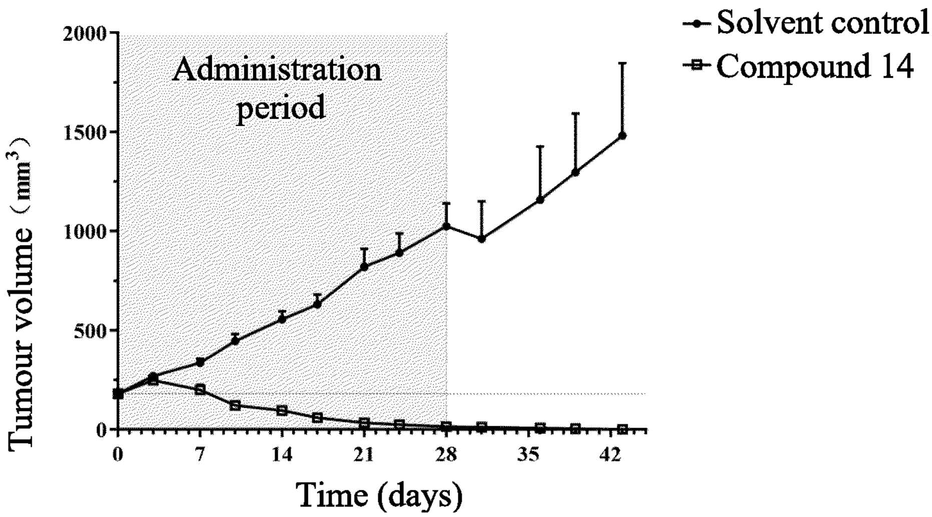
Solvent control
Compound 14
Administration period
Tumour volume（mm³）
2000
1500
1000
500
0
0
7
14
21
28
35
42
Time (days)

*FIG. 1*

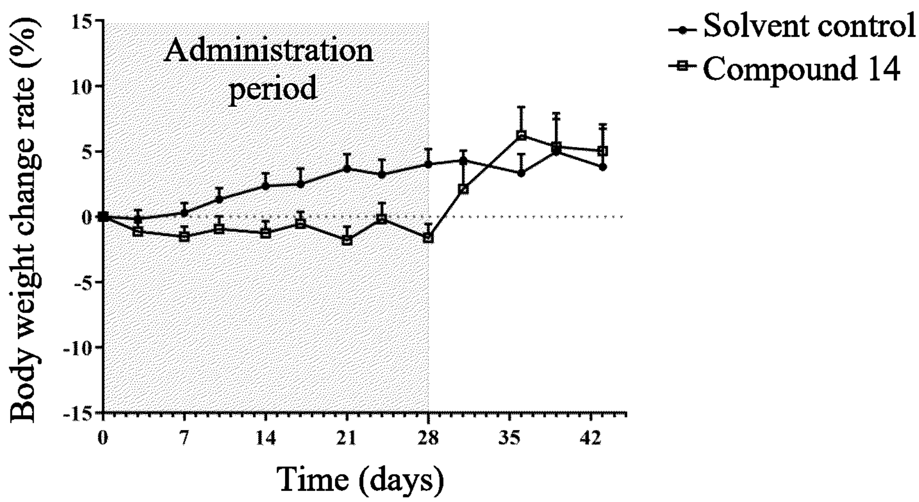
Solvent control
Compound 14
Administration period
Body weight change rate (%)
15
10
5
0
-5
-10
-15
0
7
14
21
28
35
42
Time (days)

*FIG. 2*

**INTERNATIONAL SEARCH REPORT**

International application No. **PCT/CN2022/123443**

**A. CLASSIFICATION OF SUBJECT MATTER**

C07D 471/04(2006.01)i；C07D 401/14(2006.01)i；A61K 31/496(2006.01)i；A61K 31/4375(2006.01)i；A61P 35/00(2006.01)i

According to International Patent Classification (IPC) or to both national classification and IPC

**B. FIELDS SEARCHED**

Minimum documentation searched (classification system followed by classification symbols)

C07D471/-; C07D401/-; A61K31/-; A61P35/-

Documentation searched other than minimum documentation to the extent that such documents are included in the fields searched

Electronic data base consulted during the international search (name of data base and, where practicable, search terms used)

WPI, EPODOC, CNPAT, CNKI, STN(REGISTRY): 聚(ADP-核糖)聚合酶, 氮杂喹诺酮, 氧代, 萘啶, 喹喔啉, 哌嗪, 抑制剂, PARP1, poly(ADP-ribose)polymerase, azaquinolone, oxo, naphthyridinyl, quinoxalinyl, piperazine, inhibitor, 肿瘤, 癌症, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2009053373 A1 (JANSSEN PHARMACEUTICA NV et al.) 30 April 2009 (2009-04-30)<br>description, page 9, line 6-page 89, line 1 | 1-16 |
| X | WO 2021013735 A1 (ASTRAZENECA AB) 28 January 2021 (2021-01-28)<br>description, page 2, line 7-page 61, line 5 | 1-16 |
| A | CN 102341394 A (TAKEDA PHARMACEUTICAL CO., LTD.) 01 February 2012 (2012-02-01)<br>description, embodiments 52-63, 81-85 and 94-96, and paragraphs [0010]-[0672] and [1299]-[1318] | 1-16 |
| E | WO 2022222921 A1 (WIGEN BIOMEDICINE TECHNOLOGY (SHANGHAI) CO., LTD.) 27 October 2022 (2022-10-27)<br>description, page 2, line 6-page 28, line 5 | 1-16 |
| E | WO 2022225934 A1 (XINTHERA, INC.) 27 October 2022 (2022-10-27)<br>description, paragraphs [0007]-[00449] | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2022** | **16 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/123443**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16**
because they relate to subject matter not required to be searched by this Authority, namely:

[1] Claim 16 sets forth a method for treating a mammalian disease, preferably cancer. Hence, claim 16 relates to a treatment method implemented on a human/animal body, and therefore does not comply with PCT Rule 39.1. Nevertheless, a search was still conducted on the basis of an effect claimed by the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/123443**

| Patent document cited in search report | Publication date (day/month/year) | Patent family member(s) | Publication date (day/month/year) |
|---|---|---|---|
| WO 2009053373 A1 | 30 April 2009 | PL 2215075 T3 | 30 April 2014 |
| | | US 2010222348 A1 | 02 September 2010 |
| | | ES 2448870 T3 | 17 March 2014 |
| | | EP 2215075 A1 | 11 August 2010 |
| | | JP 2011500758 A | 06 January 2011 |
| WO 2021013735 A1 | 28 January 2021 | AU 2020318599 A1 | 10 March 2022 |
| | | US 2021040084 A1 | 11 February 2021 |
| | | UY 38793 A | 26 February 2021 |
| | | CR 20220070 A | 21 March 2022 |
| | | EC SP22012826 A | 31 March 2022 |
| | | BR 112022000534 A2 | 10 May 2022 |
| | | AR 119424 A1 | 15 December 2021 |
| | | IL 289534 A | 01 March 2022 |
| | | KR 20220035941 A | 22 March 2022 |
| | | JP 2022541483 A | 26 September 2022 |
| | | CL 2022000110 A1 | 20 September 2022 |
| | | CN 114144413 A | 04 March 2022 |
| | | DO P2022000006 A | 15 March 2022 |
| | | CO 2022001590 A2 | 18 March 2022 |
| | | US 2022227768 A1 | 21 July 2022 |
| | | TW 202116750 A | 01 May 2021 |
| | | EP 3999506 A1 | 25 May 2022 |
| | | CA 3145644 A1 | 28 January 2021 |
| CN 102341394 A | 01 February 2012 | EC SP11011284 A | 31 October 2011 |
| | | WO 2010085570 A1 | 29 July 2010 |
| | | CR 20110452 A | 28 February 2012 |
| | | JP 2012515786 A | 12 July 2012 |
| | | US 2013274239 A1 | 17 October 2013 |
| | | CL 2011001754 A1 | 20 January 2012 |
| | | AU 2010206744 A1 | 04 August 2011 |
| | | SG 172958 A1 | 29 August 2011 |
| | | CA 2750106 A1 | 29 July 2010 |
| | | DO P2011000237 A | 15 September 2011 |
| | | EP 2389379 A1 | 30 November 2011 |
| | | US 2015031652 A1 | 29 January 2015 |
| | | CO 6410305 A2 | 30 March 2012 |
| | | US 2011158989 A1 | 30 June 2011 |
| | | EA 201170963 A1 | 30 March 2012 |
| | | KR 20110107396 A | 30 September 2011 |
| | | IL 213993 D0 | 31 August 2011 |
| | | BR PI1007358 A2 | 06 March 2018 |
| | | MY 152386 A | 15 September 2014 |
| | | MX 2011007741 A | 06 September 2011 |
| | | PE 20120418 A1 | 04 May 2012 |
| | | TN 2011000339 A1 | 27 March 2013 |
| | | NZ 594322 A | 25 January 2013 |
| | | US 2010190763 A1 | 29 July 2010 |
| | | US 2012122835 A1 | 17 May 2012 |
| | | MA 33053 B1 | 01 February 2012 |
| WO 2022222921 A1 | 27 October 2022 | None | |

Form PCT/ISA/210 (patent family annex) (January 2015)

**INTERNATIONAL SEARCH REPORT**
**Information on patent family members**

International application No.
**PCT/CN2022/123443**

| Patent document cited in search report | Publication date (day/month/year) | Patent family member(s) | Publication date (day/month/year) |
|---|---|---|---|
| WO 2022225934 A1 | 27 October 2022 | US 2022348574 A1 | 03 November 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021013735 A1


### Non-patent literature cited in the description


- Synthetic Organic Chemistry. John Wiley & Sons, Inc,
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modem Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopaedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002